(19) Europäisches Patentamt
European Patent Office
Office européen des brevets

(11) EP 4 238 954 A1

(12) **EUROPEAN PATENT APPLICATION**
published in accordance with Art. 153(4) EPC

(43) Date of publication:
**06.09.2023 Bulletin 2023/36**

(21) Application number: **21886293.6**

(22) Date of filing: **28.10.2021**

(51) International Patent Classification (IPC):
***C07C 37/52*** (2006.01) ***C01B 32/50*** (2017.01)
***C07C 39/16*** (2006.01) ***C07C 68/04*** (2006.01)
***C07C 69/96*** (2006.01) ***C08G 59/06*** (2006.01)
***C08G 64/30*** (2006.01) ***C08J 11/24*** (2006.01)
***C08J 11/28*** (2006.01) ***C07B 61/00*** (2006.01)

(52) Cooperative Patent Classification (CPC):
(C-Sets available)
**C07C 37/0555; C01B 32/50; C07C 68/02; C08G 59/06; C08G 64/30; C08J 11/24; C08J 11/28;** C07B 61/00; Y02P 20/141; Y02W 30/62 (Cont.)

(86) International application number:
**PCT/JP2021/039740**

(87) International publication number:
**WO 2022/092176 (05.05.2022 Gazette 2022/18)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.10.2020 JP 2020182543**
**31.03.2021 JP 2021061030**

(71) Applicant: **Mitsubishi Chemical Corporation**
**Chiyoda-ku**
**Tokyo 100-8251 (JP)**

(72) Inventors:
- **UCHIYAMA, Kei**
  **Tokyo 100-8251 (JP)**
- **NAKAMURA, Makoto**
  **Tokyo 100-8251 (JP)**

(74) Representative: **Vossius & Partner**
**Patentanwälte Rechtsanwälte mbB**
**Siebertstraße 3**
**81675 München (DE)**

# (54) BISPHENOL PRODUCTION METHOD, RECYCLED POLYCARBONATE RESIN PRODUCTION METHOD, CARBON DIOXIDE PRODUCTION METHOD, CARBONIC DIESTER PRODUCTION METHOD, EPOXY RESIN PRODUCTION METHOD, AND EPOXY RESIN CURED PRODUCT PRODUCTION METHOD

(57) A method for producing a bisphenol or the like by using a chemical recycling method that is moderate, has a small environmental load, and can efficiently degrade a polycarbonate resin is provided. In addition, a method for producing a recycled polycarbonate resin or the like by using a useful substance such as the bisphenol or the like is provided. A method for producing a bisphenol, comprising degrading a polycarbonate resin in the presence of an aromatic monoalcohol, water, and a catalyst. A method for producing carbon dioxide, comprising recovering carbon dioxide generated by the method for producing a bisphenol. A method for producing a carbonic acid diester by using the carbon dioxide. A method for producing a recycled polycarbonate resin by using the bisphenol and/or the carbonic acid diester. A method for producing an epoxy resin and a method for producing an epoxy resin cured product, by using the bisphenol

EP 4 238 954 A1

(52) Cooperative Patent Classification (CPC): (Cont.)

C-Sets
**C07C 37/0555, C07C 39/16;**
**C07C 68/02, C07C 69/96**

## Description

Technical Field

The present invention relates to a method for producing a bisphenol. Particularly, the present invention relates to a method for producing a bisphenol by using degradation of a polycarbonate resin. Further, the present invention relates to a method for producing a recycled polycarbonate resin by using a bisphenol obtained by the method for producing a bisphenol. In addition, the present invention relates to a method for producing carbon dioxide and a method for producing a carbonic acid diester, by using the degradation of a polycarbonate resin. In addition, the present invention relates to a method for producing an epoxy resin and a method for producing an epoxy resin cured product.

Background Art

A plastic is easy to use, durable, and inexpensive, and thus is mass-produced not only in Japan but also all over the world. Many of such plastics are used as "disposable" ones and thus some thereof are not appropriately treated and released into the environment. Specifically, a plastic waste flows from a river into the sea and deteriorates because of a wave or an ultraviolet ray in the process to become 5 mm or less in size. Such a small plastic waste is referred to as a microplastic. An animal or fish accidentally swallows this microplastic. As described above, a plastic waste has a tremendous influence on the ecosystem, and, in recent years, has been regarded as a marine plastic problem all over the world. A polycarbonate resin used in a wide range of fields is no exception because of the transparency, mechanical physical properties, flame retardancy, dimensional stability, and electrical characteristics thereof.

As one of the methods for recycling a polycarbonate resin, there is chemical recycling involving chemically degrading a polycarbonate resin back to a bisphenol for reuse, and hydrolysis is known as one of the methods for degrading a polycarbonate resin.

As a hydrolysis method, a method involving placing a polycarbonate resin and an alkaline aqueous solution in a pressure resistant container and hydrolyzing the polycarbonate resin at a high temperature and a high pressure is known (Patent Literature 1). In addition, a method involving dissolving a polycarbonate resin in a chlorinated hydrocarbon solvent and adding an alkali metal hydroxide as a basic catalyst to hydrolyze the polycarbonate resin is also known (Patent Literature 2).

In addition, phenolysis is known as another method for degrading a polycarbonate resin. For example, a method for degrading a polycarbonate resin by phenolysis to produce diphenyl carbonate and bisphenol A is also known (Patent Literatures 3 and 4) .

Citation List

Patent Literature

Patent Literature 1: Japanese Patent Publication No. 40-16536
Patent Literature 2: International Publication No. WO 2006/114893
Patent Literature 3: Japanese Patent Laid-Open No. 7-196582
Patent Literature 4: Japanese Patent Laid-Open No. 7-316280
Patent Literature 5: Japanese Patent Laid-Open No. 2005-97568
Patent Literature 6: Japanese Patent Laid-Open No. 2004-345883
Patent Literature 7: Japanese Patent Laid-Open No. 2006-144023

Summary of Invention

Technical Problem

Chemical recycling of a polycarbonate resin is important as one of the solutions to the marine plastic problem.

Water is used for hydrolysis of a polycarbonate resin, and when the hydrolysis is at 100°C (boiling point of water at normal pressure) or more, the vapor pressure of water provides a high pressure condition. For example, according to Example 1 of Patent Literature 1, a polycarbonate resin is degraded at 180 to 185°C, and water is used and thus provides a high pressure condition, and a pressure resistant container needs to be used. On the other hand, in a method for hydrolyzing a polycarbonate resin at around normal temperature, a chlorinated hydrocarbon solvent is used as a solvent in order to dissolve the polycarbonate resin. For example, according to Example 1 of Patent Literature

2, a polycarbonate resin is hydrolyzed at 40°C, and methylene chloride is used as a solvent. A chlorinated hydrocarbon solvent such as methylene chloride is chemically stable and thus is a flame retardant compound. Because of this, a problem thereof is that a dioxin is generated if a disposal treatment is not appropriately carried out at a high temperature.

In addition, in the phenolysis of a polycarbonate resin, the polycarbonate resin is depolymerized by using phenol, and this can be carried out at a phenolysis temperature of 100°C or more because the boiling point of phenol is high. For example, according to Example 1 of Patent Literature 3, a polycarbonate resin is phenolyzed at 160°C by using an amine as a catalyst to obtain diphenyl carbonate and bisphenol A. However, in order to purify bisphenol A from the generated diphenyl carbonate, it is necessary to carry out distillation separation at a high temperature under a strict vacuum condition, and this has the following problems: a harsh condition is required and the purification operation is complicated.

As described above, any conventional chemical recycling of a polycarbonate resin requires a condition having a large environmental load or a harsh condition, and is required to be further improved.

On the other hand, diphenyl carbonate, which is a raw material for a polycarbonate resin, is generated by carbonyl chloride and phenol in the presence of an alkaline catalyst such as pyridine, neutralized with an alkaline aqueous solution, and then obtained by distillation (Patent Literature 5). The neutralized wastewater discharged during the neutralization is treated in a wastewater treatment step and then treated with activated sludge, and a problem thereof is that the load on the activated sludge is large because a large amount of the neutralized wastewater is discharged.

In addition, in the reaction between carbonyl chloride and phenol, hydrogen chloride is by-produced in addition to diphenyl carbonate. Hydrogen chloride generated together with diphenyl carbonate is once absorbed into water to become hydrochloric acid, and then this hydrochloric acid becomes 18% by mass of hydrochloric acid with hydrogen chloride stripped in a stripping distillation column, and the stripped hydrogen chloride is converted to chlorine in an oxidation step, which is the next step (Patent Literature 6). This 18% by mass of hydrochloric acid includes a sulfur component. In order to avoid the concentration of this sulfur component in the system, part thereof is disposed of as hydrochloric acid wastewater.

In addition, carbonyl chloride, which is a raw material for diphenyl carbonate, is synthesized by reacting chlorine with carbon monoxide. According to Patent Literature 7, the unliquefied gas that has not been able to be liquefied at the time of liquefaction is subjected to a detoxification treatment with a sodium hydroxide aqueous solution (caustic soda aqueous solution), carbonyl chloride (phosgene) included in the unliquefied gas is completely degraded, and then the resulting unliquefied gas is discharged into the atmosphere as a waste gas. Because it is necessary to completely degrade carbonyl chloride for safety, a large amount of an aqueous solution having a high concentration of sodium hydroxide is used in the detoxification treatment of the unliquefied gas. A problem thereof is that the large amount of an aqueous sodium hydroxide solution having a high concentration is disposed of as sodium hydroxide wastewater.

From the viewpoint of reducing the environmental load, it is also required to effectively use a wastewater such as the neutralized wastewater discharged during the production of diphenyl carbonate, the hydrochloric acid wastewater discharged during the recovery of by-produced hydrogen chloride, or the sodium hydroxide wastewater discharged in the detoxification treatment of the unliquefied gas generated during the production of carbonyl chloride.

The present invention has been made in view of such circumstances, and an object thereof is to provide a method for producing a bisphenol, including producing a bisphenol by using a chemical recycling method that is mild, has a small environmental load, and can efficiently degrade a polycarbonate resin.

Further, another object of the present invention is to provide a method for producing a recycled polycarbonate resin by using the obtained bisphenol.

In addition, another object of the present invention is to provide a method for producing carbon dioxide by using the method for producing a bisphenol and a method for producing a carbonic acid diester by using the obtained carbon dioxide.

In addition, another object of the present invention is to provide a method for producing an epoxy resin and a method for producing an epoxy resin cured product by using the obtained epoxy resin.

Solution to Problem

The present inventors have carried out a diligent study in order to solve the above problems and, as a result, found a degradation method for degrading a polycarbonate resin in the presence of an aromatic monoalcohol such as phenol or a cresol, water, and a catalyst. In addition, the present inventors have found a method for producing a bisphenol or carbon dioxide by using the method for degrading a polycarbonate resin. Further, the present inventors have found a production method for producing a useful substance such as a recycled polycarbonate resin by using the obtained bisphenol or carbon dioxide.

That is, the present invention relates to the following invention.

<1> A method for producing a bisphenol, comprising degrading a polycarbonate resin in the presence of an aromatic

monoalcohol, water, and a catalyst.

<2> The method for producing a bisphenol according to the <1>, wherein the catalyst is any selected from the group consisting of an alkali metal hydroxide, an alkali metal carbonate, an alkylamine, a nitrogen-containing heterocyclic compound, and an acid.

<3> The method for producing a bisphenol according to the <2>, wherein the alkali metal hydroxide is sodium hydroxide or potassium hydroxide.

<4> The method for producing a bisphenol according to the <2>, wherein the alkylamine is represented by the following formula (I) :

[Formula 1]

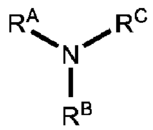

···(I)

wherein $R^A$ represents an alkyl group having 1 to 3 carbon atoms, and $R^B$ and $R^C$ each independently represent a hydrogen atom or an alkyl group having 1 to 3 carbon atoms.

<5> The method for producing a bisphenol according to the <2> or <4>, wherein the alkylamine is a tertiary amine.

<6> The method for producing a bisphenol according to the <2>, wherein the acid is any selected from the group consisting of hydrochloric acid, sulfuric acid, phosphoric acid, and a sulfonic acid.

<7> The method for producing a bisphenol according to the <1>, wherein the catalyst comprises a nitrogen-containing heterocyclic compound, and the polycarbonate resin is degraded in the coexistence of an alkali metal chloride in addition to the aromatic monoalcohol, the water, and the catalyst.

<8> The method for producing a bisphenol according to the <2> or <7>, wherein the nitrogen-containing heterocyclic compound is a pyridine.

<9> The method for producing a bisphenol according to the <7>, wherein the alkali metal chloride is sodium chloride.

<10> The method for producing a bisphenol according to any one of the <1> to <9>, wherein a reaction temperature for degrading the polycarbonate resin is 110°C or less.

<11> The method for producing a bisphenol according to any one of the <1> to <10>, wherein the polycarbonate resin is degraded in a slurry-like reaction liquid comprising the polycarbonate resin, the aromatic monoalcohol, the water, and the catalyst.

<12> The method for producing a bisphenol according to any one of the <1> to <11>, wherein a mass ratio of the water to the aromatic monoalcohol is 0.001 or more and 10 or less.

<13> The method for producing a bisphenol according to the <1>, wherein the catalyst comprises hydrochloric acid, and the polycarbonate resin is degraded in the coexistence of a bromophenol in addition to the aromatic monoalcohol, the water, and the catalyst.

<14> The method for producing a bisphenol according to the <1>, wherein the catalyst comprises sodium hydroxide, and the polycarbonate resin is degraded in the coexistence of sodium chloride and/or carbon tetrachloride in addition to the aromatic monoalcohol, the water, and the catalyst.

<15> The method for producing a bisphenol according to any one of the <1> to <14>, wherein the aromatic monoalcohol is any selected from the group consisting of phenol, a cresol, and a xylenol.

<16> The method for producing a bisphenol according to any one of the <1> to <15>, wherein the bisphenol is 2,2-bis(4-hydroxyphenyl)propane.

<17> The method for producing a bisphenol according to the <1> or <7>, wherein when a diaryl carbonate is produced by a method having the following step (a1), step (b1), step (b2), and step (b3), a neutralized wastewater removed in the step (b1) is used for degrading the polycarbonate resin:

Step (a1): A step of reacting carbonyl chloride with an aromatic monoalcohol in the presence of a nitrogen-containing heterocyclic compound to obtain a reaction liquid comprising a diaryl carbonate

Step (b1): A step of neutralizing the reaction liquid comprising a diaryl carbonate obtained in the step (a1) with an alkali metal hydroxide aqueous solution, carrying out oil water separation thereof into an oil phase comprising an aromatic diaryl and an aqueous phase comprising the nitrogen-containing heterocyclic compound and an alkali metal chloride, and then removing the aqueous phase as a neutralized wastewater

Step (b2): A step of washing the oil phase obtained in the step (b1) with water

Step (b3): A step of obtaining the diaryl carbonate from the oil phase after the step (b2).

<18> The method for producing a bisphenol according to the <17>, wherein the alkali metal chloride in the step (b1) is sodium chloride, and the alkali metal hydroxide aqueous solution in the step (b1) is a sodium hydroxide aqueous solution.

<19> The method for producing a bisphenol according to the <1> or <13>, wherein in production of a diaryl carbonate and recovery of hydrogen chloride by-produced having the following step (a1), step (c1), step (c2), and step (c3), hydrochloric acid wastewater removed in the step (c3) is used for degrading the polycarbonate resin:

Step (a1): A step of reacting carbonyl chloride with an aromatic monoalcohol in the presence of a nitrogen-containing heterocyclic compound to obtain a reaction liquid comprising a diaryl carbonate
Step (c1): A step of supplying hydrogen chloride by-produced in the step (a1) to an absorption column and absorbing the same into water or dilute hydrochloric acid to obtain concentrated hydrochloric acid
Step (c2): A step of distilling the concentrated hydrochloric acid in a stripping column, recovering hydrogen chloride gas from the top of the column, and recovering hydrochloric acid from the bottom of the column
Step (c3): A step of removing part of the hydrochloric acid recovered from the bottom of the column as hydrochloric acid wastewater outside a system and circulating the remaining hydrochloric acid to the absorption column of the step (c1).

<20> The method for producing a bisphenol according to the <1> or <14>, wherein in production of carbonyl chloride and treatment of an unliquefied gas having the following step (d1) to step (d4), sodium hydroxide wastewater removed in the step (d4) is used for degrading the polycarbonate resin:

Step (d1): A step of obtaining carbonyl chloride gas from chlorine and carbon monoxide
Step (d2): A step of cooling the carbonyl chloride gas obtained in the step (d1) to obtain liquefied carbonyl chloride
Step (d3): A step of contacting a circulating sodium hydroxide aqueous solution with an unliquefied gas that has not been liquefied in the (d2) to degrade carbonyl chloride in the unliquefied gas, and then discharging the resulting unliquefied gas
Step (d4); A step of removing part of the circulating sodium oxide aqueous solution as sodium hydroxide wastewater.

<21> A method for producing a recycled polycarbonate resin, comprising producing a recycled polycarbonate resin by using a bisphenol raw material comprising a bisphenol obtained by the method for producing a bisphenol according to any one of the <1> to <20>.

<22> A method for producing carbon dioxide, comprising recovering carbon dioxide generated by the method for producing a bisphenol according to any one of the <1> to <20>.

<23> A method for producing a carbonic acid diester, comprising producing a carbonic acid diester by using carbon dioxide obtained by the method for producing carbon dioxide according to the <22>.

<24> The method for producing a carbonic acid diester according to the <23>, wherein the method comprises a step of reacting carbon dioxide comprising the carbon dioxide with an aliphatic monoalcohol.

<25> The method for producing a carbonic acid diester according to the <23>, wherein carbon monoxide is obtained from the carbon dioxide comprising the carbon dioxide and coke, the obtained carbon monoxide is reacted with chlorine to obtain carbonyl chloride, and the obtained carbonyl chloride is reacted with an aromatic monoalcohol to obtain the carbonic acid diester.

<26> A method for producing a recycled polycarbonate resin, comprising producing a recycled polycarbonate resin by using a carbonic acid diester raw material comprising a carbonic acid diester obtained by the method for producing a carbonic acid diester according to any one of the <23> to <25>.

<27> A method for producing an epoxy resin, comprising producing an epoxy resin by using a bisphenol obtained by the method for producing a bisphenol according to any one of the <1> to <20>.

<28> The method for producing an epoxy resin according to the <27>, wherein the epoxy resin is further reacted with a polyhydroxy compound raw material.

<29> A method for producing an epoxy resin cured product, comprising curing an epoxy resin composition comprising an epoxy resin obtained by the method for producing an epoxy resin according to the <27> or <28> and a curing agent to obtain an epoxy resin cured product.

## Advantageous Effects of Invention

According to the present invention, a method for producing a bisphenol, including producing a bisphenol by using a chemical recycling method that can efficiently degrade a polycarbonate resin under a condition that is mild and has a small environmental load is provided. In addition, according to the method for producing a bisphenol according to

the present invention, the operations at the time of recovery and purification of a bisphenol are easy.

In addition, according to the present invention, it is possible to effectively use a wastewater such as the neutralized wastewater discharged during the production of diphenyl carbonate, the hydrochloric acid wastewater discharged during the recovery of by-produced hydrogen chloride, or the sodium hydroxide wastewater discharged in the detoxification treatment of the unliquefied gas generated during the production of carbonyl chloride.

Further, according to the present invention, a method for producing a recycled polycarbonate resin by using the obtained bisphenol is provided.

In addition, according to the present invention, a method for producing carbon dioxide by using the method for producing a bisphenol and a method for producing a carbonic acid diester by using the obtained carbon dioxide are provided.

In addition, according to the present invention, a method for producing an epoxy resin and a method for producing an epoxy resin cured product by using the obtained epoxy resin are provided.

Brief Description of Drawings

[Figure 1] Figure 1 shows a flow diagram for describing an example of the method for producing a bisphenol according to the present invention by using a wastewater.
[Figure 2] Figure 2 shows a flow diagram for describing an example of the method for producing a bisphenol according to the present invention by using a wastewater.
[Figure 3] Figure 3 shows a flow diagram for describing an example of the method for producing a bisphenol according to the present invention by using a wastewater.

Description of Embodiment

Hereinafter, an embodiment of the present invention will be described in detail, but the description of the constituent requirements described below is an example of the embodiment of the present invention, and the present invention is not limited to the contents of the following description as long as they do not depart from the scope of the present invention. In addition, as used herein, an expression of a range including the term "to" with numerical values or physical property values provided before and after the term is used to mean that the values before and after the term are included in the range.

<Method for producing bisphenol>

The present invention relates to a method for producing a bisphenol, including degrading a polycarbonate resin in the presence of an aromatic monoalcohol, water, and a catalyst (hereinafter, sometimes referred to as the "method for producing a bisphenol according to the present invention").

The method for producing a bisphenol according to the present invention uses a chemical recycling method involving degrading a polycarbonate resin in the presence of an aromatic monoalcohol, water, and a catalyst.

The present inventors have found that by using an aromatic monoalcohol and water in combination in the presence of a catalyst, a polycarbonate resin can be degraded into a bisphenol and carbon dioxide and/or into a salt of a bisphenol and a metal salt of carbonic acid even under a mild condition such as about the boiling point of water (normal pressure, about 100°C). In addition, it has been found that the degradation reaction of a polycarbonate resin is caused at a high reaction rate by using an aromatic monoalcohol and water in combination, without completely dissolving the polycarbonate resin by using a solvent having high solubility of a polycarbonate resin such as a halogen solvent. In addition, it has been found that carbon dioxide and the metal salt of carbonic acid generated by the degradation of a polycarbonate resin can be easily removed outside the system, and the recovery and purification of a bisphenol can be easily carried out. The present invention is based on these findings.

It is considered that by using an aromatic monoalcohol and water in combination, the reactions of solvolysis (for example, phenolysis) and hydrolysis due to the aromatic monoalcohol are caused in the system, and thus the polycarbonate resin is easily degraded even under a mild condition, and the diaryl carboxylate generated by the reaction of the polycarbonate resin with the aromatic monoalcohol is hydrolyzed to carbon dioxide, which is easily discharged outside the system, and thus the purification is also facilitated.

(Polycarbonate resin)

The polycarbonate resin used in the method for producing a bisphenol according to the present invention

includes a polymerization composition including a carbonate bond (-O-C(=O)-O-). Specifically, the polycarbonate resin used in the method for producing a bisphenol according to the present invention includes a polymer including a constitutional unit derived from a bisphenol represented by the general formula (1).

[Formula 2]

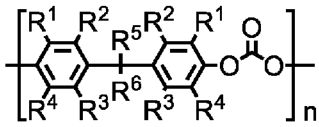

・・・(1)

The substituents $R^1$ to $R^4$ each independently represent a hydrogen atom, a halogen atom, an alkyl group, an alkoxy group, an aryl group, or the like. Examples thereof include a hydrogen atom, a fluoro group, a chloro group, a bromo group, an iodine group, a methyl group, an ethyl group, a n-propyl group, an i-propyl group, a n-butyl group, an i-butyl group, a t-butyl group, a n-pentyl group, an i-pentyl group, a n-hexyl group, a n-heptyl group, a n-octyl group, a n-nonyl group, a n-decyl group, a n-undecyl group, a n-dodecyl group, a methoxy group, an ethoxy group, a n-propoxy group, an i-propoxy group, a n-butoxy group, an i-butoxy group, a t-butoxy group, a n-pentyloxy group, an i-pentyloxy group, a n-hexyloxy group, a n-heptyloxy group, a n-octyloxy group, a n-nonyloxy group, a n-decyloxy group, a n-undecyloxy group, a n-dodecyloxy group, a cyclopropyl group, a cyclobutyl group, a cyclopentyl group, a cyclohexyl group, a cycloheptyl group, a cyclooctyl group, a cyclododecyl group, a benzyl group, a phenyl group, a tolyl group, and 2,6-dimethylphenyl group.

The substituents $R^5$ and $R^6$ each independently represent a hydrogen atom, an alkyl group, an alkoxy group, an aryl group, or the like. Examples thereof include a hydrogen atom, a methyl group, an ethyl group, a n-propyl group, an i-propyl group, a n-butyl group, an i-butyl group, a t-butyl group, a n-pentyl group, an i-pentyl group, a n-hexyl group, a n-heptyl group, a n-octyl group, a 2-ethylhexyl group, a n-nonyl group, a n-decyl group, a n-undecyl group, a n-dodecyl group, a methoxy group, an ethoxy group, a n-propoxy group, an i-propoxy group, a n-butoxy group, an i-butoxy group, a t-butoxy group, a n-pentyloxy group, an i-pentyloxy group, a n-hexyloxy group, a n-heptyloxy group, a n-octyloxy group, a n-nonyloxy group, a n-decyloxy group, a n-undecyloxy group, a n-dodecyloxy group, a cyclopropyl group, a cyclobutyl group, a cyclopentyl group, a cyclohexyl group, a cycloheptyl group, a cyclooctyl group, a cyclododecyl group, a benzyl group, a phenyl group, a tolyl group, and 2,6-dimethylphenyl group.

$R^5$ and $R^6$ may be bonded or crosslinked to each other between the two groups. Examples thereof include cyclopropylidene, cyclobutylidene, cyclopentylidene, cyclohexylidene, 3,3,5-trimethylcyclohexylidene, cycloheptylidene, cyclooctylidene, cyclononylidene, cyclodecylidene, cycloundecylidene, cyclododecylidene, fluorenylidene, xanthonylidene, and thioxanthonylidene.

Among these, a polycarbonate resin wherein in the above general formula (1), $R^1$ to $R^4$ are each a hydrogen atom and $R^5$ and $R^6$ are each a methyl group (hereinafter, sometimes referred to as a "bisphenol A type polycarbonate resin") is suitably used as a raw material.

In the general formula (1), n is not particularly limited, and is, for example, 2 to 1,000.

In addition, as the polycarbonate resin, not only a polycarbonate resin alone but also a composition including a resin other than a polycarbonate resin such as a copolymer or a polymer alloy may be used. Examples of the composition including a resin other than a polycarbonate resin include a polycarbonate/polyester copolymer, a polycarbonate/polyester alloy, a polycarbonate/polyarylate copolymer, and a polycarbonate/polyarylate alloy. When a composition including a resin other than a polycarbonate resin is used, a composition including a polycarbonate resin as the main component (the composition includes 50% by mass or more of a polycarbonate resin) is suitable.

In addition, as the polycarbonate resin, two or more different polycarbonate resins may be mixed and used. The polycarbonate resin alone may be simply referred to as polycarbonate.

From the viewpoint of chemical recycling, the polycarbonate resin is preferably a polycarbonate resin included in a waste plastic. By stirring a reaction liquid including a waste plastic in which a polycarbonate resin is included, an aromatic monoalcohol, water, and a catalyst, the polycarbonate resin included in the waste plastic can be degraded to generate a bisphenol or a salt thereof.

The polycarbonate resin is molded into various molded articles such as an optical member such as a headlamp or an optical recording medium such as an optical disk and used. As the waste plastic including a polycarbonate resin, a scrap or a defective product when molding and processing the polycarbonate resin into such a molded article, a used molded article, or the like can be used.

The waste plastic may be appropriately washed, crushed, ground, or the like before use. The method for crushing the waste plastic include coarse crushing using a jaw crusher or a gyratory crusher for crushing to 20 cm or less, medium crushing using a gyratory crusher, a corn crusher, or a mill for crushing to 1 cm or less, grinding using a

mill for crushing to 1 mm or less, or the like, and may be any method that can reduce the waste plastic to a size at which it can be supplied to a degradation tank. In addition, when the waste plastic is a thin plastic such as a CD or a DVD, the thin plastic can be cut by using a shredder or the like and supplied to a degradation tank. In addition, another resin of a copolymer or a polymer alloy, or a portion formed of a component other than a polycarbonate resin such as a layer on the front surface or the back surface of an optical disk may be removed in advance before use.

(Aromatic monoalcohol)

One of the features of the method for producing a bisphenol according to the present invention is that an aromatic monoalcohol is used. The aromatic monoalcohol is a compound in which one hydroxyl group is bonded to a carbon atom forming an aromatic ring, and is preferably any selected from the group consisting of phenol, a cresol, and a xylenol.

Examples of the cresol include orthocresol, metacresol, paracresol, and an isomer mixture including one or more thereof. If the cresol is a liquid at around 30°C, it is easy to supply it to a degradation tank, and thus the cresol is preferably orthocresol, metacresol, an isomer mixture of metacresol and paracresol, or an isomer mixture of orthocresol, metacresol, and paracresol.

Examples of the xylenol include 2,3-xylenol, 2,4-xylenol, 2,5-xylenol, 2,6-xylenol, 3,5-xylenol, 3,4-xylenol, and an isomer mixture including one or more thereof. 2,5-Xylenol is preferable because it is industrially inexpensive to obtain.

When the mass ratio of the aromatic monoalcohol to the polycarbonate resin (mass of aromatic monoalcohol/mass of polycarbonate resin) is small, the amount of the solid (polycarbonate resin) based on that of the liquid increases and the slurry concentration increases, which tends to result in poor mixing. Because of this, the mass ratio is preferably 0.01 or more, more preferably 0.03 or more, and further preferably 0.05 or more. In addition, when the mass ratio is large, the production efficiency of a bisphenol and carbon dioxide tends to deteriorate. Because of this, the mass ratio is preferably 100 or less, more preferably 70 or less, and further preferably 50 or less.

(Water)

One of the features of the method for producing a bisphenol according to the present invention is that water is used together with an aromatic monoalcohol. When the mass ratio of water to the polycarbonate resin (mass of water/mass of polycarbonate resin) is small, the degradation rate decreases, and thus the degradation time is lengthened and the efficiency tends to deteriorate.

Because of this, the mass ratio is preferably 0.1 or more, more preferably 0.5 or more, and further preferably 1.0 or more. In addition, when the mass ratio is large, the production efficiency of a bisphenol and carbon dioxide tends to decrease. Because of this, the mass ratio is preferably 100 or less, more preferably 70 or less, and further preferably 50 or less.

In addition, the mass ratio of water to the aromatic monoalcohol (mass of water/mass of aromatic monoalcohol) is preferably 0.001 or more, and more preferably 0.05 or more. In addition, the mass ratio is preferably 20 or less, and more preferably 15 or less. The mass ratio may be 10 or less, 5 or less, 1 or less, 0.5 or less, 0.2 or less, or the like. When the mass ratio of water to the aromatic monoalcohol is small, the degradation rate decreases and the degradation time is lengthened, and when the mass ratio is large, the volume of the reaction liquid increases, resulting in inefficiency.

(Catalyst)

One of the features of the method for producing a bisphenol according to the present invention is that further a catalyst is used. The catalyst may be any as long as it can accelerate the degradation of the polycarbonate resin, and a base or an acid can be used as the catalyst. As the base, one or more selected from the group consisting of an alkali metal hydroxide, an alkali metal carbonate, an alkylamine, and a nitrogen-containing heterocyclic compound are preferable. Among these, any selected from the group consisting of an alkali metal hydroxide, an alkali metal carbonate, an alkylamine, a nitrogen-containing heterocyclic compound, and an acid is preferable.

[Alkali metal hydroxide]

The alkali metal hydroxide is a salt of an alkali metal ion ($M^+$) and a hydroxide ion ($OH^-$), and is a compound represented by MOH where M represents an alkali metal atom. As the alkali metal hydroxide, sodium hydroxide or potassium hydroxide is preferable.

When the mass ratio of the alkali metal hydroxide to the polycarbonate resin (mass of alkali metal hydroxide/mass of polycarbonate resin) is small, the degradation rate decreases, the degradation time is lengthened, and the efficiency tends to deteriorate. Because of this, the mass ratio is preferably 0.01 or more, more preferably 0.1 or more, and further

preferably 0.5 or more. In addition, when the mass ratio is large, the amount of an acid required for neutralization after degradation increases, and the production efficiency of a bisphenol and carbon dioxide tends to decrease. Because of this, the mass ratio is preferably 50 or less, more preferably 30 or less, and further preferably 10 or less. In addition, the mass ratio may be 8 or less, 5 or less, 3 or less, or the like.

[Alkali metal carbonate]

The alkali metal carbonate is a salt of an alkali metal ion ($M^+$) and a carbonate ion ($CO_3^{2-}$), and is a compound represented by $M_2CO_3$ where M represents an alkali metal atom. As the alkali metal carbonate, sodium carbonate or potassium carbonate is preferable.

When the mass ratio of the alkali metal carbonate to the polycarbonate resin (mass of alkali metal carbonate/mass of polycarbonate resin) is small, the degradation rate decreases, the degradation time is lengthened, and the efficiency tends to deteriorate. Because of this, the mass ratio is preferably 0.01 or more, more preferably 0.1 or more, and further preferably 0.5 or more. In addition, when the mass ratio is large, the amount of an acid required for neutralization after degradation increases, and the production efficiency of a bisphenol and carbon dioxide tends to decrease. Because of this, the mass ratio is preferably 50 or less, more preferably 30 or less, and further preferably 10 or less. In addition, the mass ratio may be 5 or less, 1 or less, 0.5 or less, or the like.

[Alkylamine]

The alkylamine is a compound obtained by replacing at least one hydrogen atom of ammonia with an alkyl group. Among the alkylamines, a monoalkylamine, which is a primary amine, reacts with a carbonate bond moiety of the polycarbonate resin to form an isocyanate, and thus a dialkylamine, which is a secondary amine, and a trialkylamine, which is a tertiary amine, are more preferable.

The dialkylamine, which is a secondary amine, reacts with a carbonate bond moiety of the polycarbonate resin to form a tetraalkylurea, and thus a trialkylamine, which is a tertiary amine, is further preferable.

An alkylamine having a boiling point of 200°C or less is preferable, and an alkylamine having a boiling point of 160°C or less is more preferable. If an alkylamine has such a boiling point, the alkylamine can be removed by depressurization and/or heating together with an aromatic monoalcohol such as phenol. In addition, when the boiling point is too low, the alkylamine may volatilize during the degradation reaction and the degradation rate may decrease, and thus the boiling point of the alkylamine is preferably 10°C or more, and more preferably 30°C or more.

The alkylamine is preferably an alkylamine represented by the general formula (I).

[Formula 3]

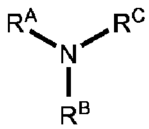

···(I)

wherein $R^A$ represents an alkyl group having 1 to 3 carbon atoms, and $R^B$ and $R^C$ each independently represent a hydrogen atom or an alkyl group having 1 to 3 carbon atoms.

$R^A$ is preferably a methyl group, an ethyl group, a n-propyl group, or an isopropyl group, and $R^B$ to $R^C$ is each independently preferably a hydrogen atom, a methyl group, an ethyl group, a n-propyl group, or an isopropyl group.

Specific examples of the alkylamine represented by the general formula (I) include methylamine, ethylamine, propylamine, dimethylamine, diethylamine, trimethylamine, and diethylamine.

When the mass ratio of the alkylamine to the polycarbonate resin (mass of alkylamine/mass of polycarbonate resin) is small, the degradation rate decreases, and thus the degradation time is lengthened and the efficiency tends to deteriorate. Because of this, the mass ratio is preferably 0.001 or more, more preferably 0.005 or more, and further preferably 0.01 or more. In addition, when the mass ratio is large, the excess alkylamine inhibits the reaction of generating carbon dioxide, and thus the mass ratio is preferably 50 or less, more preferably 20 or less, and further preferably 10 or less. In addition, the mass ratio may be 5 or less, 1 or less, 0.5 or less, or the like.

[Nitrogen-containing heterocyclic compound]

The nitrogen-containing heterocyclic compound is a compound including at least one nitrogen atom as an atom

forming a heterocycle, and may be a monocyclic compound, or a polycyclic compound obtained by condensation of a heterocycle with another aromatic heterocycle or an aromatic carbocycle. In addition, a hetero atom other than nitrogen (a sulfur atom, an oxygen atom, or a second nitrogen atom) may be included in the ring. Examples of the nitrogen-containing heterocyclic compound include a 6-membered ring compound such as a pyridine, a pyrazine, or a pyrimidine, a 5-membered ring compound such as an imidazole, and a polycyclic compound such as a quinoline, an isoquinoline, and an acridine.

Among these, a pyridine is preferable as the nitrogen-containing heterocyclic compound. The pyridine is a substituted or unsubstituted pyridine. Examples of a substituent with which a hydrogen atom of pyridine can be replaced include an alkyl group, an alkoxy group, and a hydroxy group. One or more selected from the group consisting of unsubstituted pyridine ($C_5H_5N$), methylpyridine, methoxypyridine, and hydroxypyridine are preferable, and unsubstituted pyridine is more preferable.

When the mass ratio of the nitrogen-containing heterocyclic compound to the polycarbonate resin (mass of nitrogen-containing heterocyclic compound/mass of polycarbonate resin) is small, the degradation rate decreases, and thus the degradation time is lengthened and the efficiency tends to deteriorate. Because of this, the mass ratio is preferably 0.0001 or more, more preferably 0.0005 or more, and further preferably 0.001 or more. In addition, when the mass ratio is large, the excess nitrogen-containing heterocyclic compound inhibits the reaction of generating carbon dioxide, and thus the mass ratio is preferably 100 or less, more preferably 50 or less, and further preferably 10 or less.

[Acid]

Examples of the acid include an inorganic acid such as hydrochloric acid, sulfuric acid, or phosphoric acid, and an organic acid such as carboxylic acid or a sulfonic acid.

The acid is preferably any selected from the group consisting of hydrochloric acid, sulfuric acid, phosphoric acid, and a sulfonic acid. Examples of the sulfonic acid include an alkylsulfonic acid such as methanesulfonic acid and an aromatic sulfonic acid such as toluenesulfonic acid.

When the mass ratio of the acid to the polycarbonate resin (mass of acid/mass of polycarbonate resin) is small, the degradation rate decreases, and thus the degradation time is lengthened and the efficiency tends to deteriorate. Because of this, the mass ratio is preferably 0.01 or more, more preferably 0.05 or more, and further preferably 0.1 or more. In addition, when the mass ratio is large, the amount of a base required for neutralization tends to increase. Because of this, the mass ratio is preferably 20 or less, more preferably 10 or less, and further preferably 5 or less.

(Preparation of reaction liquid)

By stirring or the like a reaction liquid including a polycarbonate resin, an aromatic monoalcohol, water, and a catalyst, the polycarbonate resin can be degraded to generate a bisphenol or a salt thereof. The degradation reaction of the polycarbonate resin proceeds by using an aromatic monoalcohol and water in combination, without completely dissolving the polycarbonate resin. Because of this, the reaction liquid prepared may be a slurry-like reaction liquid (a reaction liquid in which the polycarbonate resin is dispersed in the liquid).

The slurry concentration of the reaction liquid (mass of solid in reaction liquid/mass of reaction liquid) is preferably 0.01 or more, and more preferably 0.05 or more. In addition, the slurry concentration is preferably 0.5 or less, and more preferably 0.3 or less. When the slurry concentration (concentration of solid) is too low, the degradation efficiency decreases, and when the slurry concentration is too high, poor mixing is likely to occur.

The liquid components in the reaction liquid prepared include the aromatic monoalcohol and water as the main components, and the total mass of the aromatic monoalcohol and water based on the mass of all liquid components is 0.8 or more, 0.9 or more, 0.95 or more, or the like.

The reaction liquid is prepared preferably at 10°C or more, and more preferably at 20°C or more. In addition, the reaction liquid is prepared preferably at 40°C or less, and more preferably at 35°C or less. When the temperature at the time of preparing the reaction liquid is too low, the aromatic monoalcohol is likely to solidify depending on the type thereof, and it may be easy for poor mixing to occur or it may be difficult to carry out uniform mixing. In addition, when the temperature at the time of preparing the reaction liquid is too high, the catalyst is likely to volatilize depending on the type thereof, and it may be difficult to prepare the reaction liquid to a predetermined concentration or the degradation reaction may run out of control.

The mixing order of a polycarbonate resin, an aromatic monoalcohol, water, and a catalyst is not particularly limited, and for example, the aromatic monoalcohol, water, and the catalyst may be sequentially supplied to the polycarbonate resin, or the polycarbonate resin, water, and the catalyst may be sequentially supplied to the aromatic monoalcohol. In order to allow more uniform mixing, the polycarbonate resin is preferably supplied after the aromatic monoalcohol and/or water.

As described above, the degradation reaction of the polycarbonate resin is carried out in the presence of an

aromatic monoalcohol, water, and a catalyst, and the polycarbonate resin can also be degraded in the coexistence of a component other than an aromatic monoalcohol, water, and a catalyst therewith as long as the object of the present invention is not inhibited. Also in this case, it is preferable to disperse the polycarbonate resin in a uniform solvent including an aromatic monoalcohol, water, a catalyst, and a further component to carry out the degradation reaction. Examples of the component that can coexist include an alkali metal chloride, a bromophenol, and carbon tetrachloride.

The alkali metal chloride is a salt of an alkali metal ion ($M^+$) and a chloride ion ($Cl^-$), and is a compound represented by MCl where M represents an alkali metal atom. Specific examples thereof include sodium chloride and potassium chloride, and sodium chloride is preferable. The amount of the alkali metal chloride is not particularly limited, and when the amount thereof is too large, the alkali metal chloride may precipitate to form scaling. Because of this, the mass ratio of the alkali metal chloride to water (mass of alkali metal chloride/mass of water) is preferably 0.2 or less, and more preferably 0.1 or less. In addition, when the amount thereof is too small, the effect of adding the alkali metal chloride is weakened, and when a wastewater is used, dilution or the like is required, and a large amount of water may be used to increase the load of wastewater treatment. Because of this, the mass ratio of the alkali metal chloride to water is preferably 0.00001 or more, and more preferably 0.0001 or more.

The bromophenol is a compound in which one hydroxy group is bonded and one or two bromine atoms are bonded to carbon forming an aromatic ring. In addition, the bromophenol may have a substituent other than a hydroxy group and a bromine atom. The bromophenol is preferably monobromophenol and/or dibromophenol. The amount of the bromophenol is not particularly limited, and when the amount thereof is too large, the bromophenol may precipitate. Because of this, the mass ratio of the bromophenol to water (mass of bromophenol/mass of water) is preferably 0.001 or less, and more preferably 0.0001 or less. When the amount thereof is too small, the effect of improving the efficiency of the degradation reaction of the polycarbonate resin is weakened, and thus the mass ratio of the bromophenol to water is preferably 0.0000001 or more, and more preferably 0.000001 or more.

The amount of carbon tetrachloride is not particularly limited, and when the amount thereof is too large, a problem is that the reaction liquid undergoes oil water separation and the polycarbonate resin cannot be efficiently degraded, and thus the mass ratio of carbon tetrachloride to water (mass of carbon tetrachloride/mass of carbon tetrachloride) is preferably 0.0005 or less, and more preferably 0.0001 or less. When the amount thereof is too small, the effect of improving the efficiency of the degradation reaction of the polycarbonate resin is weakened, and thus the mass ratio of carbon tetrachloride to water is preferably 0.0000001 or more, and more preferably 0.000001 or more.

Specifically, when the catalyst includes a nitrogen-containing heterocyclic compound, the polycarbonate resin can be degraded in the coexistence of an alkali metal chloride (preferably sodium chloride) in addition to an aromatic monoalcohol, water, and a catalyst. It is considered that the polycarbonate resin can be degraded more efficiently by the alkali metal chloride acting as a promoter.

When the catalyst includes hydrochloric acid, the polycarbonate resin can be degraded in the coexistence of a bromophenol in addition to an aromatic monoalcohol, water, and a catalyst. It is considered that the polycarbonate resin can be degraded more efficiently by the bromophenol acting as a promoter.

When the catalyst includes sodium hydroxide, the polycarbonate resin can be degraded in the coexistence of sodium chloride and/or carbon tetrachloride in addition to an aromatic monoalcohol, water, and a catalyst. It is considered that the polycarbonate resin can be degraded more efficiently by the coexistence of sodium chloride and/or carbon tetrachloride.

A wastewater discharged, for example, during the production of a diaryl carbonate can also be used in order to prepare the reaction liquid. In particular, in the degradation of the polycarbonate resin in the coexistence of an aromatic monoalcohol, water, a nitrogen-containing heterocyclic compound, and an alkali metal chloride, the degradation of the polycarbonate resin in the coexistence of an aromatic monoalcohol, water, hydrochloric acid, and a bromophenol, and the degradation of the polycarbonate resin in the coexistence of an aromatic monoalcohol, water, sodium hydroxide, sodium chloride, and carbon tetrachloride, the reaction liquid can be easily prepared and the environmental load can be reduced, by using a wastewater discharged from a production plant or the like, as will be described later.

(Degradation reaction)

The presence of an aromatic monoalcohol, water, and a catalyst cleaves a carbonate bond moiety of the polycarbonate resin to generate a bisphenol and carbon dioxide, or a salt of a bisphenol and a metal carbonate. By controlling the concentration of the polycarbonate resin, the temperature at the time of preparing a reaction liquid, or the like such that the degradation reaction does not proceed during the preparation of the reaction liquid (during the mixing of the polycarbonate resin, an aromatic monoalcohol, water, and a catalyst), a step of preparing a reaction liquid including a polycarbonate resin, an aromatic monoalcohol, water, and a catalyst and a step of degrading the polycarbonate resin in the reaction liquid may be clearly separated, or the step of preparing a reaction liquid and the step of degrading the polycarbonate resin may not be clearly separated. During the preparation of the reaction liquid, the degradation reaction of the polycarbonate resin may proceed, and part of the polycarbonate resin may be degraded. By degrading part of the

polycarbonate resin during the preparation of the reaction liquid, the degradation reaction can proceed more efficiently.

The degradation reaction may be carried out under normal pressure or under pressure, and the degradation reaction is preferably carried out under normal pressure because the reaction proceeds sufficiently even under normal pressure.

(Reaction temperature)

From the preparation of the reaction liquid to the stop of the degradation reaction, the temperature may be the same as the temperature at the time of preparing the reaction liquid, and after the reaction liquid is prepared (after a polycarbonate resin, an aromatic monoalcohol, water, and a catalyst are mixed), it is preferable to raise the temperature to a predetermined reaction temperature. When the temperature at the time of preparing the reaction liquid is too high, the degradation reaction may run out of control. It is preferable to raise the temperature after preparing the reaction liquid because the degradation reaction can proceed stably thereby.

The reaction temperature is appropriately selected depending on the type of the aromatic monoalcohol, the reaction time, or the like, and when the reaction temperature is high, water in the reaction liquid evaporates and the hydrolysis stops. In addition, when the temperature is low, the aromatic monoalcohol solidifies, the solvolysis is unlikely to proceed, or the reaction rate of hydrolysis decreases, and thus the time required for degradation is lengthened. Because of these, the reaction temperature is preferably 40°C or more, and more preferably 50°C or more, 60°C or more, 70°C or more, 75°C or more, or 80°C or more, which are listed in ascending order of preference. In addition, the reaction temperature is preferably 110°C or less, more preferably 100°C or less, and further preferably 95°C or less.

In particular, the degradation of the polycarbonate resin is preferably carried out at a reaction temperature of 40 to 110°C and under normal pressure, more preferably at a reaction temperature of 50 to 100°C and under normal pressure, and further preferably at a reaction temperature of 60 to 95°C and under normal pressure.

When the reaction is carried out at the same temperature as at the time of preparation of the reaction liquid, the reaction temperature is the average temperature from the time point when the mixing of a polycarbonate resin, an aromatic monoalcohol, water, and a catalyst is completed to the time point when the operation of neutralization or distillation for stopping the degradation reaction is started. In addition, when the reaction is carried out by raising the temperature after preparing the reaction liquid, the reaction temperature is the average temperature from the time point when the predetermined temperature is reached to the time point when the operation of neutralization or distillation operation for stopping the degradation reaction is started.

(Reaction time)

The reaction time is appropriately selected depending on the slurry concentration, the reaction temperature, or the like, and when the reaction time is long, the generated bisphenol tends to be degraded, and thus the reaction time is preferably within 30 hours, more preferably within 25 hours, and further preferably within 20 hours. In addition, when the reaction time is short, the degradation reaction may not proceed sufficiently, and thus the reaction time is preferably 0.1 hours or more, more preferably 0.5 hours or more, and further preferably 1 hour or more.

The reaction time is the time from the time point when the mixing of a polycarbonate resin, an aromatic monoalcohol, water, and a catalyst is completed to the time point when the operation of neutralization or distillation for stopping the degradation reaction is started. The end point of the reaction time may be determined by tracking the degradation reaction by liquid chromatography or the like.

(Method for stopping degradation reaction of polycarbonate resin)

The method for stopping the degradation reaction of the polycarbonate resin is appropriately selected depending on the type of the catalyst used. When an alkali metal hydroxide, an alkali metal carbonate, or an acid is used as the catalyst, the degradation reaction can be stopped by neutralization or the like. In addition, when an alkylamine or a nitrogen-containing heterocyclic compound is used as the catalyst, the degradation reaction can be stopped by distilling off or neutralizing the alkylamine or the nitrogen-containing heterocyclic compound. In a method for removing an alkylamine or a nitrogen-containing heterocyclic compound by neutralization by supplying an acid, an ammonium salt or the like is generated and also needs to be removed, and thus the alkylamine or the nitrogen-containing heterocyclic compound is removed preferably by a method for distilling off the same.

(Method for recovering and purifying bisphenol)

The obtained bisphenol can be recovered and purified by a conventional method. For example, the obtained bisphenol can be recovered and purified by simple means such as crystallization or column chromatography. Specifically,

after the degradation reaction of the polycarbonate resin, the catalyst and the solvent are removed and an organic solvent is mixed, the resulting organic phase is washed with water, saline, or the like, and further, if necessary, neutralized and washed with aqueous ammonium chloride or the like. Next, the washed organic phase is cooled and subjected to crystallization.

Examples of the organic solvent that can be used include an aromatic hydrocarbon such as toluene, xylene, ethylbenzene, diethylbenzene, isopropylbenzene, or mesitylene, an aliphatic hydrocarbon such as hexane, heptane, octane, nonane, decane, undecane, or dodecane, and an aliphatic alcohol such as methanol, ethanol, n-propanol, i-propanol, n-butanol, i-butanol, t-butanol, n-pentanol, i-pentanol, n-hexanol, n-heptanol, n-octanol, n-nonanol, n-decanol, n-undecanol, n-dodecanol, ethylene glycol, diethylene glycol, or triethylene glycol.

Before the crystallization, the excess aromatic monoalcohol or the organic solvent may be distilled off by distillation before crystallization. In addition, bisphenol A forms a co-crystal with phenol when crystallized in the presence of phenol. When a polycarbonate resin containing a constitutional unit derived from bisphenol A is degraded by using phenol, it is necessary to distill off phenol before crystallization in order not to form a co-crystal.

(Method for recovering and purifying carbon dioxide)

In the method for producing a bisphenol according to the present invention, the generated carbon dioxide may be recovered and purified. Purification of carbon dioxide can be carried out by a conventional method. For example, a physical absorption method, a chemical absorption method, a cryogenic separation method, a membrane separation method, or a pressure swing adsorption method can be applied, and can be appropriately selected according to the impurity in carbon dioxide generated at the time of neutralization.

Hereinafter, by taking methods (A) to (C) for producing a bisphenol including producing bisphenol A from a polycarbonate resin containing a constitutional unit derived from 2,2-bis(4-hydroxyphenyl)propane (bisphenol A) as an example, the method for producing a bisphenol according to the present invention will be described more specifically.

<Method (A) for producing bisphenol>

The method (A) for producing a bisphenol includes a step (A1) of, in a reaction liquid including a polycarbonate resin containing a constitutional unit derived from bisphenol A, phenol, water, and an alkali metal hydroxide, degrading the polycarbonate resin, a step (A2) of neutralizing the reaction liquid after the step (A1) to obtain an organic phase in which bisphenol A is dissolved, and a step (A3) of depressurizing and/or heating the organic phase obtained in the step (A2) and then recovering bisphenol A by crystallization. The method (A) for producing a bisphenol is an example of the method for producing a bisphenol according to the present invention when phenol is used as the aromatic monoalcohol and an alkali metal hydroxide is used as the catalyst.

The main degradation reaction is carried out according to the reaction equation (2) shown below, and the main degradation products obtained in the step (A1) are an alkali metal salt of a bisphenol and an alkali metal carbonate, n in the following reaction equation (2) is 2 to 1000.

[Formula 4]

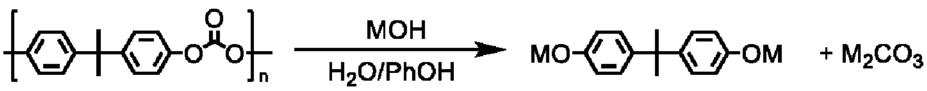

···(2)

In the method (A) for producing a bisphenol, in order to generate bisphenol A from the alkali metal salt of bisphenol A and carbon dioxide from the alkali metal carbonate, the step (A2) of neutralizing the reaction liquid after the step (A1) to obtain an organic phase in which bisphenol A is dissolved is carried out.

The neutralization is carried out by mixing an acid into the reaction liquid. Examples of the acid used include hydrochloric acid, sulfuric acid, and phosphoric acid. In the neutralization by mixing an acid, the pH of the reaction liquid may be less than 7 or more than 7, and when the pH is less than 7, the quality of bisphenol A isolated may decrease. Because of this, it is preferable to mix an acid such that the end point is where the pH of the reaction liquid is more than 7 (for example, pH 7.5 or more or pH 8.0 or more). On the other hand, when the pH of the reaction liquid is too high, a bisphenol and carbon dioxide are unlikely to be generated, and thus an acid is mixed such that the pH of the reaction liquid is 10 or less, and the pH is preferably 9.5 or less.

Further, by allowing a mixed liquid of the reaction liquid and an acid to stand, it is possible to carry out oil water separation of the mixed liquid into an organic phase (phenol phase) in which the generated bisphenol A is dissolved and an aqueous phase in which the alkali metal hydroxide and the like (the alkali metal hydroxide, the acid added for neutralization, and a salt generated by the neutralization) are dissolved, and thus if the aqueous phase is removed, the

alkali metal hydroxide and the like can be removed.

In addition, an organic solvent such as an aromatic hydrocarbon may be mixed before or after mixing an acid. An acid and an organic solvent are mixed into the reaction liquid to neutralize the reaction liquid, then oil water separation thereof is carried out, and the aqueous phase is removed to obtain an organic phase in which bisphenol A is dissolved (phase of phenol and the organic solvent). By mixing an organic solvent, it becomes easy to carry out oil water separation, and thus it becomes easier to remove the aqueous phase in which the alkali metal hydroxide and the like are dissolved.

In the step (A3), the organic phase obtained in the step (A2) is depressurized and/or heated, and then bisphenol A is recovered by crystallization. When bisphenol A is present at the time of crystallization, bisphenol A forms a co-crystal with phenol and the co-crystal precipitates, and thus in order to obtain bisphenol A, phenol is removed before crystallization in the step (A3).

Specifically, the organic phase obtained in the step (A2) is depressurized and/or heated to distill off a liquid component such as phenol. Next, an organic solvent such as an aromatic hydrocarbon is added to prepare a solution for crystallization in which bisphenol A is dissolved, and then this is cooled to precipitate bisphenol A. The precipitated bisphenol A is recovered by solid liquid separation.

In addition, even when an alkali metal carbonate is used as the catalyst, it is possible to carry out the same method as the method (A) for producing a bisphenol.

<Method (B) for producing bisphenol>

The method (B) for producing a bisphenol includes a step (B1) of, in a reaction liquid including a polycarbonate resin containing a constitutional unit derived from bisphenol A, phenol, water, and an alkylamine, degrading the polycarbonate resin, and a step (B2) of depressurizing and/or heating the reaction liquid after the step (B1) and then recovering bisphenol A by crystallization. The method (B) for producing a bisphenol is an example of the method for producing a bisphenol according to the present invention when phenol is used as the aromatic monoalcohol and an alkylamine is used as the catalyst.

The main degradation reaction is carried out according to the reaction equation (3) shown below, and the main degradation products obtained in the step (B1) are a bisphenol and carbon dioxide, n in the following reaction equation (3) is 2 to 1000.

[Formula 5]

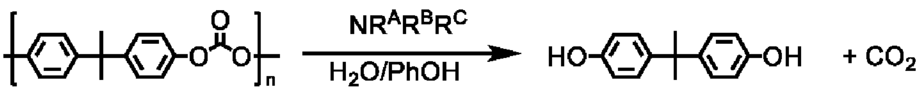


$+ CO_2$ ···(3)

In the method (B) for producing a bisphenol, the reaction liquid after the step (B1) is depressurized and/or heated, and then the step (B2) of recovering bisphenol A by crystallization is carried out. Specifically, the reaction liquid after the step (B1) is depressurized and/or heated to distill off a liquid component such as an alkylamine or phenol. Next, an organic solvent such as an aromatic hydrocarbon is added to prepare a solution for crystallization in which bisphenol A is dissolved, and then this is cooled to precipitate bisphenol A. The precipitated bisphenol A is recovered by solid liquid separation.

In addition, when an alkylamine is used as the catalyst, the alkylamine may be removed by a method involving supplying an acid for neutralization. In this case, as in the step (A2) of the method (A) for producing a bisphenol, an acid is mixed into the reaction liquid after the degradation reaction to neutralize the reaction liquid, and then oil water separation thereof is carried out, and the aqueous phase is removed to obtain an organic phase in which bisphenol A is dissolved. Next, as in the step (A3) of the method (A) for producing a bisphenol, the obtained organic phase is depressurized and/or heated, and bisphenol A can be recovered by crystallization.

As described above, examples of the method for removing the alkylamine from the degradation reaction liquid of the polycarbonate resin include a method involving distilling off the alkylamine and a method involving supplying an acid for neutralization. In the method involving supplying an acid for neutralization, an ammonium salt is generated and also needs to be removed, and thus the method involving distilling off the alkylamine is preferable. By using an alkylamine as the catalyst, the alkylamine can be removed together with phenol by depressurization and/or heating, and the neutralization is not essential, and thus the purification can be simplified.

In addition, even when a nitrogen-containing heterocyclic compound is used as the catalyst, it is possible to carry out the same method as the method (A) for producing a bisphenol or the method (B) for producing a bisphenol.

<Method (C) for producing bisphenol>

The method (C) for producing a bisphenol includes a step (C1) of, in a reaction liquid including a polycarbonate resin containing a constitutional unit derived from bisphenol A, phenol, water, and an acid, degrading the polycarbonate resin, a step (C2) of neutralizing the reaction liquid after the step (C1) to obtain an organic phase in which bisphenol A is dissolved, and a step (C3) of depressurizing and/or heating the organic phase obtained in the step (C2) and then recovering bisphenol A by crystallization. The method (C) for producing a bisphenol is an example of the method for producing a bisphenol according to the present invention when phenol is used as the aromatic monoalcohol and an acid is used as the catalyst.

The main degradation reaction is carried out according to the reaction equation (4) shown below, and the main degradation products obtained in the step (C1) are a bisphenol and carbon dioxide, n in the following reaction equation (4) is defined as in the formula (1). n in the following reaction equation (4) is 2 to 1000.

[Formula 6]

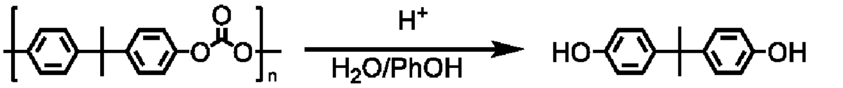


$+ CO_2$ ···(4)

In the method (C) for producing a bisphenol, after the step (C1), the step (C2) of neutralizing the reaction liquid to obtain an organic phase in which bisphenol A is dissolved is carried out. The neutralization is carried out by mixing a base into the reaction liquid. Examples of the base used include sodium carbonate and sodium hydroxide.

As in the step (A2) of the method (A) for producing a bisphenol, the neutralization is preferably carried out such that the end point is where the pH of the reaction liquid is more than 7. For example, it is preferable to mix a base such that the pH is 7.5 or more or the pH is 8.0 or more. In addition, it is preferable to mix a base such that the pH is 10 or less or 9.5 or less.

In the step (C2), as in the step (A2) of the method (A) for producing a bisphenol, a mixed liquid of the reaction liquid and a base or a mixed liquid of the reaction liquid, a base, and an organic solvent is subjected to oil water separation, and the aqueous phase is removed to obtain an organic phase in which bisphenol A is dissolved.

In the step (C3), bisphenol A is recovered from the organic phase in which bisphenol A is dissolved obtained in the step (C2). As in the step (A3) of the method (A) for producing a bisphenol, the organic phase obtained in the step (C2) is depressurized and/or heated, and then bisphenol A can be recovered by crystallization.

The methods (A) to (C) for producing a bisphenol are examples in which phenol is used as the aromatic monoalcohol, and when an aromatic monoalcohol other than phenol such as a cresol or a xylenol is used as the aromatic monoalcohol, bisphenol A does not form a co-crystal, and thus it is not essential to remove the aromatic monoalcohol by depressurization and/or heating in the step (A3), the step (B2), and the step (C3). In this case, bisphenol A can be recovered by cooling the organic phase obtained in the step (A2) or the step (C2) or the reaction liquid after the step (B1) to precipitate bisphenol A. Purification of bisphenol A can be simplified by using a cresol or a xylenol.

In addition, bisphenol A may be recovered as a co-crystal of bisphenol A and phenol. In this case, bisphenol A is recovered by cooling the organic phase obtained in the step (A2) or the step (C2) or the reaction liquid after the step (B1) without distilling off phenol to precipitate a co-crystal of bisphenol A and phenol.

In addition, as described above, the polycarbonate resin used in the method for producing a bisphenol according to the present invention is not limited to a polycarbonate resin containing a constitutional unit derived from bisphenol A. The method for producing a bisphenol according to the present invention by using a polycarbonate resin containing a constitutional unit derived from a bisphenol other than bisphenol A can also be appropriately carried out in the same manner as in the above methods (A) to (C) for producing a bisphenol.

<Method for producing bisphenol by using wastewater>

In the method for producing a bisphenol according to the present invention, as described above, a wastewater discharged from a diaryl carbonate production plant or the like can be used for preparing the reaction liquid. By using a wastewater, the wastewater can be effectively used, and a production method having a smaller environmental load can be provided.

For example, a neutralized wastewater discharged from a neutralization treatment facility of a diaryl carbonate production plant can be used for degrading the polycarbonate resin. In general, a diaryl carbonate is produced by a method including the following step (a1), step (b1), step (b2), and step (b3), and the step (b1) is a neutralization treatment.

Step (a1): A step of reacting carbonyl chloride with an aromatic monoalcohol in the presence of a nitrogen-containing

heterocyclic compound to obtain a reaction liquid including a diaryl carbonate

Step (b1): A step of neutralizing the reaction liquid including a diaryl carbonate obtained in the step (a1) with an alkali metal hydroxide aqueous solution, carrying out oil water separation thereof into an oil phase including an aromatic diaryl and an aqueous phase including the nitrogen-containing heterocyclic compound and an alkali metal chloride, and then removing the aqueous phase as a neutralized wastewater

Step (b2): A step of washing the oil phase obtained in the step (b1) with water

Step (b3): A step of obtaining the diaryl carbonate from the oil phase after the step (b2).

In the method for producing a bisphenol according to the present invention, the neutralized wastewater (aqueous phase) removed in this step (b1) can be used for degrading the polycarbonate resin. The aromatic monoalcohol is included not only in the oil phase but also in the aqueous phase, and thus the neutralized wastewater discharged in the step (b1) is water including the aromatic monoalcohol, the nitrogen-containing heterocyclic compound, and the alkali metal chloride (neutralization salt). Because of this, by mixing the polycarbonate resin and the neutralized wastewater discharged in the step (b1), a reaction liquid including the polycarbonate resin, the aromatic monoalcohol, water, the nitrogen-containing heterocyclic compound, and the alkali metal chloride can be easily prepared.

In the step (a1), the aromatic monoalcohol is the same as the aromatic monoalcohol used for degrading the polycarbonate resin. The aromatic monoalcohol used for degrading the polycarbonate resin and the aromatic monoalcohol in the step (a1) are preferably the same. In addition, in the step (a1), the nitrogen-containing heterocyclic compound is the same as the nitrogen-containing heterocyclic compound that can be used for degrading the polycarbonate resin, and is preferably a pyridine.

In addition, the alkali metal hydroxide aqueous solution used in the step (b1) is a solution obtained by dissolving the alkali metal hydroxide in water. The alkali metal hydroxide is the same as the alkali metal hydroxide that can be used for degrading the polycarbonate resin. The step (b1) is preferably a step of neutralizing the reaction liquid including a diaryl carbonate obtained in the step (a1) with a sodium hydroxide aqueous solution, carrying out oil water separation thereof into an oil phase including an aromatic diaryl and an aqueous phase including the nitrogen-containing heterocyclic compound and sodium chloride, and then removing the aqueous phase as a neutralized wastewater, and more preferably a step of neutralizing the reaction liquid including a diaryl carbonate obtained in the step (a1) with a sodium hydroxide aqueous solution, carrying out oil water separation thereof into an oil phase including an aromatic diaryl and an aqueous phase including a pyridine and sodium chloride, and then removing the aqueous phase as a neutralized wastewater.

In addition, hydrochloric acid wastewater discharged when recovering hydrogen chloride by-produced when producing a diaryl carbonate from carbonyl chloride and an aromatic monoalcohol can be used for degrading the polycarbonate resin. For example, hydrochloric acid wastewater discharged from a hydrogen chloride recovery facility installed additionally in a diaryl carbonate production plant can be used. In general, the production of a diaryl carbonate and the recovery of hydrogen chloride by-produced include the following step (a1), step (c1), step (c2), and step (c3) .

Step (a1): A step of reacting carbonyl chloride with an aromatic monoalcohol in the presence of a nitrogen-containing heterocyclic compound to obtain a reaction liquid including a diaryl carbonate

Step (c1): A step of supplying hydrogen chloride by-produced in the step (a1) to an absorption column and absorbing the same into water or dilute hydrochloric acid to obtain concentrated hydrochloric acid

Step (c2): A step of distilling the concentrated hydrochloric acid in a stripping column, recovering hydrogen chloride gas from the top of the column, and recovering hydrochloric acid from the bottom of the column

Step (c3): A step of removing part of the hydrochloric acid recovered from the bottom of the column as hydrochloric acid wastewater outside a system and circulating the remaining hydrochloric acid to the absorption column of the step (c1).

In the method for producing a bisphenol according to the present invention, the hydrochloric acid wastewater (aqueous phase) removed in this step (c3) can be used for degrading the polycarbonate resin. The hydrochloric acid wastewater discharged in the step (c3) includes a bromophenol generated by a side reaction between a small amount of Cl-Br included in carbonyl chloride used as a raw material in the step (a1) and the aromatic monoalcohol. Because of this, by mixing the polycarbonate resin, the aromatic monoalcohol, and the hydrochloric acid wastewater discharged in the step (c3), a reaction liquid including the polycarbonate resin, the aromatic monoalcohol, water, hydrochloric acid, and a bromophenol can be easily prepared.

In addition, the sodium hydroxide wastewater discharged in the detoxification treatment of the unliquefied gas generated during the production of carbonyl chloride, which is a raw material of a diaryl carbonate, can be used for degrading the polycarbonate resin. For example, the sodium hydroxide wastewater discharged from an unliquefied gas detoxification column (detoxification treatment facility) provided in a carbonyl chloride production plant can be used. In general, the production of carbonyl chloride and the treatment of an unliquefied gas include the following step (d1), step (d2), step (d3), and step (d4) .

Step (d1): A step of obtaining carbonyl chloride gas from chlorine and carbon monoxide
Step (d2): A step of cooling the carbonyl chloride gas obtained in the step (d1) to obtain liquefied carbonyl chloride
Step (d3): A step of contacting a circulating sodium hydroxide aqueous solution with an unliquefied gas that has not been liquefied in the (d2) to degrade carbonyl chloride in the unliquefied gas, and then discharging the resulting unliquefied gas
Step (d4): A step of removing part of the circulating sodium oxide aqueous solution as sodium hydroxide wastewater.

In the method for producing a bisphenol according to the present invention, the sodium hydroxide wastewater (aqueous phase) removed in this step (d4) can be used for degrading the polycarbonate resin. The sodium hydroxide wastewater discharged in the step (d3) includes sodium chloride generated by neutralization and carbon tetrachloride as a by-product. Because of this, by mixing the polycarbonate resin, the aromatic monoalcohol, and the sodium hydroxide wastewater discharged in the step (d4), a reaction liquid including the polycarbonate resin, the aromatic monoalcohol, water, sodium hydroxide, sodium chloride, and carbon tetrachloride can be easily prepared.

Hereinafter, methods (R1) to (R3) for producing a bisphenol by using a wastewater will be specifically described with reference to Figure 1 to Figure 3.

<Method (R1) for producing bisphenol by using wastewater>

Figure 1 is a flow diagram for describing an example of a method for producing a bisphenol by using a neutralized wastewater discharged from a diphenyl carbonate production plant.

(Production of diphenyl carbonate)

In a diphenyl carbonate production plant 1 shown in Figure 1, first, carbonyl chloride gas (CDC, G1) and phenol (PL) are reacted with each other in the presence of pyridine (PRD) in a DPC reactor 10 to obtain a reaction liquid (L10) including diphenyl carbonate (step (a1)). Hydrogen chloride gas (G10) by-produced at this time is sent from the upper part of the DPC reactor 10 to an activated carbon column (not shown).

The reaction liquid (L10) including diphenyl carbonate is sent from the DPC reactor 10 to a dehydrochlorination column 11, and dehydrochlorination treatment is carried out. Hydrogen chloride gas (G12) generated in the dehydrochlorination column 11 is sent to the activated carbon column (not shown) as is the hydrogen chloride gas (G10) by-produced in the DPC reactor 10.

A reaction liquid (L11) after the dehydrochlorination treatment is sent to a mixing tank 12 and next, to a neutralization tank 13. In the neutralization tank 13, hydrochloric acid that has not been able to be completely removed by the dehydrochlorination column 11 is neutralized with an aqueous sodium hydroxide solution (L12), and then oil water separation thereof is carried out. After the oil water separation, an aqueous phase (L14) is discharged as a neutralized wastewater, and an obtained oil phase (L13) is sent to a water washing tank 14 (step (b1)). This neutralized wastewater (aqueous phase (L14)) includes water, phenol, pyridine, and sodium chloride.

In the water washing tank 14, water washing treatment with water (L15) is carried out (step (b2)). The oil phase (L13) sent to the water washing tank 14 and water (L15) are mixed with each other and an aqueous phase (L17) is removed to obtain an oil phase (L16). The oil phase (L16) subjected to water washing treatment is sent sequentially to a distillation column 15 and to a distillation column 16, distillation is carried out, and purified diphenyl carbonate (G13) is recovered from the top of the distillation column 16 (step (b3)).

(Production of bisphenol)

The aqueous phase (L14, neutralized wastewater) discharged from the neutralization tank 13 is sent to a degradation tank 100 and mixed with a bisphenol A type polycarbonate resin (PC) to degrade the polycarbonate resin. In addition, the reaction liquid prepared in the degradation tank 100 may include the aqueous phase (L14, neutralized wastewater) in at least part thereof. Therefore, in addition to the aqueous phase (L14, neutralized wastewater), water ($H_2O$) or phenol (PL), which is not the neutralized wastewater (L14), can also be separately supplied to the degradation tank 100 to degrade the polycarbonate resin. In addition, a base may be separately supplied as a catalyst. By making it possible to supply water, phenol, and a base separately, the ratio of water and phenol or the amount of the catalyst can be appropriately adjusted. As the base additionally supplied at this time, pyridine used as a catalyst in the production of diphenyl carbonate is preferable. A solution (L100) including bisphenol A after PC degradation is sent to a facility that carries out a next step such as catalyst distillation or neutralization, and bisphenol A is recovered (not shown).

<Method (R2) for producing bisphenol by using wastewater>

Figure 2 is a flow diagram for describing an example of a method for producing a bisphenol by using hydrochloric acid wastewater discharged from a recovery plant 2 for hydrogen chloride by-produced during the production of diphenyl carbonate.

(Recovery of chlorine)

The hydrogen chloride gas (G10, G12) by-produced in a DPC reactor 10 is sent to an activated carbon column 20, and an organic impurity such as phenol is adsorbed and removed. Hydrogen chloride gas (G20) treated with activated carbon is sent to an absorption column 21. The hydrogen chloride gas (G20) is absorbed into water (L20) or dilute hydrochloric acid (L21, an unsaturated aqueous solution of hydrogen chloride) supplied to the absorption column 21, discharged as concentrated hydrochloric acid (L23), and stored in a tank 22 (step (c1)). Next, the concentrated hydrochloric acid (L23) is sent to a stripping column 23 and distilled, and high-purity hydrogen chloride gas (G21) is distilled out from the top of the column. In addition, dilute hydrochloric acid (L24) is discharged from the bottom of the stripping column 23 and stored in a tank 24 (step (c2)). In the dilute hydrochloric acid (L24) stored in the tank 24, a certain amount of the dilute hydrochloric acid (L24) is discharged from the tank 24 as hydrochloric acid wastewater (L25) in order to prevent the concentration of an impurity, and a remaining dilute hydrochloric acid (L26) is returned to the absorption column 21 (step (c3)). The hydrochloric acid wastewater (L25) includes water, hydrogen chloride, and a bromophenol (monobromophenol and/or dibromophenol).

(Production of bisphenol)

The hydrochloric acid wastewater (L25) discharged from the tank 24 is sent to a degradation tank 102 and mixed with a bisphenol A type polycarbonate resin (PC) and phenol (PL) to degrade the polycarbonate resin. In addition, the reaction liquid prepared in the degradation tank 102 may include the hydrochloric acid wastewater (L25) in at least part thereof. Therefore, in addition to the hydrochloric acid wastewater (L25), water ($H_2O$), which is not the hydrochloric acid wastewater (L25), can also be separately supplied to the degradation tank 102 to degrade the polycarbonate resin. In addition, an acid may be separately supplied as a catalyst. As the acid additionally supplied at this time, hydrochloric acid is preferable. By making it possible to supply water and an acid separately, the amount of the catalyst can be appropriately adjusted. A solution (L102) including bisphenol A after PC degradation is sent to a facility that carries out a next step such as catalyst distillation or neutralization, and bisphenol A is recovered (not shown).

<Method (R3) for producing bisphenol by using wastewater>

Figure 3 is a flow diagram for describing an example of a method for producing a bisphenol by using sodium hydroxide wastewater discharged from a detoxification column 34 provided in a carbonyl chloride production plant 3.

(Production of carbonyl chloride and treatment of unliquefied gas)

In the carbonyl chloride production plant 3, first, carbon monoxide gas (CO) and chlorine gas ($CL_2$) are supplied to a CDC reactor 30 filled with granular activated carbon (catalyst) to obtain crude carbonyl chloride gas (G30) (step (d1)). The obtained crude carbonyl chloride gas (G30) is sent to an aggregator 31 and cooled with brine, and liquefied carbonyl chloride (L30) is stored in a tank 32 (step (d2)). The carbonyl chloride (L30) stored in the tank 32 is evaporated by an evaporator 33 and used as carbonyl chloride gas (G1) in a diphenyl carbonate production plant or the like (not shown).

In addition, an unliquefied gas (G31) that has not been liquefied by a condenser 31 is supplied to the detoxification column 34 through which a sodium hydroxide aqueous solution (L31) is circulated, and the unliquefied gas (G31) is contacted with the sodium hydroxide aqueous solution (L31) to degrade carbonyl chloride in the unliquefied gas (G31) and then is discharged into the atmosphere as a waste gas (G32) (step (d3)). In addition, in the detoxification column 34, in order to prevent the concentration of an impurity or the like, part of the circulating sodium hydroxide (L31) is discharged as sodium hydroxide wastewater (L32) (step (d4)). The sodium hydroxide wastewater (L32) includes water, sodium hydroxide, sodium chloride, and carbon tetrachloride.

(Production of bisphenol)

The sodium hydroxide wastewater (L32) discharged from the detoxification column 34 is sent to a degradation tank 103 and mixed with a bisphenol A type polycarbonate resin (PC) and phenol (PL) to degrade the polycarbonate

resin. In addition, the reaction liquid prepared in the degradation tank 103 may include the sodium hydroxide wastewater (L32) in at least part thereof. Therefore, in addition to the sodium hydroxide wastewater (L32), water ($H_2O$), which is not the sodium hydroxide wastewater (L32), can also be separately supplied to the degradation tank 103 to degrade the polycarbonate resin. In addition, a base may be separately supplied as a catalyst. As the base additionally supplied at this time, sodium hydroxide is preferable. By making it possible to supply water and a catalyst separately, the amount of the catalyst can be appropriately adjusted. A solution (L103) including bisphenol A after PC degradation is sent to a facility that carries out a next step such as catalyst distillation or neutralization, and bisphenol A is recovered (not shown).

<Application of bisphenol>

The bisphenol obtained by the method for producing a bisphenol according to the present invention (hereinafter, sometimes referred to as a "recycled bisphenol") can be used as a constituent component of various thermoplastic resins such as a polyether resin, a polyester resin, a polyarylate resin, a polycarbonate resin, a polyurethane resin, or an acrylic resin, various thermosetting resins such as an epoxy resin, an unsaturated polyester resin, a phenol resin, a polybenzoxazine resin, or a cyanate resin, which are used in various applications such as an optical material, a recording material, an insulating material, a transparent material, an electronic material, an adhesive material, or a heat resistant material, or the like, a curing agent, an additive, a precursor thereof, or the like. In addition, the bisphenol is also useful as an additive such as a color developer for a heat sensitive recording material or the like, a fading inhibitor, a microbicidal agent, or an antimicrobial or antifungal agent.

The bisphenol is preferably used as a raw material (monomer) for a thermoplastic resin or a thermosetting resin among these because it can impart a good mechanical physical property, and more preferably used as a raw material for a polycarbonate resin or an epoxy resin among such resins. In addition, the bisphenol is also preferably used as a color developer, and is more preferably used particularly in combination with a leuco dye or a discoloration temperature adjusting agent.

<Method for producing carbon dioxide>

The present invention relates to a method for producing carbon dioxide including recovering carbon dioxide generated by the method for producing a bisphenol according to the present invention (hereinafter, sometimes referred to as the "method for producing carbon dioxide according to the present invention"). According to the method for producing carbon dioxide according to the present invention, carbon dioxide can be obtained by efficiently degrading the polycarbonate resin under a condition that is mild and has a small environmental load. As described above, in the method for producing a bisphenol according to the present invention, carbon dioxide is generated by the degradation reaction of the polycarbonate resin and/or neutralization after the degradation reaction. This carbon dioxide can be recovered and used as a raw material for a carbonic acid diester such as dimethyl carbonate or diphenyl carbonate, a raw material for an alkylene carbonate such as ethylene carbonate, a raw material for carbon monoxide, or the like.

Specifically, when the catalyst used for degrading a polycarbonate resin is a base, the method for producing carbon dioxide according to the present invention can include: a step of degrading a polycarbonate resin in the presence of an aromatic monoalcohol, water, and a base (catalyst); and a step of recovering carbon dioxide generated by the degradation of the polycarbonate resin.

In addition, when the catalyst used for degrading a polycarbonate resin is a base, carbon dioxide is also generated during the neutralization after the degradation reaction, and thus the method for producing carbon dioxide according to the present invention may include: a step of degrading a polycarbonate resin in the presence of an aromatic monoalcohol, water, and the base (catalyst); a step of neutralizing a reaction liquid in which the polycarbonate resin has been degraded; and a step of recovering carbon dioxide generated by the degradation of the polycarbonate resin and/or the neutralization.

When the catalyst used for degrading a polycarbonate resin is an acid, the method for producing carbon dioxide according to the present invention can include: a step of degrading a polycarbonate resin in the presence of an aromatic monoalcohol, water, and the acid (catalyst); and a step of recovering carbon dioxide generated by the degradation of the polycarbonate resin.

As described above, carbon dioxide can be recovered by a conventional method, and the method can be appropriately selected according to another impurity. For example, a physical absorption method, a chemical absorption method, a cryogenic separation method, a membrane separation method, a pressure swing adsorption method, or the like can be applied.

<Method for producing carbonic acid diester>

The present invention relates to a method for producing a carbonic acid diester, including producing a carbonic

acid diester by using carbon dioxide obtained by the method for producing carbon dioxide according to the present invention (hereinafter, sometimes referred to as "recycled carbon dioxide") (hereinafter, sometimes referred to as the "method for producing a carbonic acid diester according to the present invention").

As the method for producing a carbonic acid diester according to the present invention, a known method for producing a carbonic acid diester by using carbon dioxide as a raw material can be used. In addition, recycled carbon dioxide may be used as at least part of carbon dioxide as a raw material, and the content of recycled carbon dioxide in carbon dioxide is not particularly limited. For example, the content of recycled carbon dioxide in carbon dioxide is preferably 0.1% by volume or more, and more preferably 0.5% by volume or more.

By the method for producing a carbonic acid diester according to the present invention, a dialkyl carbonate such as dimethyl carbonate, a diaryl carbonate such as diphenyl carbonate, or the like can be produced. The carbonic acid diester obtained by the method for producing a carbonic acid diester according to the present invention can be used as a raw material for a polycarbonate resin, an electrolytic solution, or the like.

For example, as the method for producing a carbonic acid diester according to the present invention, the method for producing a dialkyl carbonate according to the following (M1), the method for producing a diaryl carbonate according to the following

(M2) or (M3), or the like can be used.

(M1) A method involving reacting carbon dioxide with an aliphatic monoalcohol to obtain a dialkyl carbonate

(M2) A method involving reacting carbon dioxide with an aliphatic monoalcohol to obtain a dialkyl carbonate, and reacting the obtained dialkyl carbonate with an aromatic monoalcohol to obtain a diaryl carbonate

(M3) A method involving obtaining carbon monoxide from carbon dioxide and coke, reacting the obtained carbon monoxide with chlorine to obtain carbonyl chloride, and reacting the obtained carbonyl chloride with an aromatic monoalcohol to obtain a diaryl carbonate

Examples of the aliphatic monoalcohol include an alcohol having 1 to 10 carbon atoms. An alcohol having 1 to 6 carbon atoms such as methanol, ethanol, propanol, isopropanol, butanol, pentanol, or hexanol is preferable, and methanol or butanol is more preferable.

As the aromatic monoalcohol, the same aromatic monoalcohol as used in the method for producing a bisphenol according to the present invention can be used, and phenol is preferable.

In addition, each reaction may be carried out in the presence of a known catalyst. Carbon dioxide may be reacted with an aliphatic monoalcohol in the presence of a catalyst, and examples of the catalyst include a known catalyst such as cerium oxide. As the catalyst used for the reaction between a dialkyl carbonate and an aromatic monoalcohol, for example, an organotitanium catalyst such as tetraphenoxytitanium can be used.

Above all, as described in (M1) or (M2) above, the method for producing a carbonic acid diester according to the present invention is preferably a method including a step of reacting carbon dioxide including carbon dioxide obtained by the method for producing carbon dioxide according to the present invention with an aliphatic monoalcohol.

<Method for producing recycled polycarbonate resin>

The method for producing a recycled polycarbonate resin according to the present invention is a method for producing a recycled polycarbonate resin by using a carbonic acid diester raw material including a bisphenol raw material including a bisphenol (recycled bisphenol) obtained by the method for producing a bisphenol according to the present invention, and/or a carbonic acid diester obtained by the method for producing a carbonic acid diester according to the present invention (hereinafter, sometimes referred to as a "recycled carbonic acid diester").

Hereinafter, the method for producing a recycled polycarbonate resin by using a bisphenol raw material including a recycled bisphenol will be described as a "first method for producing a recycled polycarbonate resin," and the method for producing a recycled polycarbonate resin by using a carbonic acid diester raw material including a recycled carbonic acid diester will be described as a "second method for producing a recycled polycarbonate resin." In addition, the "first method for producing a recycled polycarbonate resin" and the "second method for producing a recycled polycarbonate resin" are collectively referred as "the method for producing a recycled polycarbonate resin according to the present invention."

<First method for producing recycled polycarbonate resin>

The first method for producing a recycled polycarbonate resin is a method for producing a recycled polycarbonate resin, including producing a recycled polycarbonate resin by using a bisphenol raw material including a bisphenol (recycled bisphenol) obtained by the method for producing a bisphenol according to the present invention. The first method for producing a recycled polycarbonate resin uses a chemical recycling method involving producing a polycarbonate resin

by using, as a raw material, a recycled bisphenol obtained by degrading a polycarbonate resin included in a waste plastic or the like into a bisphenol as a monomer.

The recycled polycarbonate resin produced by the first method for producing a recycled polycarbonate resin can be obtained by using a known method for producing a polycarbonate resin by using a bisphenol as a raw material. For example, the recycled polycarbonate resin can be obtained by polymerizing a bisphenol raw material including a recycled bisphenol (bisphenol obtained by degrading a polycarbonate resin by the method for producing a bisphenol according to the present invention) and a carbonic acid diester raw material. The polymerization can be carried out by appropriately selecting a known method.

The recycled polycarbonate resin can be produced, for example, by a method involving subjecting a bisphenol raw material including a recycled bisphenol and a carbonic acid diester raw material such as diphenyl carbonate to an ester exchange reaction in the presence of an alkali metal compound and/or an alkaline earth metal compound.

The recycled bisphenol may be used as the whole of the bisphenol raw material, or may be mixed with a general bisphenol that is not a recycled bisphenol and used as part of the bisphenol raw material. The amount of the recycled bisphenol based on that of the bisphenol raw material is not particularly limited, and is any amount such as 0.1% by mass or more, 1% by mass or more, 10% by mass or more, 20% by mass or more, 30% by mass or more, 40% by mass or more, 50% by mass or more, 70% by mass or more, 80% by mass or more, or 90% by mass or more. A larger proportion of the recycled bisphenol is more environmentally friendly, and thus, from the viewpoint of consideration for the environment, the amount of the recycled bisphenol based on that of the bisphenol raw material is preferably large.

In addition, the carbonic acid diester raw material may include a recycled carbonic acid diester, or may use only a general carbonic acid diester and include no recycled carbonic acid diester.

The ester exchange reaction can be carried out by appropriately selecting a known method, and an example of a method using diphenyl carbonate as the carbonic acid diester raw material will be described below.

In the above first method for producing a recycled polycarbonate resin, it is preferable to use an excess amount of diphenyl carbonate based on that of the bisphenol raw material. The amount of diphenyl carbonate used based on that of the bisphenol raw material is preferably large from the viewpoint of the produced recycled polycarbonate resin having few terminal hydroxyl groups and being excellent in thermal stability of the polymer, and is preferably small from the viewpoint of the ester exchange reaction rate being fast and it being easy to produce a recycled polycarbonate resin having a desired molecular weight. Because of these, the amount of diphenyl carbonate used per mol of the bisphenol raw material is usually 1.001 mol or more, preferably 1.002 mol or more, and usually 1.3 mol or less, preferably 1.2 mol or less.

As a method for supplying a raw material, a bisphenol raw material and diphenyl carbonate can also be supplied in a solid state, and it is preferable to melt one or both thereof and supply the same in a liquid state.

When producing a recycled polycarbonate resin by an ester exchange reaction between diphenyl carbonate and a bisphenol raw material, an ester exchange catalyst is usually used. As this ester exchange catalyst, an alkali metal compound and/or an alkaline earth metal compound is preferably used. One of these may be used, or two or more thereof may be used in any combination and at any ratio. Practically, it is desirable to use an alkali metal compound.

The amount of the catalyst used per mol of the bisphenol raw material or diphenyl carbonate is usually 0.05 μmol or more, preferably 0.08 μmol or more, and further preferably 0.10 μmol or more. In addition, the amount thereof is usually 100 μmol or less, preferably 50 μmol or less, and further preferably 20 μmol or less.

When the amount of the catalyst used is within such a range, it is easy to obtain a polymerization activity required for producing a recycled polycarbonate resin having a desired molecular weight, and it is easy to obtain a polycarbonate resin having an excellent polymer color hue, a low level of excessive polymer branching, and excellent fluidity during the molding.

In order to produce a recycled polycarbonate resin by the above method, it is preferable to continuously supply both of the above raw materials to a raw material mixing tank and continuously supply the resulting mixture and an ester exchange catalyst to a polymerization tank.

In the production of a recycled polycarbonate resin by the ester exchange method, both of the raw materials supplied to the raw material mixing tank are usually stirred uniformly and then supplied to the polymerization tank to which a catalyst is added, to produce a polymer.

<Second method for producing recycled polycarbonate resin>

The second method for producing a recycled polycarbonate resin is a method for producing a recycled polycarbonate resin, including producing a recycled polycarbonate resin by using a carbonic acid diester raw material including a carbonic acid diester (recycled carbonic acid diester) obtained by the method for producing a bisphenol according to the present invention. As the second method for producing a recycled polycarbonate resin, a known method for polymerizing a polycarbonate resin can be appropriately selected and carried out except that a carbonic acid diester raw material including a recycled carbonic acid diester is used. For example, a recycled polycarbonate resin can be produced

by reacting a bisphenol raw material with a carbonic acid diester raw material including a recycled carbonic acid diester. The ratio of the bisphenol raw material to the carbonic acid diester raw material, the catalyst used, and the like are the same as those described in the first method for producing a recycled polycarbonate resin except that a carbonic acid diester raw material including a recycled carbonic acid diester is used.

The recycled carbonic acid diester may be used as the whole of the carbonic acid diester raw material, or may be mixed with a general carbonic acid diester that is not a recycled carbonic acid diester and used as part of the carbonic acid diester raw material. The amount of the recycled carbonic acid diester based on that of the carbonic acid diester raw material is not particularly limited, and is any amount such as 0.1% by mass or more, 1% by mass or more, 10% by mass or more, 20% by mass or more, 30% by mass or more, 40% by mass or more, 50% by mass or more, 70% by mass or more, 80% by mass or more, or 90% by mass or more. From the viewpoint of consideration for the environment, the amount of the recycled carbonic acid diester based on that of the carbonic acid diester raw material is preferably large.

The carbonic acid diester raw material in the second method for producing a recycled polycarbonate resin is preferably a diaryl carbonate raw material including a diaryl carbonate obtained by the method for producing a carbonic acid diester according to the present invention, and more preferably a diphenyl carbonate raw material including diphenyl carbonate obtained by the method for producing a carbonic acid diester according to the present invention.

When a carbonic acid diester raw material including a recycled carbonic acid diester is used, the bisphenol raw material may include a recycled bisphenol, or may use only a general bisphenol and include no recycled bisphenol.

(Recycled polycarbonate resin and composition thereof)

The recycled polycarbonate resin obtained by the method for producing a recycled polycarbonate resin according to the present invention may be used as it is, or may be used as a recycled polycarbonate resin composition including an unused polycarbonate resin and the recycled polycarbonate resin. The recycled polycarbonate resin composition can be obtained by appropriately selecting a known kneading method or the like and mixing an unused polycarbonate resin and the recycled polycarbonate resin. When the recycled polycarbonate resin composition including an unused polycarbonate resin and the recycled polycarbonate resin is prepared, the amount of the recycled polycarbonate resin is not particularly limited, and a larger proportion of the recycled polycarbonate resin is more environmentally friendly. Because of this, from the viewpoint of consideration for the environment, the amount of the recycled polycarbonate resin based on that of the recycled polycarbonate resin composition is preferably 50% by mass or more, and more preferably 70% by mass or more, 80% by mass or more, or 90% by mass or more, which are listed in ascending order of preference.

The obtained recycled polycarbonate resin or composition thereof can be molded and processed into various molded articles such as an optical member or an optical recording medium as can an unused polycarbonate resin.

<Method for producing epoxy resin>

The present invention relates to a method for producing an epoxy resin, including producing an epoxy resin by using a bisphenol obtained by the method for producing a bisphenol according to the present invention. In addition, the obtained epoxy resin may be further reacted with a polyhydroxy compound raw material to produce an epoxy resin.

As described above, the method for producing an epoxy resin according to the present invention is a method for producing an epoxy resin by using a recycled bisphenol and/or an epoxy resin produced by using a recycled bisphenol, as at least part of a raw material. The method for producing an epoxy resin according to the present invention is not particularly limited except that a recycled bisphenol (bisphenol obtained by the method for producing a bisphenol according to the present invention) and/or an epoxy resin produced by using a recycled bisphenol is used as a raw material, and a known method for producing an epoxy resin can be used. For example, the recycled bisphenol can be used as at least part of a polyhydroxy compound raw material when an epoxy resin is produced by using a one-stage method, an oxidation method, or a two-stage method, as will be described later. The obtained epoxy resin can also be used as at least part of the epoxy resin raw material when an epoxy resin is produced by using a two-stage method.

The "epoxy resin raw material" means an epoxy resin used as a raw material for an epoxy resin obtained by the method for producing an epoxy resin according to the present invention (hereinafter, sometimes referred to as a "recycled epoxy resin"). The "polyhydroxy compound" is a general term for a dihydric or higher polyhydric phenol compound and a dihydric or higher polyhydric alcohol compound, and the "polyhydroxy compound raw material" means a polyhydroxy compound used as a raw material for a recycled epoxy resin.

As the method for producing an epoxy resin according to the present invention, a one-stage method, an oxidation method, a two-stage method, or the like can be used.

The method for producing an epoxy resin by the one-stage method is a method involving using a recycled bisphenol (bisphenol obtained by the method for producing a bisphenol according to the present invention) and reacting the same with an epihalohydrin to obtain an epoxy resin.

The method for producing an epoxy resin by the oxidation method is a method involving allylating a recycled bisphenol by using an allyl halide (allyl chloride, allyl bromide, or the like) and then subjecting the resulting allylated product to an oxidation reaction to obtain an epoxy resin.

The method for producing an epoxy resin by the two-stage method is a method involving reacting an epoxy resin raw material with a polyhydroxy compound raw material, and a recycled bisphenol and/or an epoxy resin produced by using a recycled bisphenol is used as a raw material.

Hereinafter, the methods for producing an epoxy resin by using the one-stage method, the oxidation method, and the two-stage method will be described.

(Method for producing epoxy resin by one-stage method)

The method for producing an epoxy resin by the one-stage method is not particularly limited as long as it is a known production method, and will be described in detail below.

In the method for producing an epoxy resin by the one-stage method, an epoxy resin may be produced by using a recycled bisphenol in combination with a polyhydroxy compound other than the recycled bisphenol (hereinafter, sometimes referred to as a "further polyhydroxy compound"). That is, the method for producing an epoxy resin by the one-stage method is a method involving reacting a polyhydroxy compound raw material with an epihalohydrin to obtain an epoxy resin, and can be a method in which at least part of the polyhydroxy compound raw material is a recycled bisphenol.

The content of the recycled bisphenol in the polyhydroxy compound raw material is not particularly limited, and is preferably 1 to 100% by mass, and more preferably 10 to 100% by mass because a high content of the recycled bisphenol is environmentally friendly.

Here, the "further polyhydroxy compound" is a general term for a dihydric or higher polyhydric phenol compound and a dihydric or higher polyhydric alcohol compound excluding the recycled bisphenol. In the method for producing an epoxy resin by the one-stage method, the "polyhydroxy compound raw material" refers to all polyhydroxy compounds that collectively include a recycled bisphenol and a further polyhydroxy compound used if necessary.

Examples of the further polyhydroxy compound include various polyhydric phenols such as bisphenol A, tetramethyl bisphenol A, bisphenol F, tetramethyl bisphenol F, bisphenol S, bisphenol C, bisphenol AD, bisphenol AF, hydroquinone, resorcin, methylresorcin, biphenol, tetramethyl biphenol, dihydroxynaphthalene, dihydroxydiphenyl ether, a thiodiphenol, a phenol novolac resin, a cresol novolac resin, a phenol aralkyl resin, a biphenyl aralkyl resin, a naphthol aralkyl resin, a terpene phenol resin, a dicyclopentadiene phenol resin, a bisphenol A novolac resin, a naphthol novolac resin, brominated bisphenol A, or a brominated phenol novolac resin, polyhydric phenol resins obtained by condensation reactions between various phenols and various aldehydes such as benzaldehyde, hydroxybenzaldehyde, crotonaldehyde, or glyoxal, polyhydric phenol resins obtained by condensation reactions between xylene resins and phenols, various phenol resins such as a cocondensed resin of heavy oil or a pitch, a phenol, and a formaldehyde, a chain aliphatic diol such as ethylene glycol, trimethylene glycol, propylene glycol, 1,3-butanediol, 1,4-butanediol, 1,3-pentanediol, 1,4-pentanediol, 1,5-pentanediol, or 1,6-hexanediol, a cyclic aliphatic diol such as cyclohexanediol or cyclodecanediol, and a polyalkylene ether glycol such as polyethylene ether glycol, polyoxytrimethylene ether glycol, or polypropylene ether glycol.

At the time of reaction, the polyhydroxy compound raw material is dissolved in an epihalohydrin to form a uniform solution. As the epihalohydrin, epichlorohydrin or epibromohydrin is usually used, and in the present invention, epichlorohydrin is preferable.

The amount of the epihalohydrin used is usually an amount corresponding to 1.0 to 14.0 equivalents, and particularly preferably an amount corresponding to 2.0 to 10.0 equivalents, per equivalent of the hydroxyl group of the polyhydroxy compound raw material (all polyhydroxy compounds). When the amount of the epihalohydrin is equal to or greater than the above lower limit, it is easy to control the molecular weight increasing reaction, and the epoxy resin obtained can have an appropriate epoxy equivalent, and thus such an amount is preferable. On the other hand, when the amount of the epihalohydrin is less than or equal to the above upper limit, the production efficiency tends to be improved, and thus such an amount is preferable.

Next, while stirring the above solution, an alkali metal hydroxide in an amount corresponding to usually 0.1 to 3.0 equivalents, preferably 0.8 to 2.0 equivalents, per equivalent of the hydroxyl group of the polyhydroxy compound raw material is added in the form of a solid or an aqueous solution to cause a reaction. When the amount of the alkali metal hydroxide added is equal to or greater than the above lower limit, it is difficult for the unreacted hydroxyl group and the generated epoxy resin to react with each other and it is easy to control the molecular weight increasing reaction, and thus such an amount is preferable. In addition, when the amount of the alkali metal hydroxide added is less than or equal to the above upper limit, it is difficult for an impurity due to a side reaction to be generated, and thus such an amount is preferable. Examples of the alkali metal hydroxide used here usually include sodium hydroxide or potassium hydroxide.

This reaction can be carried out under normal pressure or under reduced pressure, and the reaction temperature is preferably 20 to 200°C, and more preferably 40 to 150°C. When the reaction temperature is equal to or greater than the above lower limit, it is easy to allow the reaction to proceed and it is easy to control the reaction, and thus such a reaction temperature is preferable. In addition, when the reaction temperature is less than or equal to the above upper limit, it is difficult for a side reaction to proceed and it is particularly easy to reduce the amount of the polymer, and thus such a reaction temperature is preferable.

In addition, this reaction is carried out while carrying out dehydration by a method involving azeotroping the reaction liquid while holding a predetermined temperature if necessary, cooling the volatilized vapor, carrying out oil/water separation of the obtained condensate liquid, and returning the oil resulting from the removal of water to the reaction system. The alkali metal hydroxide is added intermittently or continuously in small amounts over preferably 0.1 to 24 hours, more preferably 0.5 to 10 hours, in order to suppress a rapid reaction. When the addition time of the alkali metal hydroxide is equal to or greater than the above lower limit, it is possible to prevent rapid progress of the reaction, and it is easy to control the reaction temperature, and thus such an addition time is preferable. When the addition time is less than or equal to the above upper limit, it is easy to reduce the amount of the polymer, and thus such an addition time is preferable.

After the reaction is completed, the insoluble by-produced salt can be removed by filtration or removed by washing with water, and then the unreacted epihalohydrin can be distilled off and removed by heating and/or distilling off under reduced pressure.

In addition, in this reaction, a catalyst such as a quaternary ammonium salt such as tetramethylammonium chloride or tetraethylammonium bromide, a tertiary amine such as benzyldimethylamine or 2,4,6-tris(dimethylaminomethyl) phenol, an imidazole such as 2-ethyl-4-methylimidazole or 2-phenylimidazole, a phosphonium salt such as ethyltriphenylphosphonium iodide, or a phosphine such as triphenylphosphine may be used.

Further, in this reaction, an inert organic solvent such as an alcohol such as ethanol or isopropanol, a ketone such as acetone, methyl ethyl ketone, or methyl isobutyl ketone, an ether such as dioxane or ethylene glycol dimethyl ether, a glycol ether such as methoxypropanol, or an aprotic polar solvent such as dimethyl sulfoxide or dimethylformamide may be used.

[Production of epoxy resin having reduced total chlorine content]

When it is necessary to reduce the total chlorine content of the epoxy resin thus obtained, it is possible to produce an epoxy resin having a total chlorine content reduced by reaction with an alkali.

An organic solvent for dissolving the epoxy resin may be used for the reaction with an alkali. The organic solvent used for the reaction is not particularly limited, and it is preferable to use a ketone-based organic solvent from the viewpoint of production efficiency, handleability, workability, or the like. In addition, from the viewpoint of further reducing the amount of hydrolyzable chlorine, an aprotic polar solvent may be used.

Examples of the ketone-based organic solvent include a ketone-based solvent such as methyl ethyl ketone, methyl isobutyl ketone, or cyclohexanone. Methyl isobutyl ketone is particularly preferable because of the effect, ease of a post-treatment, or the like. These may be used singly or as a mixture of two or more.

Examples of the aprotic polar solvent include dimethyl sulfoxide, diethyl sulfoxide, dimethyl sulfone, sulfolane, dimethylformamide, dimethylacetamide, and hexamethylphosphoramide. These may be used singly or as a mixture of two or more. Among these aprotic polar solvents, dimethyl sulfoxide is preferable because it is easily available and has an excellent effect.

The amount of such a solvent used is an amount that allows the concentration of the epoxy resin in a liquid to be treated with an alkali to be usually 1 to 95% by mass, and preferably 5 to 80% by mass.

As the alkali, a solid or a solution of an alkali metal hydroxide can be used. Examples of the alkali metal hydroxide include potassium hydroxide and sodium hydroxide, and sodium hydroxide is preferable. In addition, as the alkali metal hydroxide, one dissolved in an organic solvent or water may be used. Preferably, the alkali metal hydroxide is used as a solution obtained by dissolving the same in an aqueous solvent or an organic solvent.

The amount of the alkali metal hydroxide used is preferably 0.01 to 20.0 parts by mass or less per 100 parts by mass of the epoxy resin in terms of the solid content of the alkali metal hydroxide. The amount thereof is more preferably 0.10 to 10.0 parts by mass. When the amount of the alkali metal hydroxide used is less than or equal to the above lower limit, the effect of reducing the total chlorine content is low, and when the amount thereof is equal to or greater than the above upper limit, a large amount of the polymer is generated and thus the yield is lowered.

The reaction temperature is preferably 20 to 200°C, and more preferably 40 to 150°C, and the reaction time is preferably 0.1 to 24 hours, and more preferably 0.5 to 10 hours.

After the reaction, the excess alkali metal hydroxide and the by-produced salt can be removed by a method such as washing with water, and further the organic solvent can be removed by distilling off under heating and/or reduced pressure and/or steam distillation.

(Method for producing epoxy resin by oxidation method)

The method for producing an epoxy resin by the oxidation method is not particularly limited as long as it is a known production method, and can be carried out, for example, according to a method disclosed in Japanese Patent Laid-Open No. 2011-225711, Japanese Patent Laid-Open No. 2012-092247, Japanese Patent Laid-Open No. 2012-111858, or the like.

Even in the method for producing an epoxy resin by the oxidation method, as for the one-stage method, an epoxy resin may be produced by using a recycled bisphenol in combination with a further polyhydroxy compound other than the recycled bisphenol. That is, the method for producing an epoxy resin by the oxidation method is a method involving allylating a polyhydroxy compound raw material by using an allyl halide and then subjecting the resulting allylated product to an oxidation reaction to obtain an epoxy resin, and can be a method in which at least part of the polyhydroxy compound raw material is a recycled bisphenol.

In the method for producing an epoxy resin by the oxidation method, the "polyhydroxy compound raw material" refers to all polyhydroxy compounds that collectively include a recycled bisphenol and a further polyhydroxy compound used if necessary, and examples of the further polyhydroxy compound include the same ones as for the one-stage method. The content of the recycled bisphenol in the polyhydroxy compound raw material is not particularly limited, and is preferably 1 to 100% by mass, and more preferably 10 to 100% by mass because a high content of the recycled bisphenol is environmentally friendly.

(Method for producing epoxy resin by two-stage method)

The method for producing an epoxy resin by the two-stage method is not particularly limited as long as it is a known production method, and will be described in detail below.

The method for producing an epoxy resin by the two-stage method can be a method including a step of reacting an epoxy resin raw material with a polyhydroxy compound raw material, wherein at least part of the epoxy resin raw material is an epoxy resin produced by using a recycled bisphenol, and/or at least part of the polyhydroxy compound raw material is a recycled bisphenol.

That is, the method for producing an epoxy resin by the two-stage method is any of the following methods (i) to (iii).

Method (i): A method involving reacting a further epoxy resin other than an epoxy resin produced by using a recycled bisphenol with a polyhydroxy compound raw material including a recycled bisphenol

In the method (i), the epoxy resin raw material is a further epoxy resin other than an epoxy resin produced by using a recycled bisphenol. In addition, the "polyhydroxy compound raw material" refers to all polyhydroxy compounds that collectively include a recycled bisphenol and a further polyhydroxy compound used if necessary.

Method (ii): A method involving reacting an epoxy resin raw material including an epoxy resin produced by using a recycled bisphenol with a polyhydroxy compound raw material including a recycled bisphenol

In the method (ii), the epoxy resin raw material is all epoxy resins that collectively include an epoxy resin produced by using a recycled bisphenol and a further epoxy resin used if necessary. In addition, the "polyhydroxy compound raw material" refers to all polyhydroxy compounds that collectively include a recycled bisphenol and a further polyhydroxy compound used if necessary.

Method (iii): A method involving reacting an epoxy resin raw material including an epoxy resin produced using a recycled bisphenol with a further polyhydroxy compound raw material other than a recycled bisphenol

In the method (iii), the epoxy resin raw material is all epoxy resins that collectively include an epoxy resin produced by using a recycled bisphenol and a further epoxy resin used if necessary. The polyhydroxy compound raw material is a further polyhydroxy compound other than a recycled bisphenol.

The epoxy resin produced by using a recycled bisphenol used in the method (ii) and the method (iii) can be obtained by the method for producing an epoxy resin by the one-stage method or the method for producing an epoxy resin by the oxidation method. In addition, the epoxy resin obtained by the method (i) may be used. The further epoxy resin other than an epoxy resin produced by using a recycled bisphenol is the same as the further epoxy resin described later in the method for producing an epoxy resin cured product, and the further polyhydroxy compound is the same as for the one-stage method.

In the method (i) and the method (ii), in the polyhydroxy compound raw material including a recycled bisphenol, the content of the recycled bisphenol is not particularly limited, and is preferably 1 to 100% by mass, and more preferably 10 to 100% by mass because a high content of the recycled bisphenol is environmentally friendly.

In addition, in the method (ii) and the method (ii), in the epoxy resin raw material including an epoxy resin produced by using a recycled bisphenol, the content of the epoxy resin produced by using a recycled bisphenol is not particularly limited, and is preferably 1 to 100% by mass, and more preferably 10 to 100% by mass because a high

content of the epoxy resin produced by using a recycled bisphenol is environmentally friendly.

In the reaction by the two-stage method, the amounts of the epoxy resin raw material and the polyhydroxy compound raw material used are preferably such that the blending equivalent ratio is (epoxy group equivalent):(hydroxyl group equivalent) = 1:0.1 to 2.0. The blending equivalent ratio is more preferably 1:0.2 to 1.2. When this equivalent ratio is within such a range, it is easy to increase the molecular weight, and it is possible to leave more epoxy group ends, and thus such an equivalent ratio is preferable.

In addition, a catalyst may be used in the reaction by the two-stage method, and the catalyst may be any compound as long as the compound has a catalytic ability to promote the reaction between an epoxy group and a phenolic hydroxyl group or an alcoholic hydroxyl group. Examples thereof include an alkali metal compound, an organophosphorus compound, a tertiary amine, a quaternary ammonium salt, a cyclic amine, and an imidazole. Among these, a quaternary ammonium salt is preferable. In addition, only one catalyst can be used, or two or more can be used in combination. The amount of the catalyst used is usually 0.001 to 10% by mass based on that of the epoxy resin raw material.

In addition, in the reaction by the two-stage method, a solvent may be used, and the solvent may be any solvent as long as this solvent dissolves the epoxy resin raw material. Examples thereof include an aromatic solvent, a ketone-based solvent, an amide-based solvent, and a glycol ether-based solvent. Only one solvent may be used, or two or more can be used in combination. In addition, the resin concentration of the solvent is preferably 10 to 95% by mass. The resin concentration is more preferably 20 to 80% by mass. In addition, when a highly viscous product is generated in the middle of the reaction, the solvent can also be additionally added to continue the reaction. After completion of the reaction, the solvent can, if necessary, be removed or further added.

In the reaction by the two-stage method, the reaction temperature is preferably 20 to 250°C, and more preferably 50 to 200°C. When the reaction temperature is equal to or greater than the above upper limit, the epoxy resin generated may deteriorate. In addition, when the reaction temperature is less than or equal to the above lower limit, the reaction may not proceed sufficiently. In addition, the reaction time is usually 0.1 to 24 hours, and preferably 0.5 to 12 hours.

<Method for producing epoxy resin cured product>

The present invention relates to a method for producing an epoxy resin cured product, including curing an epoxy resin composition including an epoxy resin obtained by the method for producing an epoxy resin according to the present invention and a curing agent to obtain an epoxy resin cured product. In the method for producing an epoxy resin cured product according to the present invention, an epoxy resin obtained by the above method for producing an epoxy resin according to the present invention is mixed with a curing agent to obtain a composition including the epoxy resin and the curing agent (hereinafter, sometimes referred to as an "epoxy resin composition"), and then the epoxy resin composition is cured to obtain an epoxy resin cured product.

In addition, if necessary, a further epoxy resin other than the epoxy resin obtained by the method for producing an epoxy resin according to the present invention (hereinafter, sometimes simply referred to as a "further epoxy resin"), a curing agent, a curing accelerator, an inorganic filler, a coupling agent, or the like can be appropriately blended into the epoxy resin composition.

The content of the recycled epoxy resin in the epoxy resin composition is not particularly limited. A high content of the recycled epoxy resin is environmentally friendly, and thus the recycled epoxy resin is preferably 40 parts by mass or more, and more preferably 60 parts by mass or more per 100 parts by mass of all epoxy resin components in the recycled epoxy resin composition. When a further epoxy resin is included, the recycled epoxy resin can be 40 to 99 parts by mass, 60 to 99 parts by mass, or the like per 100 parts by mass of all epoxy resin components in the epoxy resin composition. "All epoxy resin components" correspond to the amount of all epoxy resins included in the epoxy resin composition, and is the total amount of the recycled epoxy resin and a further epoxy resin used if needed.

(Curing agent)

In the present invention, the curing agent refers to a substance that contributes to the crosslinking reaction and/or the chain length extension reaction between the epoxy groups of an epoxy resin. In the present invention, even if a substance is usually referred to as a "curing accelerator," when the substance is a substance that contributes to the crosslinking reaction and/or the chain length extension reaction between the epoxy groups of an epoxy resin, the substance is regarded as a curing agent.

In the epoxy resin composition, the content of the curing agent is preferably 0.1 to 1000 parts by mass per 100 parts by mass of all epoxy resin components. In addition, the content thereof is more preferably 500 parts by mass or less.

The curing agent is not particularly limited, and all generally known epoxy resin curing agents can be used. Examples thereof include a phenol-based curing agent, an amine-based curing agent such an aliphatic amine, a polyether amine, an alicyclic amine, or an aromatic amine, an acid anhydride-based curing agent, an amide-based curing agent,

a tertiary amine, and an imidazole. Such curing agents may be used singly or in combinations of two or more. When two or more curing agents are used in combination, they may be mixed in advance to prepare a mixed curing agent before use, or when mixing each component of an epoxy resin obtained by the method for producing an epoxy resin according to the present invention and a further epoxy resin, each component of the curing agent may be added separately to mix these components at the same time.

[Phenol-based curing agent]

Specific examples of the phenol-based curing agent include various polyhydric phenols such as a recycled bisphenol, bisphenol A, tetramethyl bisphenol A, bisphenol F, tetramethyl bisphenol F, bisphenol C, bisphenol S, bisphenol AD, bisphenol AF, hydroquinone, resorcin, methylresorcin, biphenol, tetramethyl biphenol, dihydroxynaphthalene, dihydroxydiphenyl ether, a thiodiphenol, a phenol novolac resin, a cresol novolac resin, a phenol aralkyl resin, a biphenyl aralkyl resin, a naphthol aralkyl resin, terpene phenol resin, a dicyclopentadiene phenol resin, a bisphenol A novolac resin, a trisphenol methane type resin, a naphthol novolac resin, brominated bisphenol A, or a brominated phenol novolac resin, polyhydric phenol resins obtained by condensation reactions between various phenols and various aldehydes such as benzaldehyde, hydroxybenzaldehyde, crotonaldehyde, or glyoxal, polyhydric phenol resins obtained by condensation reactions between xylene resins and phenols, and various phenol resins such as a cocondensed resin of heavy oil or a pitch, a phenol, and a formaldehyde, a phenol/benzaldehyde/xylylene dimethoxide polycondensate, a phenol/benzaldehyde/xylylene dihalide polycondensate, a phenol/benzaldehyde/4,4'-dimethoxide biphenyl polycondensate, or a phenol/benzaldehyde/4,4'-dihalide biphenyl polycondensate.
Only one of these phenol-based curing agents may be used, or two or more thereof may be used in any combination and at any blending ratio.
The amount of the phenol-based curing agent blended is preferably 0.1 to 1000 parts by mass, and more preferably 500 parts by mass or less per 100 parts by mass of all epoxy resin components in the epoxy resin composition.

[Amine-based curing agent]

Examples of the amine-based curing agent (excluding a tertiary amine) include an aliphatic amine, a polyether amine, an alicyclic amine, and an aromatic amine.
Examples of the aliphatic amine include ethylenediamine, 1,3-diaminopropane, 1,4-diaminopropane, hexamethylenediamine, 2,5-dimethylhexamethylenediamine, trimethylhexamethylenediamine, diethylenetriamine, iminobispropylamine, bis(hexamethylene)triamine, triethylenetetramine, tetraethylenepentamine, pentaethylenehexamine, N-hydroxyethylethylenediamine, and tetra(hydroxyethyl)ethylenediamine.
Examples of the polyether amine include triethylene glycol diamine, tetraethylene glycol diamine, diethylene glycol bis(propylamine), polyoxypropylenediamine, and a polyoxypropylenetriamine.
Examples of the alicyclic amine include isophoronediamine, metasendiamine, N-aminoethylpiperazine, bis(4-amino-3-methyldicyclohexyl)methane, bis(aminomethyl)cyclohexane, 3,9-bis(3-aminopropyl)-2,4,8,10-tetraoxaspiro(5,5)undecane, and norbornenediamine.
Examples of the aromatic amine include tetrachloro-p-xylenediamine, m-xylenediamine, p-xylenediamine, m-phenylenediamine, o-phenylenediamine, p-phenylenediamine, 2,4-diaminoanisol, 2,4-toluenediamine, 2,4-diaminodiphenylmethane, 4,4'-diaminodiphenylmethane, 4,4'-diamino-1,2-diphenylethane, 2,4-diaminodiphenylsulfone, 4,4'-diaminodiphenylsulfone, m-aminophenol, m-aminobenzylamine, benzyldimethylamine, 2-(dimethylaminomethyl)phenol, triethanolamine, methylbenzylamine, α-(m-aminophenyl)ethylamine, α-(p-aminophenyl)ethylamine, diaminodiethyldimethyldiphenylmethane, and α,α'-bis(4-aminophenyl)-p-diisopropylbenzene.
Only one of the amine-based curing agents listed above may be used, or two or more thereof may be used in any combination and at any blending ratio.
It is preferable to use such an amine-based curing agent such that the equivalent ratio of the functional group in the curing agent to the epoxy group in all epoxy resin components included in the epoxy resin composition is in the range of 0.1 to 2.0. The equivalent ratio is more preferably in the range of 0.8 to 1.2. When the equivalent ratio is within such a range, an unreacted epoxy group and a functional group of the curing agent are unlikely to remain, and thus such an equivalent ratio is preferable.

[Tertiary amine]

Examples of the tertiary amine include 1,8-diazabicyclo(5,4,0)undecene-7, triethylenediamine, benzyldimethylamine, triethanolamine, dimethylaminoethanol, and tris(dimethylaminomethyl)phenol.
Only one of the tertiary amines listed above may be used, or two or more thereof may be used in any combination and at any blending ratio.

It is preferable to use such a tertiary amine such that the equivalent ratio of the functional group in the curing agent to the epoxy group in all epoxy resin components included in the epoxy resin composition is in the range of 0.1 to 2.0. The equivalent ratio is more preferably in the range of 0.8 to 1.2. When the equivalent ratio is within such a range, an unreacted epoxy group and a functional group of the curing agent are unlikely to remain, and thus such an equivalent ratio is preferable.

[Acid anhydride-based curing agent]

Examples of the acid anhydride-based curing agent include an acid anhydride and a modified product of an acid anhydride.

Examples of the acid anhydride include phthalic anhydride, trimellitic anhydride, pyromellitic anhydride, benzophenonetetracarboxylic anhydride, dodecenylsuccinic anhydride, polyadipic anhydride, polyazelaic anhydride, polysebacic anhydride, poly(ethyloctadecanedioic) anhydride, poly(phenylhexadecanedioic) anhydride, tetrahydrophthalic anhydride, methyltetrahydrophthalic anhydride, methylhexahydrophthalic anhydride, hexahydrophthalic anhydride, methylhimic anhydride, trialkyltetrahydrophthalic anhydride, methylcyclohexenedicarboxylic anhydride, methylcyclohexenetetracarboxylic anhydride, ethylene glycol bistrimellitate dianhydride, HET anhydride, nadic anhydride, methylnadic anhydride, 5-(2,5-dioxotetrahydro-3-furanyl)-3-methyl-3-cyclohexane-1,2-dicarboxylic anhydride, 3,4-dicarboxy-1,2,3,4-tetrahydro-1-naphthalenesuccinic dianhydride, and 1-methyl-dicarboxy-1,2,3,4-tetrahydro-1-naphthalenesuccinic dianhydride.

Examples of the modified product of an acid anhydride include products obtained by modifying the above acid anhydrides with glycol. Here, examples of the glycol that can be used for the modification include an alkylene glycol such as ethylene glycol, propylene glycol, or neopentyl glycol, and a polyether glycol such as polyethylene glycol, polypropylene glycol, or polytetramethylene ether glycol. Further, a copolymerized polyether glycol of two or more of these glycols and/or a polyether glycol can also be used.

Only one of the acid anhydride-based curing agents listed above may be used, or two or more thereof may be used in any combination and at any blending ratio.

When an acid anhydride-based curing agent is used, it is preferable to use the acid anhydride-based curing agent such that the equivalent ratio of the functional group in the curing agent to the epoxy group in all epoxy resin components in the epoxy resin composition is in the range of 0.1 to 2.0. The equivalent ratio is more preferably in the range of 0.8 to 1.2. When the equivalent ratio is within such a range, an unreacted epoxy group and a functional group of the curing agent are unlikely to remain, and thus such an equivalent ratio is preferable.

[Amide-based curing agent]

Examples of the amide-based curing agent include dicyandiamide and a derivative thereof, and a polyamide resin.

Only one of the amide-based curing agents may be used, or two or more thereof may be mixed and used in any combination and at any ratio.

When an amide-based curing agent is used, it is preferable to use the amide-based curing agent such that the amount of the amide-based curing agent is 0.1 to 20% by mass based on the total amount of all epoxy resin components in the epoxy resin composition and the amide-based curing agent.

[Imidazole]

Examples of the imidazole include 2-phenylimidazole, 2-ethyl-4(5)-methylimidazole, 2-phenyl-4-methylimidazole, 1-benzyl-2-methylimidazole, 1-benzyl-2-phenylimidazole, 1-cyanoethyl-2-undecylimidazole, 1-cyano-2-phenylimidazole, 1-cyanoethyl-2-undecylimidazole trimellitate, 1-cyanoethyl-2-phenylimidazolium trimellitate, 2,4-diamino-6-[2'-methylimidazolyl-(1')]-ethyl-s-triazine, 2,4-diamino-6-[2'-ethyl-4'-methylimidazolyl-(1')]-ethyls-triazine, a 2,4-diamino-6-[2'-methylimidazolyl-(1')]-ethyl-s-triazine isocyanuric acid adduct, a 2-phenylimidazole isocyanuric acid adduct, 2-phenyl-4,5-dihydroxymethylimidazole, 2-phenyl-4-methyl-5-hydroxymethylimidazole, and adducts of an epoxy resin and the above imidazoles. An imidazole has a catalytic ability, and thus can also be generally classified as a curing accelerator, but in the present invention, an imidazole is classified as a curing agent.

Only one of the imidazoles listed above may be used, or two or more thereof may be mixed and used in any combination and at any ratio.

When an imidazole is used, it is preferable to use the imidazole such that the amount of the imidazole is 0.1 to 20% by mass based on the total amount of all epoxy resin components in the epoxy resin composition and the imidazole.

[Further curing agent]

In the epoxy resin composition, a further curing agent can be used in addition to the curing agent. The further curing agent that can be used in the epoxy resin composition is not particularly limited, and all generally known curing agents for an epoxy resin can be used.
Only one of these further curing agents may be used, or two or more thereof may be used in combination.

(Further epoxy resin)

The epoxy resin composition can include a further epoxy resin other than the epoxy resin obtained by the method for producing an epoxy resin according to the present invention. By including a further epoxy resin, various physical properties can be improved.
The further epoxy resin that can be used in the epoxy resin composition corresponds to all epoxy resins other than the epoxy resin obtained by the method for producing an epoxy resin according to the present invention. Specific examples include a bisphenol A type epoxy resin, a bisphenol C type epoxy resin, a trisphenol methane type epoxy resin, an anthracene type epoxy resin, a phenol-modified xylene resin type epoxy resin, a bisphenol cyclododecyl type epoxy resin, a bisphenol diisopropylidene resorcin type epoxy resin, a bisphenol F type epoxy resin, a bisphenol AD type epoxy resin, a bisphenol AF type epoxy resin, a hydroquinone type epoxy resin, a methylhydroquinone type epoxy resin, a dibutylhydroquinone type epoxy resin, a resorcin type epoxy resin, a methylresorcin type epoxy resin, a biphenol type epoxy resin, a tetramethyl biphenol type epoxy resin, a tetramethyl bisphenol F type epoxy resin, a dihydroxydiphenyl ether type epoxy resin, an epoxy resin derived from a thiodiphenol, a dihydroxynaphthalene type epoxy resin, a dihydroxyanthracene type epoxy resin, a dihydroxydihydroanthracene type epoxy resin, a dicyclopentadiene type epoxy resin, an epoxy resin derived from a dihydroxystilbene, a phenol novolac type epoxy resin, a cresol novolac type epoxy resin, a bisphenol A novolac type epoxy resin, a naphthol novolac type epoxy resin, a phenol aralkyl type epoxy resin, a naphthol aralkyl type epoxy resin, a biphenyl aralkyl type epoxy resin, a terpene phenol type epoxy resin, a dicyclopentadiene phenol type epoxy resin, an epoxy resin derived from a condensate of phenol/hydroxybenzaldehyde, an epoxy resin derived from a condensate of phenol/crotonaldehyde, an epoxy resin derived from a condensate of phenol/glyoxal, an epoxy resin derived from a cocondensed resin of heavy oil or a pitch, a phenol, and a formaldehyde, an epoxy resin derived from diaminodiphenylmethane, an epoxy resin derived from aminophenol, an epoxy resin derived from xylenediamine, an epoxy resin derived from methylhexahydrophthalic acid, and an epoxy resin derived from a dimer acid. Only one of these may be used, or two or more thereof may be used in any combination and at any blending ratio.
When the epoxy resin composition includes a further epoxy resin as described above, the content thereof is preferably 1 to 60 parts by mass, and more preferably 40 parts by mass or less per 100 parts by mass of all epoxy resin components in the composition.

(Curing accelerator)

The epoxy resin composition preferably includes a curing accelerator. By including a curing accelerator, it is possible to shorten the curing time and lower the curing temperature, and it is possible to easily obtain a desired cured product.
The curing accelerator is not particularly limited, and specific examples thereof include an organic phosphine, a phosphorus-based compound such as a phosphonium salt, a tetraphenylboron salt, an organic acid dihydrazide, and a boron halide amine complex.
Examples of the phosphorus-based compound that can be used as a curing accelerator include an organic phosphine such as triphenylphosphine, diphenyl(p-tolyl)phosphine, a tris(alkylphenyl)phosphine, a tris(alkoxyphenyl)phosphine, a tris(alkyl/alkoxyphenyl)phosphine, a tris(dialkylphenyl)phosphine, a tris(trialkylphenyl)phosphine, a tris(tetraalkylphenyl)phosphine, a tris(dialkoxyphenyl)phosphine, a tris(trialkoxyphenyl)phosphine, a tris(tetraalkoxyphenyl)phosphine, a trialkylphosphine, a dialkylarylphosphine, or an alkyldiarylphosphine, complexes of these organic phosphines and an organic boron compound, and compounds obtained by adding, to these organic phosphines, a compound such as maleic anhydride, a quinone compound such as 1,4-benzoquinone, 2,5-toluquinone, 1,4-naphthoquinone, 2,3-dimethylbenzoquinone, 2,6-dimethylbenzoquinone, 2,3-dimethoxy-5-methyl-1,4-benzoquinone, 2,3-dimethoxy-1,4-benzoquinone, or phenyl-1,4-benzoquinone, or diazophenylmethane.
Among the curing accelerators listed above, an organic phosphine and a phosphonium salt are preferable, and an organic phosphine is most preferable. In addition, only one of the curing accelerators listed above may be used, or two or more thereof may be mixed and used in any combination and at any ratio.
The curing accelerator is preferably used in the range of 0.1 parts by mass or more and 20 parts by mass or less per 100 parts by mass of all epoxy resin components in the epoxy resin composition. When the content of the curing accelerator is equal to or greater than the above lower limit value, it is possible to obtain a good curing acceleration

effect, and on the other hand, when the content thereof is less than or equal to the above upper limit value, it is easy to obtain a desired cured physical property, and thus such a content is preferable.

(Inorganic filler)

An inorganic filler can be blended into the epoxy resin composition. Examples of the inorganic filler include fused silica, crystalline silica, a glass powder, alumina, calcium carbonate, calcium sulfate, talc, and boron nitride. Only one of these may be used, or two or more thereof may be used in any combination and at any blending ratio. The amount of the inorganic filler blended is preferably 10 to 95% by mass based on the total amount of the epoxy resin composition.

(Release agent)

A release agent can be blended into the epoxy resin composition. As the release agent, for example, a natural wax such as carnauba wax, a synthetic wax such as polyethylene wax, a higher fatty acid and a metal salt thereof such as stearic acid or zinc stearate, or a hydrocarbon-based release agent such as paraffin can be used. Only one of these may be used, or two or more thereof may be used in any combination and at any blending ratio.

The content of the release agent blended is preferably 0.001 to 10.0 parts by mass per 100 parts by mass of all epoxy resin components in the epoxy resin composition. When the amount of the release agent blended is within the above range, good releasability can be developed while maintaining a curing characteristic.

[Coupling agent]

A coupling agent can be blended into the epoxy resin composition. The coupling agent is preferably used in combination with an inorganic filler, and by blending a coupling agent, the adhesiveness between an epoxy resin as a matrix and an inorganic filler can be improved. Examples of the coupling agent include a silane coupling agent and a titanate coupling agent.

Examples of the silane coupling agent include an epoxysilane such as γ-glycidoxypropyltrimethoxysilane, γ-glycidoxypropyltriethoxysilane, or β-(3,4-epoxycyclohexyl)ethyltrimethoxysilane, an aminosilane such as γ-aminopropyltriethoxysilane, N-β(aminoethyl)γ-aminopropyltrimethoxysilane, N-β(aminoethyl)γ-aminopropylmethyldimethoxysilane, γ-aminopropyltrimethoxysilane, or γ-ureidopropyltriethoxy silane, a mercaptosilane such as 3-mercaptopropyltrimethoxysilane, a vinylsilane such as p-styryltrimethoxysilane, vinyltrichlorosilane, vinyltris(β-methoxyethoxy)silane, vinyltrimethoxysilane, vinyltriethoxysilane, or γ-methacryloxypropyltrimethoxysilane, and further, an epoxy-based, amino-based, or vinyl-based polymer type silane.

Examples of the titanate coupling agent include isopropyltriisostearoyl titanate, isopropyltri(N-aminoethyl/aminoethyl) titanate, diisopropylbis(dioctyl phosphate) titanate, tetraisopropylbis(dioctyl phosphite) titanate, tetraoctylbis(ditridecyl phosphite) titanate, tetra(2,2-diallyloxymethyl-1-butyl)bis(ditridecyl) phosphite titanate, bis(dioctyl pyrophosphate) oxyacetate titanate, and bis(dioctyl pyrophosphate) ethylene titanate.

Only one of these coupling agents may be used, or two or more thereof may be mixed and used in any combination and at any ratio.

When a coupling agent is used in the epoxy resin composition, the amount thereof blended is preferably 0.001 to 10.0 parts by mass per 100 parts by mass of all epoxy resin components. When the amount of the coupling agent blended is equal to or greater than the above lower limit value, the effect of improving the adhesiveness between an epoxy resin as a matrix and an inorganic filler due to the blending of the coupling agent tends to be improved, and on the other hand, when the amount of the agent blended is less than or equal to the above upper limit value, the coupling agent is unlikely to bleed out from the cured product obtained, and thus such an amount thereof is preferable.

(Further blending component)

A component other than those described above can be blended into the epoxy resin composition. Examples of the further blending component include a flame retardant, a plasticizer, a reactive diluent, and a pigment, and these can be appropriately blended if necessary. However, this does not prevent the blending of a component other than those listed above.

Examples of the flame retardant include a halogen-based flame retardant such as a brominated epoxy resin or a brominated phenol resin, an antimony compound such as antimony trioxide, a phosphorus-based flame retardant such as red phosphorus, a phosphoric acid ester, or a phosphine, a nitrogen-based flame retardant such as a melamine derivative, and an inorganic flame retardant such as aluminum hydroxide or magnesium hydroxide.

(Curing method)

An epoxy resin cured product can be obtained by curing the epoxy resin composition. The curing method is not particularly limited, and usually, a cured product can be obtained by a thermal curing reaction by heating. At the time of the thermal curing reaction, it is preferable to appropriately select the curing temperature depending on the type of the curing agent used. For example, when a phenol-based curing agent is used, the curing temperature is usually 80 to 250°C. In addition, it is also possible to lower the curing temperature by adding a curing accelerator to these curing agents. The reaction time is preferably 0.01 to 20 hours. When the reaction time is equal to or greater than the above lower limit value, the curing reaction tends to easily proceed sufficiently, and thus such a reaction time is preferable. On the other hand, when the reaction time is less than or equal to the above upper limit value, it is easy to reduce deterioration due to heating and energy loss during the heating, and thus such a reaction time is preferable.

(Applications)

The epoxy resin cured product obtained by curing the epoxy resin composition has a low linear expansion coefficient, and a cured product having excellent thermal cracking resistance can be obtained.

Therefore, the epoxy resin cured product can be effectively used in any application as long as the application requires these physical properties. For example, the epoxy resin cured product can be suitably used for any of applications such as a coating material field such as an electrodeposition coating material for an automobile, a heavy-duty anticorrosion coating material for a ship/bridge, or a coating material for inner surface coating of a beverage can; an electrical or electronic field such as a laminated plate, a semiconductor encapsulant, an insulating powder coating material, or coil impregnation; or a civil engineering/construction/adhesive field such as seismic reinforcement of a bridge, concrete reinforcement, a floor material of a building, lining of a waterworks facility, drainage/water permeable pavement, or an adhesive for a vehicle/aircraft.

The epoxy resin composition may be used after being cured for the above applications, or may be cured in a production step in the above applications.

Examples

Hereinafter, the present invention will be described more specifically with reference to Examples and Comparative Examples, but the present invention is not limited by the following Examples as long as they do not depart from the scope of the present invention.

[Raw material and reagents]

As the polycarbonate resin, the polycarbonate resin "NOVAREX (registered trademark) M7027BF" manufactured by Mitsubishi Engineering-Plastics Corporation was used.

As phenol, orthocresol, metacresol, a cresol isomer mixture (orthocresol, metacresol, paracresol), toluene, sodium hydroxide, potassium hydroxide, methylamine, dimethylamine, trimethylamine, ethylamine, diethylamine, triethylamine, tripropylamine, p-toluenesulfonic acid, methanesulfonic acid, 35% hydrochloric acid, 98% sulfuric acid, 85% phosphoric acid, acetic acid, acetonitrile, cesium carbonate, pyridine, sodium chloride, dibromophenol, and carbon tetrachloride, reagents of FUJIFILM Wako Pure Chemical Corporation were used.

As diphenyl carbonate, a product of Mitsubishi Chemical Corporation was used.

[Analysis]

The generation and purity of a bisphenol were checked by high performance liquid chromatography under the following procedure and conditions.

- Apparatus: LC-2010A manufactured by Shimadzu Corporation, Waters Corporation 5 μm 150 mm × 4.6 mm ID
- Method: Low pressure gradient method
- Analysis temperature: 40°C
- Eluent composition:

    Liquid A acetonitrile
    Liquid B a solution of 85% phosphoric acid:water = 1 mL:999 mL

At minute 0 of the analysis time, the eluent composition was liquid A:liquid B = 35:65 (volume ratio, the same

applies hereinafter); at minute 0 to minute 5 of the analysis time, the eluent composition was maintained at liquid A:liquid B = 35:65; and then at minute 5 to minute 40 of the analysis time, the eluent composition was gradually changed to liquid A:liquid B = 90:10.

- Flow rate: 0.85 mL/min
- Detection wavelength: 280 nm

Analysis of pyridine and phenol included in the neutralized wastewater was carried out by gas chromatography under the following procedure and conditions.

- Apparatus: GC-2014 manufactured by Shimadzu Corporation
  Agilent DB-1 0.530 mm × 30 m 1.5 μm
- Detection method: FID
- Vaporization chamber temperature: 230°C
- Detector temperature: 300°C
- At minute 0 to minute 5 of the analysis time, the column temperature was kept at 50°C; at minute 5 to minute 30 of the analysis time, the column temperature was gradually raised to 280°C; and at minute 30 to minute 40 of the analysis time, the column temperature was maintained at 280°C.
- Quantification method: Internal standard method using biphenyl as the internal standard

The amount of sodium chloride included in the neutralized wastewater was calculated from the mass of the residue after evaporating the neutralized wastewater to dryness.
Analysis of dibromophenol included in the hydrochloric acid wastewater was carried out by gas chromatography under the following procedure and conditions.

- Apparatus: GC-2014 manufactured by Shimadzu Corporation
  Agilent DB-1 0.530 mm × 30 m 1.5 μm
- Detection method: FID
- Vaporization chamber temperature: 230°C
- Detector temperature: 300°C
- At minute 0 to minute 5 of the analysis time, the column temperature was kept at 50°C; at minute 5 to minute 30 of the analysis time, the column temperature was gradually raised to 280°C; and at minute 30 to minute 40 of the analysis time, the column temperature was maintained at 280°C.
- The quantification was carried out by the absolute calibration curve method.

The concentration of hydrogen chloride included in the hydrochloric acid wastewater was measured by using the following apparatus by neutralization titration.
Apparatus: Automatic Potentiometric titrator AT-610, Kyoto Electronics Manufacturing Co., Ltd.
Analysis of carbon tetrachloride included in the sodium hydroxide wastewater was carried out by gas chromatography under the following procedure and conditions.

- Apparatus: Agilent Technologies, Inc.
  J&W DB-17 0.32 mm × 30 m × 0.5 μm
- At minute 0 of the analysis time, the column temperature was set to 50°C, and the temperature was raised at a rate of 10°C per minute to 250°C.
- Detector: MS

The concentration of sodium hydroxide included in the sodium hydroxide wastewater was measured by using the following apparatus.

- Apparatus: Automatic Potentiometric titrator AT-610, Kyoto Electronics Manufacturing Co., Ltd.

The concentration of sodium chloride included in the sodium hydroxide wastewater was calculated by measuring the chloride ion concentration by using the following apparatus and determining the amount of sodium equimolar to the obtained chloride ion.

- Apparatus: Automatic Potentiometric titrator AT-610, Kyoto Electronics Manufacturing Co., Ltd.

[Viscosity average molecular weight (Mv)]

The viscosity average molecular weight (Mv) was calculated by dissolving a polycarbonate resin in methylene chloride (concentration of 6.0 g/L), measuring the specific viscosity (ηsp) at 20°C by using an Ubbelohde viscosity tube, and using the following expression to measure the viscosity average molecular weight (Mv).

$$\eta sp/C = [\eta]\ (1 + 0.28\ \eta sp)$$

$$[\eta] = 1.23 \times 10^{-4} Mv^{0.83}$$

[Molten color of bisphenol]

For the molten color of a bisphenol, 20 g of the bisphenol was placed in a test tube "P-24" (2 mmcp × 200 mm) manufactured by Nichiden-Rika Glass Co., Ltd., melted at 174°C for 30 minutes, and the Hazen color value thereof was measured by using "OME7700" manufactured by Nippon Denshoku Industries Co., Ltd.

[Measurement of pH]

The pH was measured by using a pH meter "pH METER ES-73" manufactured by HORIBA, Ltd. for an aqueous phase at 25°C taken out from a flask.

(Example 1)

[Example 1-1]

In a jacket-type separable flask equipped with a Dimroth condenser tube, a stirring blade, and a thermometer, 80 g of the polycarbonate resin (because the repeating unit of the polycarbonate resin is 254 g/mol, 80 g / 254 g/mol = 0.315 mol in terms of the repeating unit), 30 g of water, 250 g of phenol, and 320 g of a 25% by mass sodium hydroxide aqueous solution were placed at room temperature in a nitrogen atmosphere. The reaction liquid was slurry-like.

After that, the internal temperature was raised to 80°C, and the reaction was carried out for 5 hours while maintaining 80°C, to obtain a uniform solution.

200 g of toluene was added to the obtained uniform solution, and then when 35% by mass hydrochloric acid was added until the pH of the aqueous phase reached 8.6, carbon dioxide gas was generated.

After that, stirring was stopped, oil water separation was carried out, and the aqueous phase was withdrawn from the flask to obtain an organic phase 1. When the composition of part of the obtained organic phase 1 was checked by high performance liquid chromatography, bisphenol A was found to have been generated.

The obtained organic phase 1 was transferred to a distillation apparatus equipped with a thermometer, a stirring blade, a distilling-out tube, and a pressure regulator, and while observing the amount distilled out, the internal temperature was gradually raised to 180°C and the internal pressure was gradually lowered from normal pressure to 100 hPa to distill off water, toluene, and phenol.

After that, the pressure inside the flask was restored with nitrogen, the internal temperature was lowered to 80°C, and 200 g of toluene was added to obtain an organic phase 2. The obtained organic phase 2 was washed 5 times with 50 g of desalinated water to obtain an organic phase 3.

The temperature of the obtained organic phase 3 was lowered to 20°C to obtain a slurry. The obtained slurry was filtered to obtain a cake.

The obtained cake was dried on a rotary evaporator to obtain 35 g of bisphenol A. The purity of the obtained bisphenol A was 99.8% by mass, and the molten color thereof was APHA 165.

[Example 1-2]

The same procedure as in Example 1-1 was carried out except that in Example 1-1, 320 g of a 25% by mass potassium hydroxide aqueous solution was used instead of 320 g of a 25% by mass sodium hydroxide aqueous solution. The amount of the obtained bisphenol A was 41 g, the purity thereof was 99.8% by mass, and the molten color thereof was APHA 157.

(Example 2)

In a jacket-type separable flask equipped with a Dimroth condenser tube, a stirring blade, and a thermometer, 80 g (0.315 mol in terms of the repeating unit) of the polycarbonate resin, 30 g of water, 250 g of phenol, and 10 g of sodium carbonate were placed at room temperature in a nitrogen atmosphere. The reaction liquid was slurry-like.
After that, the internal temperature was raised to 85°C, and the reaction was carried out for 5 hours while maintaining 85°C, to obtain a uniform solution.
When the composition of part of the obtained uniform solution was checked by high performance liquid chromatography, the generation of bisphenol A was 19.2% by mass. The mass of the uniform solution was 80 g + 30 g + 250 g + 10 g = 370 g, the generated bisphenol was 19.2% by mass × 370 g / 228 g/mol = 0.312 mol, and the reaction rate was 0.312 mol / 0.315 mol × 100 = 99%.

(Example 3)

[Example 3-1]

In a jacket-type separable flask equipped with a Dimroth condenser tube, a stirring blade, and a thermometer, 80 g (0.315 mol in terms of the repeating unit) of the polycarbonate resin, 30 g of water, 240 g of phenol, and 2 g of a 40% by mass methylamine aqueous solution were placed at room temperature in a nitrogen atmosphere. The reaction liquid was slurry-like.
After that, the internal temperature was raised to 80°C, and the reaction was carried out for 5 hours while maintaining 80°C, to obtain a uniform solution. During the reaction, the generation of carbon dioxide was observed.
When part of the obtained uniform solution was taken out and the composition thereof was checked by high performance liquid chromatography, the generation of bisphenol A was 19.9% by mass.
The mass of the uniform solution was 80 g + 30 g + 240 g + 2 g = 352 g, the generated bisphenol was 19.2% by mass × 352 g / 228.29 g/mol = 0.307 mol, and the reaction rate was 0.307 mol / 0.315 mol × 100 = 97%.

[Example 3-2]

The same procedure as in Example 3-1 was carried out except that in Example 3-1, 2 g of a 50% by mass dimethylamine aqueous solution was added instead of 2 g of a 40% by mass methylamine aqueous solution.
When part of the obtained uniform solution was taken out and the composition thereof was checked by high performance liquid chromatography, the generation of bisphenol A was 19.6% by mass.
The mass of the uniform solution was 80 g + 30 g + 240 g + 2 g = 352 g, the generated bisphenol was 19.6% by mass × 352 g / 228.29 g/mol = 0.302 mol, and the reaction rate was 0.302 mol / 0.315 mol × 100 = 96%.

[Example 3-3]

The same procedure as in Example 3-1 was carried out except that in Example 3-1, 5 g of a trimethylamine aqueous solution was added instead of 2 g of a 40% by mass methylamine aqueous solution.
When part of the obtained uniform solution was taken out and the composition thereof was checked by high performance liquid chromatography, the generation of bisphenol A was 19.6% by mass.
The mass of the uniform solution was 80 g + 30 g + 240 g + 5 g = 355 g, the generated bisphenol was 19.6% by mass × 355 g / 228.29 g/mol = 0.304 mol, and the reaction rate was 0.304 mol / 0.315 mol × 100 = 97%.

[Example 3-4]

The same procedure as in Example 3-1 was carried out except that in Example 3-1, 5 g of a 70% by mass ethylamine aqueous solution was added instead of 2 g of a 40% by mass methylamine aqueous solution.
When part of the obtained uniform solution was taken out and the composition thereof was checked by high performance liquid chromatography, the generation of bisphenol A was 20.1% by mass.
The mass of the uniform solution was 80 g + 30 g + 240 g + 5 g = 355 g, the generated bisphenol was 20.1% by mass × 355 g / 228.29 g/mol = 0.313 mol, and the reaction rate was 0.313 mol / 0.315 mol × 100 = 99%.

[Example 3-5]

The same procedure as in Example 3-1 was carried out except that in Example 3-1, 10 g of diethylamine was added instead of 2 g of a 40% by mass methylamine aqueous solution.

When part of the obtained uniform solution was taken out and the composition thereof was checked by high performance liquid chromatography, the generation of bisphenol A was 19.7% by mass.
The mass of the uniform solution was 80 g + 30 g + 240 g + 10 g = 360 g, the generated bisphenol was 19.7% by mass × 360 g / 228.29 g/mol = 0.311 mol, and the reaction rate was 0.311 mol / 0.315 mol × 100 = 99%.

[Example 3-6]

The same procedure as in Example 3-1 was carried out except that in Example 3-1, 10 g of triethylamine was added instead of 2 g of a 40% by mass methylamine aqueous solution.
When part of the obtained uniform solution was taken out and the composition thereof was checked by high performance liquid chromatography, the generation of bisphenol A was 19.6% by mass. The mass of the uniform solution was 80 g + 30 g + 240 g + 10 g = 360 g, the generated bisphenol was 19.6% by mass × 360 g / 228.29 g/mol = 0.309 mol, and the reaction rate was 0.309 mol / 0.315 mol × 100 = 98%.

[Example 3-7]

The same procedure as in Example 3-1 was carried out except that in Example 3-1, 10 g of tripropylamine was added instead of 2 g of a 40% by mass methylamine aqueous solution.
When part of the obtained uniform solution was taken out and the composition thereof was checked by high performance liquid chromatography, the generation of bisphenol A was 19.4% by mass. The mass of the uniform solution was 80 g + 30 g + 240 g + 10 g = 360 g, the generated bisphenol was 19.4% by mass × 360 g / 228.29 g/mol = 0.306 mol, and the reaction rate was 0.306 mol / 0.315 mol × 100 = 97%.
Results of Example 3-1 to Example 3-7 are shown in Table 1.

[Table 1]

| | Example 3-1 | Example 3-2 | Example 3-3 | Example 3-4 |
|---|---|---|---|---|
| Catalyst | Methylamine | Dimethylamine | Trimethylamine | Ethylamine |
| Organic solvent | Phenol | Phenol | Phenol | Phenol |
| Water | Yes | Yes | Yes | Yes |
| Reaction temperature | 80°C | 80°C | 80°C | 80°C |
| Reaction rate | 97% | 96% | 97% | 99% |

| | Example 3-5 | Example 3-6 | Example 3-7 |
|---|---|---|---|
| Catalyst | Diethylamine | Triethylamine | Tripropylamine |
| Organic solvent | Phenol | Phenol | Phenol |
| Water | Yes | Yes | Yes |
| Reaction temperature | 80°C | 80°C | 80°C |
| Reaction rate | 99% | 98% | 97% |

[Example 3-8]

The same procedure as in Example 3-6 was carried out except that in Example 3-6, the internal temperature was raised to 70°C and the reaction was carried out for 5 hours while maintaining 70°C, instead of raising the internal temperature to 80°C and carrying out the reaction for 5 hours while maintaining 80°C.
When part of the obtained uniform solution was taken out and the composition thereof was checked by high performance liquid chromatography, the generation of bisphenol A was 6.5% by mass. The mass of the solution was 80 g + 30 g + 240 g + 10 g = 360 g, the generated bisphenol was 6.5% by mass × 360 g / 228.29 g/mol = 0.103 mol, and the reaction rate was 0.103 mol / 0.315 mol × 100 = 33%.

[Example 3-8-2]

The same procedure as in Example 3-8 was carried out except that in Example 3-8, the reaction was carried out for 13 hours while maintaining 70°C, instead of carrying out the reaction for 5 hours while maintaining 70°C.

When part of the obtained uniform solution was taken out and the composition thereof was checked by high performance liquid chromatography, the generation of bisphenol A was 13.1% by mass. The mass of the solution was 80 g + 30 g + 240 g + 10 g = 360 g, the generated bisphenol was 13.1% by mass × 360 g / 228.29 g/mol = 0.207 mol, and the reaction rate was 0.207 mol / 0.315 mol × 100 = 66%.

Results of Example 3-8 and Example 3-8-2 are shown in Table 2.

[Table 2]

| | Example 3-8 | Example 3-8-2 |
|---|---|---|
| Catalyst | Triethylamine | Triethylamine |
| Organic solvent | Phenol | Phenol |
| Water | Yes | Yes |
| Reaction temperature | 70°C | 70°C |
| Reaction time | 5 hours | 13 hours |
| Reaction rate | 33% | 66% |

[Example 3-9]

The same procedure as in Example 3-6 was carried out except that in Example 3-6, the internal temperature was raised to 60°C and the reaction was carried out for 5 hours while maintaining 60°C, instead of raising the internal temperature to 80°C and carrying out the reaction for 5 hours while maintaining 80°C.

When part of the obtained uniform solution was taken out and the composition thereof was checked by high performance liquid chromatography, the generation of bisphenol A was 1.8% by mass. The mass of the solution was 80 g + 30 g + 240 g + 10 g = 360 g, the generated bisphenol was 1.8% by mass × 360 g / 228.29 g/mol = 0.028 mol, and the reaction rate was 0.028 mol / 0.315 mol × 100 = 9%.

[Example 3-9-2]

The same procedure as in Example 3-9 was carried out except that in Example 3-9, the reaction was carried out for 56 hours while maintaining 60°C, instead of carrying out the reaction for 5 hours while maintaining 60°C.

When part of the obtained uniform solution was taken out and the composition thereof was checked by high performance liquid chromatography, the generation of bisphenol A was 12.9% by mass. The mass of the solution was 80 g + 30 g + 240 g + 10 g = 360 g, the generated bisphenol was 12.9% by mass × 360 g / 228.29 g/mol = 0.203 mol, and the reaction rate was 0.203 mol / 0.315 mol × 100 = 64%.

Results of Example 3-9 and Example 3-9-2 are shown in Table 3.

[Table 3]

| | Example 3-9 | Example 3-9-2 |
|---|---|---|
| Catalyst | Triethylamine | Triethylamine |
| Organic solvent | Phenol | Phenol |
| Water | Yes | Yes |
| Reaction temperature | 60°C | 60°C |
| Reaction time | 5 hours | 56 hours |
| Reaction rate | 9% | 64% |

[Comparative Example 3-1]

In a jacket-type separable flask equipped with a Dimroth condenser tube, a stirring blade, and a thermometer, 80 g (0.315 mol in terms of the repeating unit) of the polycarbonate resin, 30 g of water, 240 g of methylene chloride, and 2 g of a 40% by mass methylamine aqueous solution were placed at room temperature in a nitrogen atmosphere.
After that, because the boiling point of methylene chloride was 40°C, the internal temperature was raised to 40°C, and the reaction was carried out for 5 hours while maintaining 40°C.
When part of the obtained uniform solution was taken out and the composition thereof was checked by high performance liquid chromatography, bisphenol A was obtained only in a trace amount.

[Comparative Example 3-2]

The same procedure as in Example 3-6 was carried out except that in Example 3-6, 30 g of water was not used. During the reaction, no generation of carbon dioxide was observed.
When part of the obtained uniform solution was taken out and the composition thereof was checked by high performance liquid chromatography, the generation of bisphenol A was 13.1% by mass.
The mass of the uniform solution was 80 g + 240 g + 10 g = 330 g, the generated bisphenol was 13.1% by mass $\times$ 322 g / 228 g/mol = 0.190 mol, and the reaction rate was 0.190 mol / 0.315 mol $\times$ 100 = 60%.
In addition, the amount of bisphenol A generated was at the same level and remained unchanged, even if the reaction time was further extended.

[Comparative Example 3-3]

The same procedure as in Comparative Example 3-2 was carried out except that in Comparative Example 3-2, the reaction temperature was changed from 80°C to 60°C. During the reaction, no generation of carbon dioxide was observed.
When part of the obtained uniform solution was taken out and the composition thereof was checked by high performance liquid chromatography, the amount of bisphenol A generated was a trace amount.
In addition, the amount of bisphenol A generated remained unchanged even if the reaction time was further extended.

[Comparative Example 3-4]

The same procedure as in Example 3-6 was carried out except that in Example 3-6, 240 g of phenol was not used.
In the obtained reaction liquid, most of the supplied polycarbonate resin remained as a solid. When part of the obtained reaction liquid was taken out and the composition thereof was checked by high performance liquid chromatography, a trace amount of bisphenol A was found to have been generated.
Results of Comparative Example 3-1 to Comparative Example 3-4 are shown in Table 4.

[Table 4]

| | Comparative Example 3-1 | Comparative Example 3-2 | Comparative Example 3-3 | Comparative Example 3-4 |
|---|---|---|---|---|
| Catalyst | Methylamine | Triethylamine | Triethylamine | Triethylamine |
| Organic solvent | Methylene chloride | Phenol | Phenol | No |
| Water | Yes | No | No | Yes |
| Reaction temperature | 40°C | 80°C | 60°C | 80°C |
| Reaction rate | Trace amount | 60% | Trace amount | Trace amount |

From Table 1 to Table 4, it can be seen that the polycarbonate resin can be efficiently degraded by using phenol and water in combination. In addition, a chlorinated hydrocarbon solvent such as methylene chloride is not used, and thus it can be seen that the degradation method has a small environmental load. In addition, in Examples 3-1 to 3-9-2,

the polycarbonate resin is degraded into bisphenol A and carbon dioxide, and thus it is also easy to recover and purify bisphenol A.

[Example 3-10]

The reaction liquid obtained in Example 3-6 was transferred to a distillation apparatus equipped with a thermometer, a stirring blade, a distilling-out tube, and a pressure regulator, and while observing the amount distilled out, the internal temperature was gradually raised to 180°C and the internal pressure was gradually lowered from normal pressure to 100 hPa to distill off water, triethylamine, and phenol.
After that, the pressure inside the flask was restored with nitrogen, the internal temperature was lowered to 80°C, and 200 g of toluene was added to obtain an organic phase 1.
The obtained organic phase 1 was washed 5 times with 50 g of desalinated water to obtain an organic phase 2. The temperature of the obtained organic phase 2 was lowered to 20°C to obtain a slurry. The obtained slurry was filtered to obtain a cake.
The obtained cake was dried on a rotary evaporator to obtain 32 g of bisphenol A. The purity of the obtained bisphenol A was 99.8% by mass, and the molten color thereof was APHA 155.

(Example 4)

[Example 4-1]

To a jacket-type separable flask equipped with a Dimroth condenser tube, a stirring blade, and a thermometer, 240 g of orthocresol and 30 g of water were supplied in a nitrogen atmosphere, then 15 g of triethylamine was added, and the resulting mixture was stirred. After that, 80 g (0.315 mol in terms of the repeating unit) of the polycarbonate resin was added.
After that, the internal temperature was raised to 80°C, and the reaction was carried out for 5 hours while maintaining 80°C, to obtain a uniform solution.
When part of the obtained uniform solution was taken out and the composition thereof was checked by high performance liquid chromatography, the generation of bisphenol A was 19.4% by mass. The mass of the uniform solution was 240 g + 30 g + 15 g + 80 g = 365 g, the generated bisphenol was 19.4% by mass × 365 g / 228.29 g/mol = 0.310 mol, and the reaction rate was 0.310 mol / 0.315 mol × 100 = 98%.
The obtained reaction liquid was cooled to 20°C and allowed to stand for 12 hours to obtain a slurry liquid. The obtained slurry liquid was filtered under reduced pressure to obtain a cake 1.
The obtained cake 1 was suspended and washed with toluene to obtain 21 g of a cake 2.
When the composition of part of the cake 2 was checked by high performance liquid chromatography, orthocresol was 0.5% by mass and bisphenol A was 78.2% by mass. Orthocresol included in the cake 2 was 21 g × 0.5% by mass / 108 g/mol = 1 mmol, bisphenol A is 21 g × 78.2% by mass / 228 g/mol = 72 mmol, and the cake 2 was found to be a cake of bisphenol A.

[Example 4-2]

The same procedure as in Example 4-1 was carried out except that in Example 4-1, metacresol was used instead of orthocresol. 20 g of a cake 2 was obtained.
When the composition of part of the cake 2 was checked by high performance liquid chromatography, metacresol was 0.6% by mass and bisphenol A was 78.5% by mass. Metacresol included in the cake 2 was 20 g × 0.6% by mass / 108 g/mol = 1 mmol, bisphenol A is 20 g × 78.5% by mass / 228 g/mol = 69 mmol, and the cake 2 was found to be a cake of bisphenol A.

[Example 4-3]

The same procedure as in Example 4-1 was carried out except that in Example 4-1, a cresol isomer mixture was used instead of orthocresol. 22 g of a cake 2 was obtained.
When the composition of part of the cake 2 was checked by high performance liquid chromatography, the cresol isomer mixture was 0.3% by mass and bisphenol A was 79.7% by mass. The cresol isomer mixture included in the cake 2 was 22 g × 0.3% by mass / 108 g/mol = 1 mmol, bisphenol A is 22 g × 79.7% by mass / 228 g/mol = 77 mmol, and the cake 2 was found to be a cake of bisphenol A.

[Example 4-4]

The cake obtained in Example 4-1 was placed in a separable flask equipped with a thermometer, a stirring blade, and a condenser tube. Further, 50 g of toluene and 50 g of desalinated water were added, and the temperature was raised to 80°C to obtain an organic phase 1. The obtained organic phase 1 was washed 5 times with 50 g of desalinated water to obtain an organic phase 2.
The temperature of the obtained organic phase 2 was lowered to 20°C to obtain a slurry. The obtained slurry was filtered to obtain a cake.
The obtained cake was dried on a rotary evaporator to obtain 11 g of bisphenol A. The purity of the obtained bisphenol A was 99.8% by mass, and the molten color thereof was APHA 95.

(Example 5)

The same procedure as in Example 4-1 was carried out except that in Example 4-1, phenol was used instead of orthocresol. 25 g of a cake 2 was obtained.
When the composition of part of the cake 2 was checked by high performance liquid chromatography, phenol was 23% by mass and bisphenol A was 57% by mass. Phenol included in the cake 2 was 25 g $\times$ 23% by mass / 94 g/mol = 61 mmol, bisphenol A is 25 g $\times$ 57% by mass / 228 g/mol = 63 mmol, and the cake 2 was found to be a cake of a co-crystal of phenol and bisphenol A.
The organic solvents and cakes in Examples 4-1 to 4-3 and Example 5 are summarized in Table 5.
From the results of Examples 4-1 to 4-3, it can be seen that a cake of bisphenol A can be obtained by using a cresol without distilling off phenol.
In addition, from the results of Example 5, it can be seen that a co-crystal of bisphenol A and phenol can be obtained by crystallization in the presence of phenol.

[Table 5]

| | Example 4-1 | Example 4-2 | Example 4-3 | Example 5 |
|---|---|---|---|---|
| Aromatic monoalcohol | Orthocresol | Metacresol | Cresol isomer mixture | Phenol |
| Distilling off of aromatic monoalcohol | No | No | No | No |
| Cake | Cake of bisphenol A | Cake of bisphenol A | Cake of bisphenol A | Cake of co-crystal of bisphenol A and phenol |

(Example 6)

[Example 6-1]

In a jacket-type separable flask equipped with a Dimroth condenser tube, a stirring blade, and a thermometer, 80 g (0.315 mol in terms of the repeating unit) of the polycarbonate resin, 100 g of water, 240 g of phenol, and 80 g of p-toluenesulfonic acid were placed at room temperature in a nitrogen atmosphere.
After that, the internal temperature was raised to 80°C, and the reaction was carried out for 1 hour while maintaining 80°C, to obtain a reaction liquid. During the reaction, the generation of carbon dioxide was observed.
When part of the obtained reaction liquid was taken out and the composition thereof was checked by high performance liquid chromatography, the generation of bisphenol A was 13.2% by mass. The mass of the reaction liquid was 80 g + 100 g + 240 g + 80 g = 500 g, the generated bisphenol was 13.2% by mass $\times$ 500 g / 228.29 g/mol = 0.289 mol, and the reaction rate was 0.289 mol / 0.315 mol $\times$ 100 = 92%.

[Example 6-2]

The same procedure as in Example 6-1 was carried out except that in Example 6-1, 40 g of methanesulfonic acid was added instead of 80 g of p-toluenesulfonic acid.
When part of the obtained reaction liquid was taken out and the composition thereof was checked by high performance liquid chromatography, the generation of bisphenol A was 14.3% by mass. The mass of the reaction liquid was 80 g + 100 g + 240 g + 40 g = 460 g, the generated bisphenol was 14.3% by mass $\times$ 460 g / 228.29 g/mol = 0.288

mol, and the reaction rate was 0.288 mol / 0.315 mol × 100 = 91%.

[Example 6-3]

The same procedure as in Example 6-1 was carried out except that in Example 6-1, 240 g of 35% hydrochloric acid was added instead of 80 g of p-toluenesulfonic acid.

When part of the obtained reaction liquid was taken out and the composition thereof was checked by high performance liquid chromatography, the generation of bisphenol A was 8.5% by mass. The mass of the reaction liquid was 80 g + 100 g + 240 g + 240 g = 660 g, the generated bisphenol was 8.5% by mass × 660 g / 228.29 g/mol = 0.246 mol, and the reaction rate was 0.246 mol / 0.315 mol × 100 = 78%.

[Example 6-4]

The same procedure as in Example 6-1 was carried out except that in Example 6-1, 100 g of 98% sulfuric acid was added instead of 80 g of p-toluenesulfonic acid.

When part of the obtained reaction liquid was taken out and the composition thereof was checked by high performance liquid chromatography, the generation of bisphenol A was 11.8% by mass. The mass of the reaction liquid was 80 g + 100 g + 240 g + 100 g = 520 g, the generated bisphenol was 11.8% by mass × 520 g / 228.29 g/mol = 0.269 mol, and the reaction rate was 0.269 mol / 0.315 mol × 100 = 85%.

[Example 6-5]

The same procedure as in Example 6-1 was carried out except that in Example 6-1, 200 g of 85% phosphoric acid was added instead of 80 g of p-toluenesulfonic acid.

When part of the obtained reaction liquid was taken out and the composition thereof was checked by high performance liquid chromatography, the generation of bisphenol A was 9.6% by mass. The mass of the reaction liquid was 80 g + 100 g + 240 g + 200 g = 620 g, the generated bisphenol was 9.6% by mass × 620 g / 228.29 g/mol = 0.261 mol, and the reaction rate was 0.261 mol / 0.315 mol × 100 = 83%.

The acids and reaction rates in Examples 6-1 to 6-5 are summarized in Table 6. As a result, it can be seen that the polycarbonate resin can be degraded by using an acid.

[Table 6]

| | Example 6-1 | Example 6-2 | Example 6-3 | Example 6-4 | Example 6-5 |
|---|---|---|---|---|---|
| Acid | p-Toluenesulfonic acid | Methanesulfonic acid | 35% Hydrochloric acid | 98% Sulfuric acid | 85% Phosphoric acid |
| Reaction rate | 92% | 91% | 78% | 85% | 83% |

[Example 6-6]

200 g of toluene was added to the reaction liquid obtained in Example 6-1 and then a 25% sodium hydroxide aqueous solution was added to adjust the pH to 9.1. The aqueous phase was removed to obtain an organic phase 1.

The obtained organic phase 1 was transferred to a distillation apparatus equipped with a thermometer, a stirring blade, a distilling-out tube, and a pressure regulator, and while observing the amount distilled out, the internal temperature was gradually raised to 180°C and the internal pressure was gradually lowered from normal pressure to 100 hPa to distill off water and phenol. After that, the pressure inside the flask was restored with nitrogen, the internal temperature was lowered to 80°C, and 200 g of toluene was added to obtain an organic phase 2. The obtained organic phase 2 was washed 5 times with 50 g of desalinated water to obtain an organic phase 3.

The temperature of the obtained organic phase 3 was lowered to 20°C to obtain a slurry. The obtained slurry was filtered to obtain a cake. The obtained cake was dried on a rotary evaporator to obtain 25 g of bisphenol A. The purity of the obtained bisphenol A was 99.8% by mass, and the molten color thereof was APHA 162.

(Example 7)

[Example 7-1]

In a glass reaction tank having an internal volume of 45 mL equipped with a stirrer and a distilling-out tube, 10.00 g (0.04 mol) of bisphenol A obtained in Example 1-1, 9.95 g (0.05 mol) of diphenyl carbonate, and 18 μL of a 400 ppm by mass cesium carbonate aqueous solution were placed. The operation of reducing the pressure of the glass reaction tank to about 100 Pa and subsequently restoring the pressure to atmospheric pressure with nitrogen was repeated three times to purge the inside of the reaction tank with nitrogen. After that, the reaction tank was immersed in an oil bath at 220°C to dissolve the contents thereof.

The rotation speed of the stirrer was set to 100 revolutions per minute, and the pressure inside the reaction tank was reduced from 101.3 kPa to 13.3 kPa in absolute pressure over 40 minutes while distilling off phenol by-produced by the oligomerization reaction of bisphenol A and diphenyl carbonate in the reaction tank. Subsequently, an ester exchange reaction was carried out for 80 minutes while holding the pressure inside the reaction tank at 13.3 kPa and further distilling off phenol. After that, the temperature outside the reaction tank was raised to 290°C, and the pressure inside the reaction tank was reduced from 13.3 kPa to 399 Pa in absolute pressure over 40 minutes to remove the distilled-out phenol outside the system. After that, the absolute pressure of the reaction tank was reduced to 30 Pa, and a polycondensation reaction was carried out. The polycondensation reaction was completed when the stirrer in the reaction tank had a predetermined stirring power. The time from the temperature raising to 290°C to the completion of the polymerization was 120 minutes.

Next, the pressure of the reaction tank was restored to 101.3 kPa in absolute pressure with nitrogen, then the pressure was increased to 0.2 MPa in gauge pressure, and the polycarbonate resin was withdrawn from the reaction tank to obtain a recycled polycarbonate resin.

The viscosity average molecular weight (Mv) of the obtained recycled polycarbonate resin was 27100.

[Example 7-2]

The same procedure as in Example 7-1 was carried out except that in Example 7-1, bisphenol A obtained in Example 3-10 was used instead of bisphenol A obtained in Example 1-1.

The viscosity average molecular weight (Mv) of the obtained recycled polycarbonate resin was 26800.

[Example 7-3]

The same procedure as in Example 7-1 was carried out except that in Example 7-1, bisphenol A obtained in Example 4-4 was used instead of bisphenol A obtained in Example 1-1.

The viscosity average molecular weight (Mv) of the obtained recycled polycarbonate resin was 24000.

[Example 7-4]

The same procedure as in Example 7-1 was carried out except that in Example 7-1, bisphenol A obtained in Example 6-6 was used instead of bisphenol A obtained in Example 1-1.

The viscosity average molecular weight (Mv) of the obtained recycled polycarbonate resin was 26000.

(Example 8)

[Example 8-1]

8-1-1: Obtaining neutralized wastewater

Diphenyl carbonate was produced according to ▲2▼ Production of diphenyl carbonate (paragraphs 0050 and 0051) of Example 12 of the patent literature Japanese Patent Application Laid-Open No. 2004-345883, and the aqueous phase generated by liquid separation in the neutralization mixing tank was obtained as a neutralized wastewater. The composition of the obtained neutralized wastewater was such that pyridine was 0.3% by mass, phenol was 1.4% by mass, and sodium chloride was 4% by mass.

8-1-2: Production of bisphenol (degradation of polycarbonate resin)

In a jacket-type separable flask equipped with a Dimroth condenser tube, a stirring blade, and a thermometer,

the polycarbonate resin (80 g, 0.315 mol in terms of the repeating unit), pyridine (2 g), the neutralized wastewater obtained in the 8-1-1 (29 g), and phenol (240 g) were placed at room temperature in a nitrogen atmosphere. The reaction liquid was slurry-like. The mass of the reaction liquid was 80 g + 2 g + 29 g + 240 g = 351 g. After that, the temperature was raised to 85°C, and the reaction was carried out for 4 hours while maintaining 85°C.

When the composition of part of the obtained reaction liquid was checked by high performance liquid chromatography, bisphenol A was 15.5% by mass (15.5 / 100 × 351 g = 54.4 g, and the degradation rate of the polycarbonate resin was 54.4 g / 228 g/mol / 0.315 mol × 100 = 75.7 mol%).

The obtained reaction liquid was transferred to a distillation apparatus equipped with a thermometer, a stirring blade, a distilling-out tube, and a pressure regulator, and while observing the amount distilled out, the internal temperature was gradually raised to 180°C and the internal pressure was gradually lowered from normal pressure to 10 kPa to distill off water, pyridine, and phenol.

After that, the pressure inside the flask was restored with nitrogen, the internal temperature was lowered to 80°C, and 200 g of toluene was added to obtain an organic phase 1.

The obtained organic phase 1 was washed 5 times with 50 g of desalinated water to obtain an organic phase 2. The temperature of the obtained organic phase 2 was lowered to 20°C to obtain a slurry. The obtained slurry was filtered to obtain a cake.

The obtained cake was dried on a rotary evaporator to obtain 31 g of bisphenol A. The purity of the obtained bisphenol A was 99.8% by mass.

[Example 8-2]

Sodium chloride as a reagent and water (desalinated water) were mixed such that the concentration of sodium chloride was 4% by mass, to obtain a 4% by mass sodium chloride aqueous solution (makeup sodium chloride aqueous solution). The same procedure as in Example 8-1 was carried out except that in Example 8-1, the makeup sodium chloride aqueous solution (29 g) was used instead of the neutralized wastewater (29 g).

When the composition of part of the obtained reaction liquid was checked by high performance liquid chromatography, bisphenol A was 14.6% by mass (14.6 / 100 × 351 g = 51.2 g, and the degradation rate of the polycarbonate resin was 51.2 g / 228 g/mol / 0.315 mol × 100 = 71.3 mol%).

[Example 8-3]

The same procedure as in Example 8-1 was carried out except that in Example 8-1, water (desalinated water) (29 g) was used instead of the neutralized wastewater (29 g).

When the composition of part of the obtained reaction liquid was checked by high performance liquid chromatography, bisphenol A was 14.2% by mass (14.2 / 100 × 351 g = 49.8 g, and the degradation rate of the polycarbonate resin was 49.8 g / 228 g/mol / 0.315 mol × 100 = 69.3 mol%).

The types of the catalysts and the organic solvents, use or no use of water, sodium chloride, and a wastewater, and the reaction rates (degradation rates of the polycarbonate resin) in Examples 8-1 to 8-3 are summarized in Table 7. From Table 7, it can be seen that by using pyridine, phenol, water, and sodium chloride in combination, the degradation rate of the polycarbonate resin is improved as compared with when sodium chloride was not used. In addition, it can be seen that a wastewater from a diphenyl carbonate plant can be used. Because the wastewater from the diphenyl carbonate plant can be used, the wastewater can be recycled and the environmental load can be lowered.

[Table 7]

| | Example 8-1 | Example 8-2 | Example 8-3 |
|---|---|---|---|
| Catalyst | Pyridine | Pyridine | Pyridine |
| Organic solvent | Phenol | Phenol | Phenol |
| Water | Yes | Yes | Yes |
| Sodium chloride | Yes | Yes | No |
| Use of neutralized wastewater | Yes | No | No |
| Reaction rate | 75.7% | 71.3% | 69.3% |

[Example 8-4]

The same procedure as in Example 7-1 was carried out except that in Example 7-1, bisphenol A obtained in Example 8-1 was used instead of bisphenol A obtained in Example 1-1, and the time from the temperature raising to 290°C to the completion of the polymerization was changed from 120 minutes to 140 minutes.
The viscosity average molecular weight (Mv) of the obtained recycled polycarbonate resin was 26900.

(Example 9)

[Example 9-1]

9-1-1: Obtaining hydrochloric acid wastewater

Chlorine was produced according to ▲3▼ Production of chlorine of Example 12 of the patent literature Japanese Patent Application Laid-Open No. 2004-345883 (paragraphs 0055 to 0058), and aqueous hydrochloric acid continuously withdrawn from the bottom of a stripping distillation column was obtained as hydrochloric acid wastewater. The composition of the obtained hydrochloric acid wastewater was such that hydrogen chloride was 18% by mass and dibromophenol was 50 ppm by mass.

9-1-2: Production of bisphenol (degradation of polycarbonate resin)

In a jacket-type separable flask equipped with a Dimroth condenser tube, a stirring blade, and a thermometer, the polycarbonate resin (80 g, 0.315 mol in terms of the repeating unit), the hydrochloric acid wastewater obtained in the 9-1-1 (300 g), and phenol (200 g) were placed at room temperature in a nitrogen atmosphere. The reaction liquid was slurry-like. The mass of the reaction liquid was 80 g + 300 g + 200 g = 580 g. After that, the temperature was raised to 80°C, and the reaction was carried out for 60 minutes while maintaining 80°C.
When the composition of part of the obtained reaction liquid was checked by high performance liquid chromatography, bisphenol A was 8.0% by mass (8.0 / 100 × 580 g = 46.4 g, and the degradation rate of the polycarbonate resin was 46.4 g / 228 g/mol / 0.315 mol × 100 = 64.6 mol%).

[Example 9-2]

Dibromophenol as a reagent, 35% hydrochloric acid as a reagent, and water (desalinated water) were mixed such that dibromophenol was 50 ppm by mass and hydrogen chloride was 18% by mass, to obtain a hydrochloric acid aqueous solution (makeup hydrochloric acid waste liquid). The same procedure as in Example 9-1 was carried out except that in Example 9-1, the makeup hydrochloric acid waste liquid (300 g) was used instead of the hydrochloric acid wastewater (300 g).
When the composition of part of the obtained reaction liquid was checked by high performance liquid chromatography, bisphenol A was 7.7% by mass (7.7 / 100 × 580 g = 44.7 g, and the degradation rate of the polycarbonate resin was 44.7 g / 228 g/mol / 0.315 mol × 100 = 62.2 mol%).

[Example 9-3]

35% hydrochloric acid as a reagent and water (desalinated water) were used to prepare a hydrochloric acid aqueous solution (makeup hydrochloric acid aqueous solution) containing 18% by mass of hydrogen hydrochloride. The same procedure as in Example 9-1 was carried out except that in Example 9-1, the 18% by mass of hydrogen hydrochloride (makeup hydrochloric acid aqueous solution) (300 g) was used instead of the hydrochloric acid wastewater (300 g).
When the composition of part of the obtained reaction liquid was checked by high performance liquid chromatography, bisphenol A was 6.9% by mass (6.9 / 100 × 580 g = 40.0 g, and the degradation rate of the polycarbonate resin was 40.0 g / 228 g/mol / 0.315 mol × 100 = 55.7 mol%).
The types of the catalysts and the organic solvents, use or no use of water, dibromophenol, and a wastewater, and the reaction rates (degradation rates of the polycarbonate resin) in Examples 9-1 to 9-3 are summarized in Table 8. From Table 8, it can be seen that the polycarbonate resin can be degraded by using hydrochloric acid wastewater discharged from a hydrogen chloride recovery plant by-produced during the production of diphenyl carbonate. It can be seen that by using hydrochloric acid wastewater, the wastewater can be recycled, and the environmental load is low. In addition, it can be seen that when dibromophenol is contained, the degradation rate of the polycarbonate resin is further increased.

[Table 8]

| | Example 9-1 | Example 9-2 | Example 9-3 |
|---|---|---|---|
| Catalyst | 18% Hydrochloric acid | 18% Hydrochloric acid | 18% Hydrochloric acid |
| Organic solvent | Phenol | Phenol | Phenol |
| Water | Yes | Yes | Yes |
| Dibromophenol | Yes | Yes | No |
| Use of hydrochloric acid wastewater | Yes | No | No |
| Reaction rate | 64.6% | 62.2% | 55.7% |

[Example 9-4]

600 g of toluene was added to the reaction liquid obtained in Example 9-1 and the temperature was set to 80°C, and then a sodium hydroxide aqueous solution and a sodium bicarbonate aqueous solution were added until the pH of the aqueous phase reached 8.5.

After that, stirring was stopped, oil water separation was carried out, and the aqueous phase was withdrawn from the flask to obtain an organic phase 1.

The obtained organic phase 1 was transferred to a distillation apparatus equipped with a thermometer, a stirring blade, a distilling-out tube, and a pressure regulator, and while observing the amount distilled out, the internal temperature was gradually raised to 180°C and the internal pressure was gradually lowered from normal pressure to 10 kPa to distill off water, toluene, and phenol.

After that, the pressure inside the flask was restored with nitrogen, the internal temperature was lowered to 80°C, and 200 g of toluene was added to obtain an organic phase 2. The obtained organic phase 2 was washed 5 times with 50 g of desalinated water to obtain an organic phase 3.

The temperature of the obtained organic phase 3 was lowered to 20°C to obtain a slurry. The obtained slurry was filtered to obtain a cake.

The obtained cake was dried on a rotary evaporator to obtain 25 g of bisphenol A. The purity of the obtained bisphenol A was 99.8% by mass.

[Example 9-5]

The same procedure as in Example 7-1 was carried out except that in Example 7-1, bisphenol A obtained in Example 9-4 was used instead of bisphenol A obtained in Example 1-1, and the time from the temperature raising to 290°C to the completion of the polymerization was changed from 120 minutes to 140 minutes.

The viscosity average molecular weight (Mv) of the obtained recycled polycarbonate resin was 27200.

(Example 10)

[Example 10-1]

10-1-1: Obtaining sodium hydroxide wastewater

Phosgene was produced according to ▲1▼ Production of phosgene of Example 12 of the patent literature Japanese Patent Application Laid-Open No. 2004-345883, and part of caustic soda was withdrawn from a detoxification column through which a caustic soda aqueous solution was circulated, to obtain sodium hydroxide wastewater. The composition of the obtained sodium hydroxide wastewater was such that carbon tetrachloride was 50 ppm by mass, sodium chloride was 0.1% by mass, and sodium hydroxide was 25% by mass.

10-1-2: Production of bisphenol (degradation of polycarbonate resin)

In a jacket-type separable flask equipped with a Dimroth condenser tube, a stirring blade, and a thermometer, the polycarbonate resin (80 g, 0.315 mol in terms of the repeating unit), the sodium hydroxide wastewater (80 g) obtained in the 10-1-1, water (40 g), and phenol (400 g) were placed at room temperature in a nitrogen atmosphere. The reaction liquid was slurry-like. The mass of the reaction liquid was 80 g + 80 g + 40 g + 400 g = 600 g.

After that, the temperature was raised to 60°C, and the reaction was carried out for 70 minutes while maintaining 60°C.

When the composition of part of the obtained reaction liquid was checked by high performance liquid chromatography, bisphenol A was 9.2% by mass (9.2 / 100 $\times$ 600 g = 55.2 g, and the degradation rate of the polycarbonate resin was 55.2 g / 228 g/mol / 0.315 mol $\times$ 100 = 76.9 mol%).

[Example 10-2]

Carbon tetrachloride as a reagent, sodium chloride as a reagent, sodium hydroxide as a reagent, and water (desalinated water) were mixed such that carbon tetrachloride was 50 ppm by mass, sodium chloride was 0.1% by mass, and sodium hydroxide was 25% by mass, to obtain a sodium hydroxide aqueous solution (makeup sodium hydroxide waste liquid).

The same procedure as in Example 10-1 was carried out except that in Example 10-1, the makeup sodium hydroxide waste liquid (80 g) was used instead of the sodium hydroxide wastewater (80 g).

When the composition of part of the obtained reaction liquid was checked by high performance liquid chromatography, bisphenol A was 8.9% by mass (8.9 / 100 $\times$ 600 g = 53.4 g, and the degradation rate of the polycarbonate resin was 53.4 g / 228 g/mol / 0.315 mol $\times$ 100 = 74.3 mol%).

[Example 10-3]

A 25% by mass sodium hydroxide aqueous solution (makeup sodium hydroxide aqueous solution) was prepared by using sodium hydroxide as a reagent and water (desalinated water).

The same procedure as in Example 10-1 was carried out except that in Example 10-1, the makeup sodium hydroxide aqueous solution (80 g) was used instead of the sodium hydroxide wastewater (80 g).

When the composition of part of the obtained reaction liquid was checked by high performance liquid chromatography, bisphenol A was 8.5% by mass (8.5 / 100 $\times$ 600 g = 51.0 g, and the degradation rate of the polycarbonate resin was 51.0 g / 228 g/mol / 0.315 mol $\times$ 100 = 71.0 mol%).

The types of the catalysts and the organic solvents, use or no use of water, carbon tetrachloride, sodium chloride, and a wastewater, and the reaction rates (degradation rates of the polycarbonate resin) in Example 10-1 to Example 10-3 are summarized in Table 9. From Table 9, it can be seen that the polycarbonate resin can be degraded by using sodium hydroxide wastewater discharged from a detoxification treatment facility of a carbonyl chloride (phosgene) production plant. It can be seen that because sodium hydroxide wastewater can be used, the wastewater can be recycled and the environmental load is low. In addition, it can be seen that when sodium hydroxide wastewater is used, the degradation rate of the polycarbonate resin is further increased.

[Table 9]

| | Example 10-1 | Example 10-2 | Example 10-3 |
|---|---|---|---|
| Catalyst | NaOH | NaOH | NaOH |
| Organic solvent | Phenol | Phenol | Phenol |
| Water | Yes | Yes | Yes |
| Carbon tetrachloride | Yes | Yes | No |
| Sodium chloride | Yes | Yes | No |
| Use of sodium hydroxide wastewater | Yes | No | No |
| Reaction rate | 76.9% | 74.3% | 71.0% |

[Example 10-4]

In a jacket-type separable flask equipped with a Dimroth condenser tube, a stirring blade, and a thermometer, the polycarbonate resin (80 g, 0.315 mol in terms of the repeating unit), the sodium hydroxide wastewater obtained in the 10-1-1 (80 g), water (40 g), and phenol (400 g) were placed at room temperature in a nitrogen atmosphere. The reaction liquid was slurry-like. The mass of the reaction liquid was 80 g + 80 g + 40 g + 400 g = 600 g.

After that, the temperature was raised to 80°C, and the reaction was carried out for 70 minutes while maintaining 80°C.

When the composition of part of the obtained reaction liquid was checked by high performance liquid chromatography, bisphenol A was 11.8% by mass (11.8 / 100 × 600 g = 70.8 g, and the degradation rate of the polycarbonate resin was 70.8 g / 228 g/mol / 0.315 mol × 100 = 98.6 mol%).

600 g of toluene was added to the obtained reaction liquid, and then when 35% by mass hydrochloric acid was added until the pH of the aqueous phase reached 8.6, carbon dioxide gas was generated.

After that, stirring was stopped, oil water separation was carried out, and the aqueous phase was withdrawn from the flask to obtain an organic phase 1.

The obtained organic phase 1 was transferred to a distillation apparatus equipped with a thermometer, a stirring blade, a distilling-out tube, and a pressure regulator, and while observing the amount distilled out, the internal temperature was gradually raised to 180°C and the internal pressure was gradually lowered from normal pressure to 10 kPa to distill off water, toluene, and phenol.

After that, the pressure inside the flask was restored with nitrogen, the internal temperature was lowered to 80°C, and 200 g of toluene was added to obtain an organic phase 2. The obtained organic phase 2 was washed 5 times with 50 g of desalinated water to obtain an organic phase 3.

The temperature of the obtained organic phase 3 was lowered to 20°C to obtain a slurry. The obtained slurry was filtered to obtain a cake.

The obtained cake was dried on a rotary evaporator to obtain 26 g of bisphenol A. The purity of the obtained bisphenol A was 99.8% by mass.

[Example 10-5]

The same procedure as in Example 7-1 was carried out except that in Example 7-1, bisphenol A obtained in Example 10-4 was used instead of bisphenol A obtained in Example 1-1.

The viscosity average molecular weight (Mv) of the obtained recycled polycarbonate resin was 26500.

[Example 11]

In a four-necked flask having an internal volume of 1 L equipped with a thermometer, a stirrer, and a condenser tube, 46 g of bisphenol A obtained in Example 1-1, 259 g of epichlorohydrin, 100 g of isopropanol, and 36 g of water were placed, and the temperature was raised to 40°C to uniformly dissolve the bisphenol A, and then 38 g of a 48.5% by mass sodium hydroxide aqueous solution was added dropwise over 90 minutes. Simultaneously with the dropwise addition, the temperature was raised from 40°C to 65°C over 90 minutes. After that, the temperature was held at 65°C for 30 minutes to complete the reaction, the reaction liquid was transferred to a 1 L separatory funnel, 69 g of water at 65°C was added, and the resulting mixture was allowed to stand at 65°C for 1 hour. After the standing, the aqueous phase was withdrawn from the separated oil phase and aqueous phase to remove a by-produced salt and excess sodium hydroxide. After that, epichlorohydrin was completely removed under reduced pressure at 150°C.

After that, 102 g of methyl isobutyl ketone was added, the temperature was raised to 65°C to cause uniform dissolution, then 1.4 g of a 48.5% by mass sodium hydroxide aqueous solution was added and reacted for 60 minutes, and then 57 g of methyl isobutyl ketone was added, and washing with water was carried out 4 times by using 200 g of water.

After that, methyl isobutyl ketone was completely removed under reduced pressure at 150°C to obtain an epoxy resin of Example 11.

The epoxy equivalent of the obtained epoxy resin according to JIS K7236 (2009) and, as a result, was found to be 175 g/equivalent.

[Reference Example 11c]

The same procedure as in Example 11 was carried out except that bisphenol A (manufactured by Mitsubishi Chemical Corporation) was used instead of bisphenol A obtained in Example 1-1, to obtain an epoxy resin of Reference Example 11c.

The epoxy equivalent of the obtained epoxy resin according to JIS K7236 (2009) and, as a result, was found to be 173 g/equivalent.

(Epoxy resin composition, epoxy resin cured product, and evaluation of curing physical properties)

[Example 12]

The epoxy resin of Example 11, a curing agent (trade name RIKACID MH-700 manufactured by New Japan Chemical Co., Ltd.), and a curing catalyst (trade name CUREZOL 2E4MZ manufactured by Shikoku Chemicals Corporation) were weighed at the proportions shown in Table 10. Next, these were stirred and mixed at room temperature until uniformity was reached, to obtain an epoxy resin composition.

Two glass plates each with a release PET film attached were provided, and the glass plate spacing was adjusted to 3 mm with both the release PET films inside, to create a mold. The epoxy resin composition was cast into this mold and heated at 100°C for 3 hours and then at 140°C for 3 hours to obtain an epoxy resin cured product.

The obtained cured product was cut into a cylinder having a diameter of 1 cm and a thickness of 3 mm to obtain a test piece. By using a thermomechanical analyzer (TMA: TMA/SS6100 manufactured by Seiko Instruments Inc.) in compression mode, the test piece was subjected to the first temperature raising at 5°C/min (from 30°C to 200°C), the first temperature lowering at 10°C/min (from 200°C to 30°C), and the second temperature raising: 5°C/min and from 30°C to 200°C, to measure the second linear expansion coefficients $\alpha1$ and $\alpha2$.

[Reference Example 12c]

The epoxy resin of Reference Example 11, a curing agent (trade name RIKACID MH-700 manufactured by New Japan Chemical Co., Ltd.), and a curing catalyst (trade name CUREZOL 2E4MZ manufactured by Shikoku Chemicals Corporation) were weighed at the proportions shown in Table 10. Next, these were stirred and mixed at room temperature until uniformity was reached, to obtain an epoxy resin composition.

The obtained epoxy resin composition was cured in the same manner as in Example 12, the obtained epoxy resin cured product was evaluated, and the linear expansion coefficients $\alpha1$ and $\alpha2$ were determined.

[Table 10]

| | | Example 12 | Reference Example 12c |
|---|---|---|---|
| Recycled epoxy resin of Example 11 | Parts | 100 | - |
| Epoxy resin of Reference Example 11c | Parts | - | 100 |
| Curing agent | Parts | 94 | 95 |
| Curing catalyst | Parts | 1 | 1 |
| Linear expansion coefficient $\alpha1$ | ppm/°C | 61 | 62 |
| Linear expansion coefficient $\alpha2$ | ppm/°C | 181 | 185 |

[Evaluation of results]

From Table 10, it can be seen that the epoxy resin cured product of Example 12 has a lower linear expansion coefficient than the epoxy resin cured product of Reference Example 12c and superior thermal cracking resistance thereto.

[Reference Example 13c]

Tetraphenoxytitanium was synthesized according to the following procedure and used.

200 g (2.1 mol) of phenol and 100 mL of toluene were placed in a 500 mL three-necked flask equipped with a receiver and a distilling-out tube, and the inside of the flask was purged with a nitrogen flow. The flask was immersed in a 100°C oil bath to obtain a uniform solution. 57 g (0.2 mol) of tetraisopropoxytitanium was added thereto. When the internal temperature of the bottom of the flask was held at 100°C, distilling-out of generated i-propyl alcohol started. After that, the internal temperature was gradually raised to 116°C to distill out 80 mL of a distillate, which was a mixture of i-propyl alcohol and toluene. 50 mL of hexane was added to the obtained still residue, and then the resulting mixture was cooled to room temperature and crystallized. The precipitated red crystal was obtained by filtration and dried on a rotary evaporator equipped with an oil bath at an oil bath temperature of 140°C and a pressure of 50 Torr to obtain 60 g (0.1 mol) of tetraphenoxytitanium.

[Example 13]

13-1: Synthesis of dimethyl carbonate

The synthesis of dimethyl carbonate from carbon dioxide was carried out according to Non-Patent Literature ChemSusChem, 2013, Vol.6, pp.1341-1344.

0.4 g of cerium oxide fired at 600°C in advance, 10.4 g of cyanopyridine, and 1.6 g of methanol (1.6 g / 32 g/mol = 50 mmol) were placed in a 200 mL autoclave equipped with an induction stirring blade, a pressure gauge, and a thermometer. Carbon dioxide obtained in Example 1-1 was introduced thereinto into the autoclave by a compressor. After purging three times, the internal pressure of the autoclave was set to 5 MPa. After that, the autoclave was installed in an electric furnace, and the reaction was carried out at an internal temperature of 120°C for 12 hours. After the reaction, the autoclave was immersed in ice water to restore the internal pressure to normal pressure. Cerium oxide was removed by filtering the obtained reaction liquid to obtain 12.6 g of a mixed liquid.

When part of the obtained mixed liquid was analyzed by gas chromatography, it was found that dimethyl carbonate was 16.2% by mass (16.2% by mass × 12.6 g = 2.0 g, 2.0 g / 90 g/mol = 22 mmol), and the reaction rate was 22 mmol × 2 / 50 mmol × 100% = 88%.

The above operation was carried out a plurality of times to obtain 200 g of the mixed liquid. The obtained mixed liquid was placed in a 1 L eggplant-shaped flask, and then the flask was installed in an evaporator equipped with a water bath to remove the initial fraction to obtain 31 g of the main fraction. When part of the obtained main fraction was analyzed by gas chromatography, the purity of dimethyl carbonate was found to be 97% by mass.

13-2: Synthesis of diphenyl carbonate

In a flask equipped with a distilling-out tube and a stirring blade, 31 g of the main fraction (30 g of dimethyl carbonate), 40 g of dimethyl carbonate (70 g in total of dimethyl carbonate, 0.78 mol) as a reagent, 500 g (5.32 mol) of phenol, and 5 g of tetraphenoxytitanium were placed. Under normal pressure, generated methanol was distilled out together with dimethyl carbonate. After the distilling-out stopped, the pressure was set to 1 kPa, the temperature was gradually raised to 185°C, and the reaction was carried out while distilling off dimethyl carbonate. After that, the oil bath was set to 210°C to obtain 11 g of diphenyl carbonate.

13-3: Synthesis of recycled polycarbonate resin

In a glass reaction tank having an internal volume of 45 mL equipped with a stirrer and a distilling-out tube, 10.00 g (0.04 mol) of bisphenol A, 9.95 g (0.05 mol) of the obtained diphenyl carbonate, and 18 μL of a 400 ppm by mass cesium carbonate aqueous solution were placed. The operation of reducing the pressure of the glass reaction tank to about 100 Pa and subsequently restoring the pressure to atmospheric pressure with nitrogen was repeated three times to purge the inside of the reaction tank with nitrogen. After that, the reaction tank was immersed in an oil bath at 220°C to dissolve the contents thereof.

The rotation speed of the stirrer was set to 100 revolutions per minute, and the pressure inside the reaction tank was reduced from 101.3 kPa to 13.3 kPa in absolute pressure over 40 minutes while distilling off phenol by-produced by the oligomerization reaction of bisphenol A and diphenyl carbonate in the reaction tank.

Subsequently, an ester exchange reaction was carried out for 80 minutes while holding the pressure inside the reaction tank at 13.3 kPa and further distilling off phenol.

After that, the temperature outside the reaction tank was raised to 290°C, and the pressure inside the reaction tank was reduced from 13.3 kPa to 399 Pa in absolute pressure over 40 minutes to remove the distilled-out phenol outside the system.

After that, the absolute pressure of the reaction tank was reduced to 30 Pa, and a polycondensation reaction was carried out. The polycondensation reaction was completed when the stirrer in the reaction tank had a predetermined stirring power. The time from the temperature raising to 290°C to the completion of the polymerization was 120 minutes.

Next, the pressure of the reaction tank was restored to 101.3 kPa in absolute pressure with nitrogen, then the pressure was increased to 0.2 MPa in gauge pressure, and the polycarbonate resin was withdrawn from the reaction tank to obtain a polycarbonate resin.

The viscosity average molecular weight (Mv) of the obtained polycarbonate resin was 24800.

Industrial Applicability

According to the method for producing a bisphenol according to the present invention, a bisphenol can be obtained from a waste plastic or the like by using chemical recycling. Further, by using this bisphenol, a polycarbonate

resin can be produced again, which is industrially useful.

Reference Signs List

| | |
|---|---|
| 1 | diphenyl carbonate production plant |
| 2 | Hydrogen chloride recovery plant |
| 3 | carbonyl chloride production plant |
| 10 | DPC reactor |
| 11 | dehydrochlorination column |
| 12 | mixing tank |
| 13 | neutralization tank |
| 14 | water washing tank |
| 15, 16 | distillation column |
| 20 | activated carbon column |
| 21 | absorption column |
| 22, 24, 32 | tank |
| 23 | stripping column |
| 30 | CDC reactor |
| 31 | Aggregator |
| 33 | evaporator |
| 34 | detoxification column |
| 100, 102, 103 | degradation tank |
| G10, G12, G20 | hydrogen chloride gas |
| G30 | crude carbonyl chloride gas |
| G31 | unliquefied gas |
| G32 | waste gas |
| G13 | diphenyl carbonate |
| L10 | reaction liquid including diphenyl carbonate |
| L11 | reaction liquid after dehydrochlorination treatment |
| L12, L31 | sodium hydroxide aqueous solution |
| L13, L16 | oil phase |
| L14 | aqueous phase (neutralized wastewater) |
| L15, L20 | water |
| L17 | aqueous phase |
| L21, L24, L26 | dilute hydrochloric acid |
| L23 | concentrated hydrochloric acid |
| L25 | hydrochloric acid wastewater |
| L30 | carbonyl chloride |
| L32 | sodium hydroxide waste liquid |
| L100, L102, L103 | solution including bisphenol A |
| CDC, G1 | carbonyl chloride gas |
| CO | carbon monoxide gas |
| $CL_2$ | chlorine gas |
| $H_2O$ | water |
| PC | polycarbonate resin |
| PL | phenol |
| PRD | pyridine |
| acid | acid |
| base | base |

**Claims**

1. A method for producing a bisphenol, comprising degrading a polycarbonate resin in the presence of an aromatic monoalcohol, water, and a catalyst.

2. The method for producing a bisphenol according to claim 1, wherein the catalyst is any selected from the group consisting of an alkali metal hydroxide, an alkali metal carbonate, an alkylamine, a nitrogen-containing heterocyclic compound, and an acid.

3. The method for producing a bisphenol according to claim 2, wherein the alkali metal hydroxide is sodium hydroxide or potassium hydroxide.

4. The method for producing a bisphenol according to claim 2, wherein the alkylamine is represented by the following formula (I) :

[Formula 1]

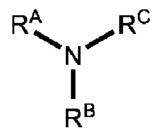

...(I)

wherein $R^A$ represents an alkyl group having 1 to 3 carbon atoms, and $R^B$ and $R^C$ each independently represent a hydrogen atom or an alkyl group having 1 to 3 carbon atoms.

5. The method for producing a bisphenol according to claim 2 or 4, wherein the alkylamine is a tertiary amine.

6. The method for producing a bisphenol according to claim 2, wherein the acid is any selected from the group consisting of hydrochloric acid, sulfuric acid, phosphoric acid, and a sulfonic acid.

7. The method for producing a bisphenol according to claim 1, wherein the catalyst comprises a nitrogen-containing heterocyclic compound, and
the polycarbonate resin is degraded in the coexistence of an alkali metal chloride in addition to the aromatic monoalcohol, the water, and the catalyst.

8. The method for producing a bisphenol according to claim 2 or 7, wherein the nitrogen-containing heterocyclic compound is a pyridine.

9. The method for producing a bisphenol according to claim 7, wherein the alkali metal chloride is sodium chloride.

10. The method for producing a bisphenol according to any one of claims 1 to 9, wherein a reaction temperature for degrading the polycarbonate resin is 110°C or less.

11. The method for producing a bisphenol according to any one of claims 1 to 10, wherein the polycarbonate resin is degraded in a slurry-like reaction liquid comprising the polycarbonate resin, the aromatic monoalcohol, the water, and the catalyst.

12. The method for producing a bisphenol according to any one of claims 1 to 11, wherein a mass ratio of the water to the aromatic monoalcohol is 0.001 or more and 10 or less.

13. The method for producing a bisphenol according to claim 1, wherein the catalyst comprises hydrochloric acid, and the polycarbonate resin is degraded in the coexistence of a bromophenol in addition to the aromatic monoalcohol, the water, and the catalyst.

14. The method for producing a bisphenol according to claim 1, wherein the catalyst comprises sodium hydroxide, and the polycarbonate resin is degraded in the coexistence of sodium chloride and/or carbon tetrachloride in addition to the aromatic monoalcohol, the water, and the catalyst.

15. The method for producing a bisphenol according to any one of claims 1 to 14, wherein the aromatic monoalcohol is any selected from the group consisting of phenol, a cresol, and a xylenol.

16. The method for producing a bisphenol according to any one of claims 1 to 15, wherein the bisphenol is 2,2-bis(4-hydroxyphenyl)propane.

**17.** The method for producing a bisphenol according to claim 1 or 7, wherein when a diaryl carbonate is produced by a method comprising the following step (a1), step (b1), step (b2), and step (b3), a neutralized wastewater removed in the step (b1) is used for degrading the polycarbonate resin:

Step (a1): A step of reacting carbonyl chloride with an aromatic monoalcohol in the presence of a nitrogen-containing heterocyclic compound to obtain a reaction liquid comprising a diaryl carbonate
Step (b1): A step of neutralizing the reaction liquid comprising a diaryl carbonate obtained in the step (a1) with an alkali metal hydroxide aqueous solution, carrying out oil water separation thereof into an oil phase comprising an aromatic diaryl and an aqueous phase comprising the nitrogen-containing heterocyclic compound and an alkali metal chloride, and then removing the aqueous phase as a neutralized wastewater
Step (b2): A step of washing the oil phase obtained in the step (b1) with water
Step (b3): A step of obtaining the diaryl carbonate from the oil phase after the step (b2).

**18.** The method for producing a bisphenol according to claim 17,

wherein the alkali metal chloride in the step (b1) is sodium chloride, and
the alkali metal hydroxide aqueous solution in the step (b1) is a sodium hydroxide aqueous solution.

**19.** The method for producing a bisphenol according to claim 1 or 13, wherein in production of a diaryl carbonate and recovery of hydrogen chloride by-produced comprising the following step (a1), step (c1), step (c2), and step (c3), hydrochloric acid wastewater removed in the step (c3) is used for degrading the polycarbonate resin:

Step (a1): A step of reacting carbonyl chloride with an aromatic monoalcohol in the presence of a nitrogen-containing heterocyclic compound to obtain a reaction liquid comprising a diaryl carbonate
Step (c1): A step of supplying hydrogen chloride by-produced in the step (a1) to an absorption column and absorbing the same into water or dilute hydrochloric acid to obtain concentrated hydrochloric acid
Step (c2): A step of distilling the concentrated hydrochloric acid in a stripping column, recovering hydrogen chloride gas from the top of the column, and recovering hydrochloric acid from the bottom of the column
Step (c3): A step of removing part of the hydrochloric acid recovered from the bottom of the column as hydrochloric acid wastewater outside a system and circulating the remaining hydrochloric acid to the absorption column of the step (c1).

**20.** The method for producing a bisphenol according to claim 1 or 14, wherein in production of carbonyl chloride and treatment of an unliquefied gas comprising the following step (d1) to step (d4), sodium hydroxide wastewater removed in the step (d4) is used for degrading the polycarbonate resin:

Step (d1): A step of obtaining carbonyl chloride gas from chlorine and carbon monoxide
Step (d2): A step of cooling the carbonyl chloride gas obtained in the step (d1) to obtain liquefied carbonyl chloride
Step (d3): A step of contacting a circulating sodium hydroxide aqueous solution with an unliquefied gas that has not been liquefied in the (d2) to degrade carbonyl chloride in the unliquefied gas, and then discharging the resulting unliquefied gas
Step (d4); A step of removing part of the circulating sodium oxide aqueous solution as sodium hydroxide wastewater.

**21.** A method for producing a recycled polycarbonate resin, comprising producing a recycled polycarbonate resin by using a bisphenol raw material comprising a bisphenol obtained by the method for producing a bisphenol according to any one of claims 1 to 20.

**22.** A method for producing carbon dioxide, comprising recovering carbon dioxide generated by the method for producing a bisphenol according to any one of claims 1 to 20.

**23.** A method for producing a carbonic acid diester, comprising producing a carbonic acid diester by using carbon dioxide obtained by the method for producing carbon dioxide according to claim 22.

**24.** The method for producing a carbonic acid diester according to claim 23, wherein the method comprises a step of reacting carbon dioxide comprising the carbon dioxide with an aliphatic monoalcohol.

**25.** The method for producing a carbonic acid diester according to claim 23, wherein carbon monoxide is obtained from carbon dioxide comprising the carbon dioxide and coke, the obtained carbon monoxide is reacted with chlorine to obtain carbonyl chloride, and the obtained carbonyl chloride is reacted with an aromatic monoalcohol to obtain the carbonic acid diester.

**26.** A method for producing a recycled polycarbonate resin, comprising producing a recycled polycarbonate resin by using a carbonic acid diester raw material comprising a carbonic acid diester obtained by the method for producing a carbonic acid diester according to any one of claims 23 to 25.

**27.** A method for producing an epoxy resin, comprising producing an epoxy resin by using a bisphenol obtained by the method for producing a bisphenol according to any one of claims 1 to 20.

**28.** The method for producing an epoxy resin according to claim 27, wherein the epoxy resin is further reacted with a polyhydroxy compound raw material.

**29.** A method for producing an epoxy resin cured product, comprising curing an epoxy resin composition comprising an epoxy resin obtained by the method for producing an epoxy resin according to claim 27 or 28 and a curing agent to obtain an epoxy resin cured product.

FIG. 1

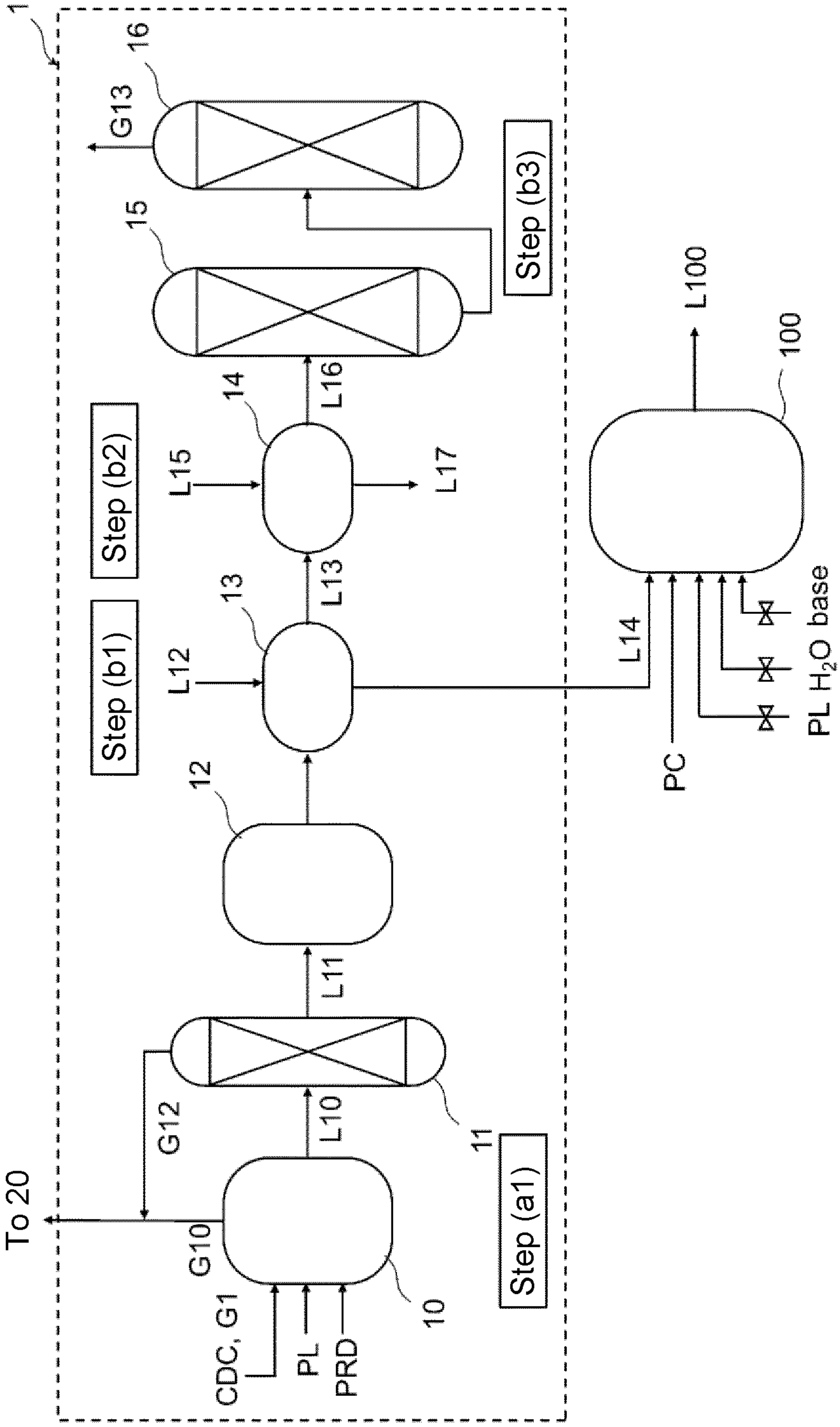
1
16
G13
15
Step (b3)
L100
100
14
L16
Step (b2)
L15
L17
13
L13
Step (b1)
L12
L14
PC
PL $H_2O$ base
12
L11
G12
L10
11
Step (a1)
To 20
G10
CDC, G1
PL
PRD
10

FIG. 2

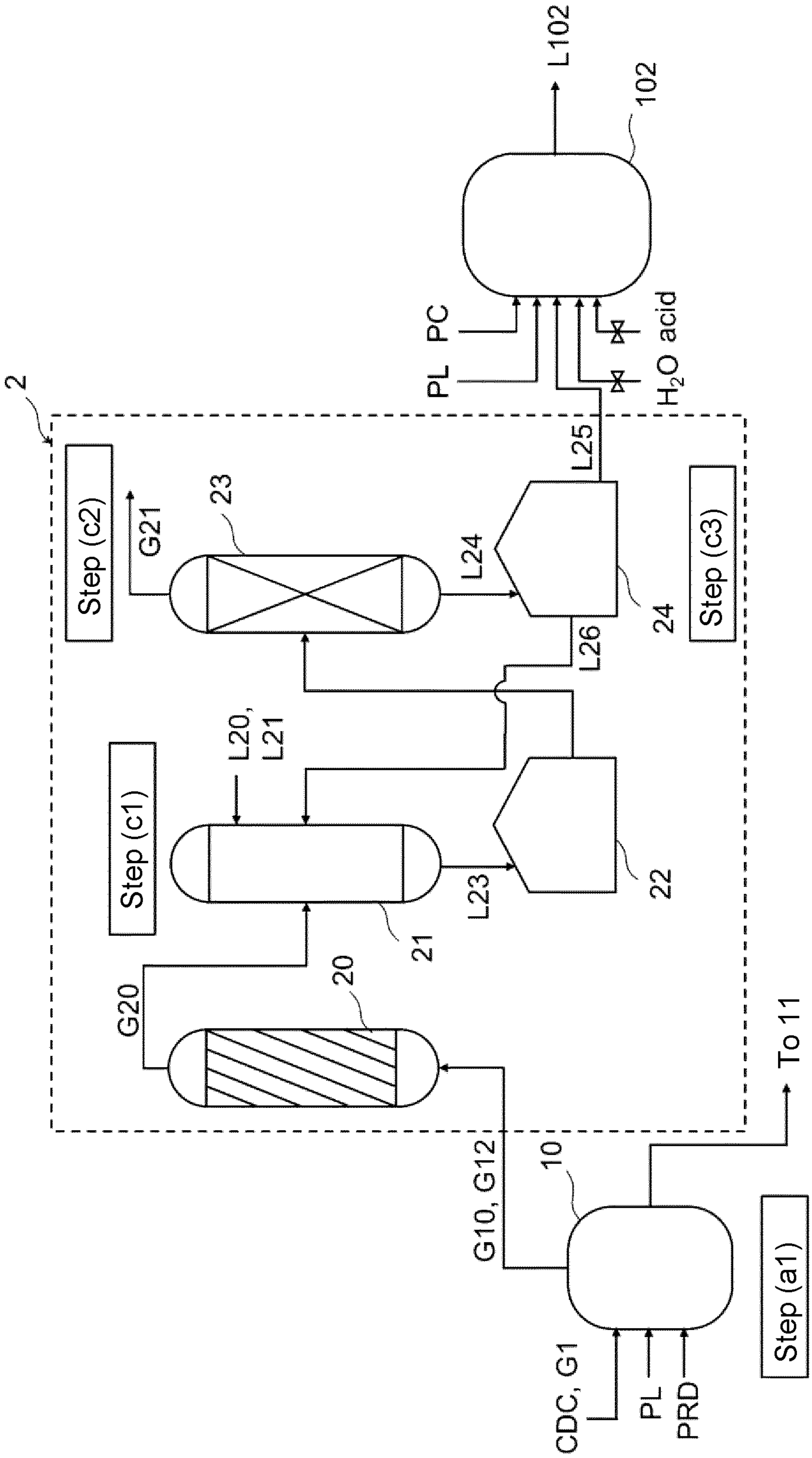
2
Step (c2)
G21
23
L24
L25
Step (c3)
24
L26
L20,
L21
Step (c1)
21
22
L23
20
G20
To 11
10
G10, G12
CDC, G1
PL
PRD
Step (a1)
PL
PC
$H_2O$
acid
102
L102

FIG. 3

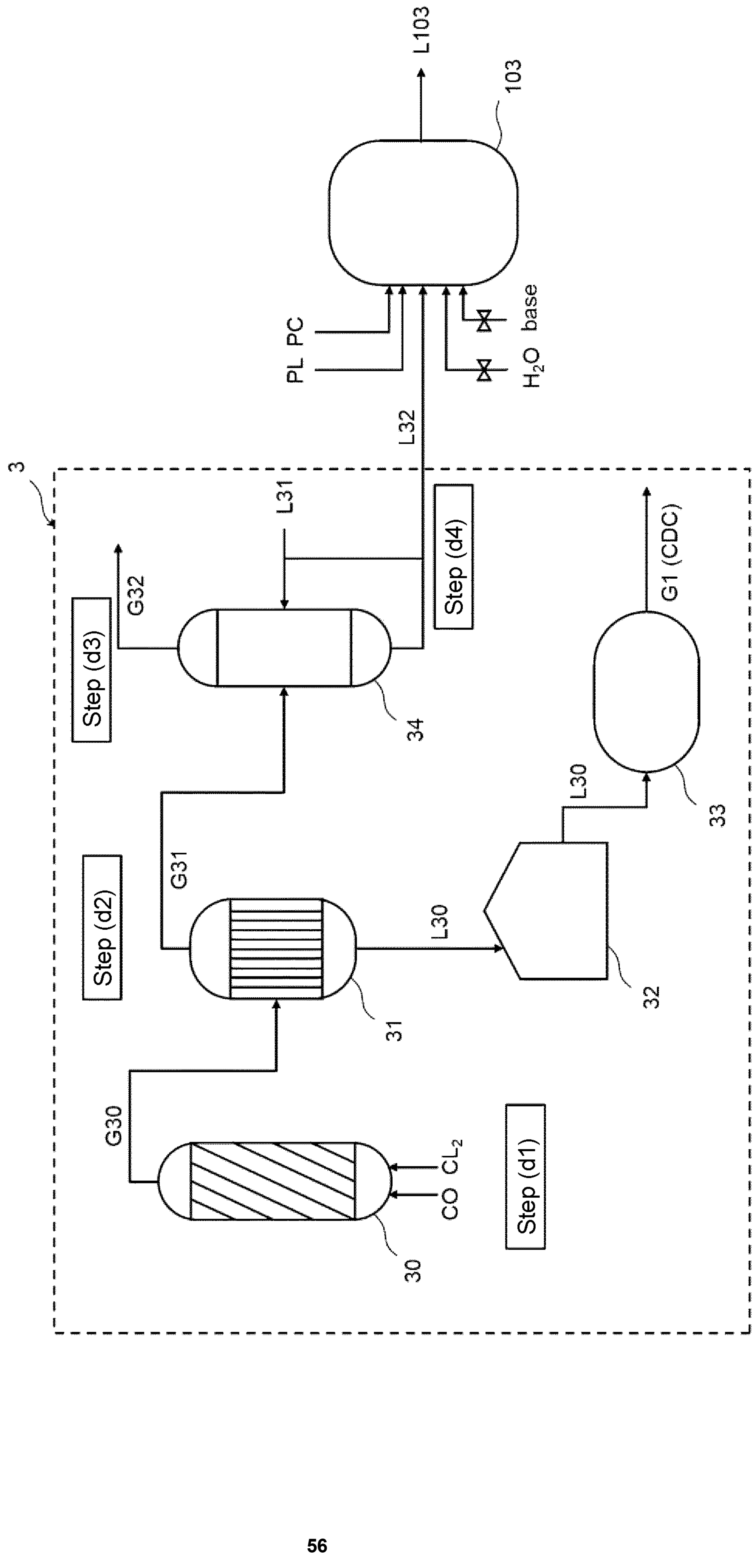
L103
103
PL
PC
$H_2O$
base
L32
3
L31
G32
Step (d3)
Step (d4)
34
G31
Step (d2)
L30
L30
G1 (CDC)
33
32
31
G30
CO
$CL_2$
Step (d1)
30

**INTERNATIONAL SEARCH REPORT**

International application No. **PCT/JP2021/039740**

**A. CLASSIFICATION OF SUBJECT MATTER**

***C07C 37/52***(2006.01)i; ***C01B 32/50***(2017.01)i; ***C07C 39/16***(2006.01)i; ***C07C 68/04***(2006.01)i; ***C07C 69/96***(2006.01)i; ***C08G 59/06***(2006.01)i; ***C08G 64/30***(2006.01)i; ***C08J 11/24***(2006.01)i; ***C08J 11/28***(2006.01)i; *C07B 61/00*(2006.01)n
FI: C07C37/52; C01B32/50; C07C39/16 ZAB; C07C68/04 A; C07C69/96 Z; C08G59/06; C08G64/30; C08J11/24; C08J11/28; C07B61/00 300

According to International Patent Classification (IPC) or to both national classification and IPC

**B. FIELDS SEARCHED**

Minimum documentation searched (classification system followed by classification symbols)

C07C37/52; C01B32/50; C07C39/16; C07C68/04; C07C69/96; C08G59/06; C08G64/30; C08J11/24; C08J11/28; C07B61/00

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JST7580/JSTChina (JDreamIII)

**C. DOCUMENTS CONSIDERED TO BE RELEVANT**

| Category* | Citation of document, with indication, where appropriate, of the relevant passages | Relevant to claim No. |
|---|---|---|
| P, A | WO 2020/257237 A1 (SABIC GLOBAL TECHNOLOGIES B.V.) 24 December 2020 (2020-12-24)<br>entire text | 1-29 |
| A | JP 40-16536 B1 (TEIJIN CHEMICALS LTD) 29 July 1965 (1965-07-29)<br>entire text | 1-29 |
| A | JP 6-287295 A (TEIJIN CHEMICALS LTD) 11 October 1994 (1994-10-11)<br>entire text | 1-29 |
| A | JP 6-56985 A (BAYER AG) 01 March 1994 (1994-03-01)<br>entire text | 1-29 |
| A | JP 2004-51620 A (VICTOR CO OF JAPAN LTD) 19 February 2004 (2004-02-19)<br>entire text | 1-29 |
| A | JP 2005-8773 A (VICTOR CO OF JAPAN LTD) 13 January 2005 (2005-01-13)<br>entire text | 1-29 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **09 December 2021** | **28 December 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2021/039740**

**C. DOCUMENTS CONSIDERED TO BE RELEVANT**

| Category* | Citation of document, with indication, where appropriate, of the relevant passages | Relevant to claim No. |
|---|---|---|
| A | JP 7-196582 A (GENERAL ELECTRIC CO <GE>) 01 August 1995 (1995-08-01) entire text | 1-29 |
| A | JP 2008-195646 A (TEIJIN CHEMICALS LTD) 28 August 2008 (2008-08-28) entire text | 1-29 |

Form PCT/ISA/210 (second sheet) (January 2015)

**INTERNATIONAL SEARCH REPORT**
**Information on patent family members**

International application No.
**PCT/JP2021/039740**

| Patent document cited in search report | Publication date (day/month/year) | Patent family member(s) | Publication date (day/month/year) |
|---|---|---|---|
| WO 2020/257237 A1 | 24 December 2020 | (Family: none) | |
| JP 40-16536 B1 | 29 July 1965 | (Family: none) | |
| JP 6-287295 A | 11 October 1994 | (Family: none) | |
| JP 6-56985 A | 01 March 1994 | US 5391802 A<br>entire text<br>DE 4220412 A | |
| JP 2004-51620 A | 19 February 2004 | (Family: none) | |
| JP 2005-8773 A | 13 January 2005 | (Family: none) | |
| JP 7-196582 A | 01 August 1995 | US 5336814 A<br>entire text<br>EP 641758 A2<br>CN 1103062 A | |
| JP 2008-195646 A | 28 August 2008 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 4016536 B
- WO 2006114893 A
- JP 7196582 A
- JP 7316280 A
- JP 2005097568 A
- JP 2004345883 A
- JP 2006144023 A
- JP 2011225711 A
- JP 2012092247 A
- JP 2012111858 A

### Non-patent literature cited in the description

- *Non-Patent Literature ChemSusChem,* 2013, vol. 6, 1341-1344